# FASCICULE DE BREVET EUROPEEN

(11) **EP 1 594 964 B1**
(45) Date de publication et mention de la délivrance du brevet: **11.04.2012**
(21) Numéro de dépôt: 04709264.8
(22) Date de dépôt: 09.02.2004
(51) Int. Cl.: C12N 15/113

(54) **OLIGONUCLEOTIDES INHIBANT L'EXPRESSION DE LA PROTEINE OB-RGRP ET PROCEDE DE DETECTION DE COMPOSES MODIFIANT L'INTERACTION ENTRE LES PROTEINES DE LA FAMILLE DE OB-RGRP ET LE RECEPTEUR DE LA LEPTINE**
OLIGONUKLEOTIDE, WELCHE DIE EXPRESSION DES OB-RGRP PROTEINS INHIBIEREN, UND VERFAHREN ZUR DETEKTION VON VERBINDUNGEN, WELCHE DIE WECHSELWIRKUNG ZWISCHEN PROTEINEN AUS DER OB-RGRP FAMILIE UND DEM LEPTIN-REZEPTOR MODIFIZIEREN
OLIGONUCLEOTIDES WHICH INHIBIT THE EXPRESSION OF THE OB-RGRP PROTEIN AND METHOD FOR DETECTION OF COMPOUNDS MODIFYING THE INTERACTION BETWEEN THE PROTEINS OF THE OB-RGRP FAMILY AND THE LEPTIN RECEPTOR

(30) Priorité: 10.02.2003 FR 0301543
(43) Date de publication de la demande: 16.11.2005
(73) Titulaire: Aventis Pharma S.A., 92160 Antony (FR); INSTITUT NATIONAL DE LA SANTE ET DE LA RECHERCHE MEDICALE (INSERM), 75654 Paris Cédex 13 (FR); Centre National de la Recherche Scientifique (CNRS), 75006 Paris (FR)
(72) Inventeur: JOCKERS, Ralf, F-91440 BURES SUR YVETTE (FR); COUTURIER, Cyril, F-75014 PARIS (FR); UHLMANN, Eugen, 61479 GLASHÜTTEN (DE)
(74) Mandataire: Bouvet, Philippe
(86) Numéro de dépôt international: PCT/FR2004/000294
(87) Numéro de publication internationale: WO 2004/072293

(56) Documents cités:
- EP-A- 0 969 091
- WO-A-98/05792
- WO-A-03/072787
- DATABASE GENESEQ 15 juillet 2002 (2002-07-15), SHOSHAN ET AL.: "Human spliced transcript detection oligonucleotide SEQ ID No:13216" XP002289755 Database accession no. ABN40468 & WO 02/10449 A (MINTZ ELI ; SHOSHAN AVI (US); COMPUGEN INC (US); FAIGLER SIMCHON (US);) 7 février 2002 (2002-02-07)
- DATABASE GENESEQ 15 juillet 2002 (2002-07-15), SHOSHAN ET AL.: "Human spliced transcript detection oligonucleotide SEQ ID NO:19168" XP002289756 Database accession no. ABN46420
- DATABASE GENESEQ 31 octobre 2002 (2002-10-31), LEGRAIN AND DAVIET: "Human cDNA encoding an adipocyte bait protein, OBRGRP_v2" XP002289757 Database accession no. ACA56897
- DATABASE GENESEQ 14 janvier 1999 (1999-01-14), JACOBS ET AL.: "Human secreted protein clone gc57 4 probe" XP002289758 Database accession no. AAV80755
- DATABASE GENESEQ 9 novembre 2001 (2001-11-09), SHIMKETS AND LEACH: "Human silent SNP containing nucleic acid SEQ:2460" XP002289759 Database accession no. AAI75519
- SERON K ET AL: "OB-RGRP AND LEPROTL1 DEFINE A NEW FAMILY OF LIPID RAFT-ASSOCIATED TETRASPANNING MEMBRANE PROTEINS LOCALIZED AT THE TGN AND/OR ENDOSOMES" BIOLOGY OF THE CELL, ELSEVIER, PARIS, FR, vol. 93, no. 3/4, 7 novembre 2001 (2001-11-07), page 227, XP001154434 ISSN: 0248-4900
- EFFERTH THOMAS ET AL: "Leptin contributes to the protection of human leukemic cells from cisplatinum cytoxicity" ANTICANCER RESEARCH, vol. 20, no. 4, juillet 2000 (2000-07), pages 2541-2546, XP009018644 ISSN: 0250-7005
- BAILLEUL B ET AL: "THE LEPTIN RECEPTOR PROMOTER CONTROLS EXPRESSION OF A SECOND DISTINCT PROTEIN" NUCLEIC ACIDS RESEARCH, OXFORD UNIVERSITY PRESS, SURREY, GB, vol. 25, no. 14, 15 juillet 1997 (1997-07-15), pages 2752-2758, XP002050420 ISSN: 0305-1048
- TARTAGLIA L A ET AL: "IDENTIFICATION AND EXPRESSION CLONING OF A LEPTIN RECEPTOR, OB-R" CELL, CELL PRESS, CAMBRIDGE, NA, US, vol. 83, no. 7, 29 décembre 1995 (1995-12-29), pages 1263-1271, XP000602068 ISSN: 0092-8674
- HUANG Y ET AL: "Cloning and characterization of a novel human leptin receptor overlapping transcript-like 1 gene (LEPROTL1)" BIOCHIMICA ET BIOPHYSICA ACTA. GENE STRUCTURE AND EXPRESSION, ELSEVIER, AMSTERDAM, NL, vol. 1517, no. 2, 26 janvier 2001 (2001-01-26), pages 327-331, XP004248823 ISSN: 0167-4781
- LUNDIN A ET AL: "Expression and intracellular localization of leptin receptor long isoform-GFP chimera" BIOCHIMICA ET BIOPHYSICA ACTA. MOLECULAR CELL RESEARCH, ELSEVIER SCIENCE PUBLISHERS, AMSTERDAM, NL, vol. 1499, no. 1-2, 11 décembre 2000 (2000-12-11), pages 130-138, XP004278202 ISSN: 0167-4889
- BOUTE N ET AL: "MONITORING THE ACTIVATION STATE OF THE INSULIN RECEPTOR USING BIOLUMINESCENCE RESONANCE ENERGY TRANSFER" MOLECULAR PHARMACOLOGY, BALTIMORE, MD, US, vol. 4, no. 60, octobre 2001 (2001-10), pages 640-645, XP001053695 ISSN: 0026-895X
- BOUTE N ET AL: "The use of resonance energy transfer in high-throughput screening: BRET versus FRET" TRENDS IN PHARMACOLOGICAL SCIENCES, ELSEVIER TRENDS JOURNAL, CAMBRIDGE, GB, vol. 23, no. 8, août 2002 (2002-08), pages 351-354, XP004386171 ISSN: 0165-6147

### SEQUENCE LISTING

<110> AVENTIS PHARMA S.A. CNRS
   INSERM
<120> OB RGRP
<130> FRAV2003/0005
<160> 47
<170> PatentIn version 3.1
<210> 1
   <211> 648
   <212> DNA
   <213> Homo sapiens
<400> 1
   cactttattc tgattacagt gcattgaatt tcttagaact catactatct gtatacatgt      60
   gcacatgcgg cattttacta tgaaatttaa tatgctgggt tttttaatac ctttatatat     120
   catgttcact ttaagaaaga cttcataagt aggagatgag ttttattctc agcaaataga     180
   cctgtcaaat ttagattatg ttactcaaat tatgttactt gtttggctgt tcatgtagtc     240
   acggtgctct cagaaaatat attaacgcag tcttgtaggc agctgccacc ttatgcagtg     300
   catcgaaacc ttttgcttgg ggatgtgctt ggagaggcag ataacgctga agcaggcctc     360
   tcatgaccca ggaaggccgg ggtggatccc tctttgtgtt gtagtccatg ctattaaaag     420
   tgtggcccac agaccaagag cctcaacatt tcctagagcc ttattagaaa tgcagaatct     480
   gaagccccac tctggaccca ggacattttg atgagatcca aaggagttgt atgcacatga     540
   aagtttgaga agcatcatca tagagaagta aacatcacac ccaacttcct tatctttcca     600
   gtggctaaac cacttaacct ctctgggtgt tacctgctca tttgttta                  648
<210> 2
   <211> 20
   <212> DNA
   <213> Artificial Sequence
<220>
   <223> AS14
   <220>
   <221> misc_feature
   <223> antisens AS14
<400> 2
   aatgccgcat gtgcacatgt 20
<210> 3
   <211> 396
   <212> DNA
   <213> Homo sapiens
<220>
   <221> CDS
   <222> (1)..(396)
   <223>
<400> 3
   atg gcg ggc gtt aaa gct ctc gtg gca tta tcc ttc agt ggg gct att      48
   Met Ala Gly Val Lys Ala Leu Val Ala Leu Ser Phe Ser Gly Ala Ile
   1               5                   10                  15
   gga ctg act ttt ctt atg ctg gga tgt gcc tta gag gat tat ggc gtt      96
   Gly Leu Thr Phe Leu Met Leu Gly Cys Ala Leu Glu Asp Tyr Gly Val
               20                  25                  30
   tac tgg ccc tta ttc gtc ctg att ttc cac gcc atc tcc ccc atc ccc      144
   Tyr Trp Pro Leu Phe Val Leu Ile Phe His Ala Ile Ser Pro Ile Pro
           35                  40                  45
   cat ttc att gcc aaa aga gtc acc tat gac tca gat gca acc agt agt      192
   His Phe Ile Ala Lys Arg Val Thr Tyr Asp Ser Asp Ala Thr Ser Ser
       50                  55                  60
   gcc tgt cgg gaa ctg gca tat ttc ttc act act gga att gtt gtt tct      240
   Ala Cys Arg Glu Leu Ala Tyr Phe Phe Thr Thr Gly Ile Val Val Ser
   65                  70                  75                  80
   gcc ttt gga ttt cct gtt att ctt gct cgt gtg gct gtg atc aaa tgg      288
   Ala Phe Gly Phe Pro Val Ile Leu Ala Arg Val Ala Val Ile Lys Trp
                   85                  90                  95
   gga gcc tgc ggc ctt gtg ttg gca ggc aat gca gtc att ttc ctt aca      336
   Gly Ala Cys Gly Leu Val Leu Ala Gly Asn Ala Val Ile Phe Leu Thr
               100                 105                 110
   att caa ggg ttt ttc ctt ata ttt gga aga gga gat gat ttt agc tgg      384
   Ile Gln Gly Phe Phe Leu Ile Phe Gly Arg Gly Asp Asp Phe Ser Trp
           115                 120                 125
   gag cag tgg tag                                                      396
   Glu Gln Trp
       130
<210> 4
   <211> 131
   <212> PRT
   <213> Homo sapiens
<400> 4
   Met Ala Gly Val Lys Ala Leu Val Ala Leu Ser Phe Ser Gly Ala Ile
   1               5                   10                  15
   Gly Leu Thr Phe Leu Met Leu Gly Cys Ala Leu Glu Asp Tyr Gly Val
               20                  25                  30
   Tyr Trp Pro Leu Phe Val Leu Ile Phe His Ala Ile Ser Pro Ile Pro
           35                  40                  45
   His Phe Ile Ala Lys Arg Val Thr Tyr Asp Ser Asp Ala Thr Ser Ser
       50                  55                  60
   Ala Cys Arg Glu Leu Ala Tyr Phe Phe Thr Thr Gly Ile Val Val Ser
   65                  70                  75                  80
   Ala Phe Gly Phe Pro Val Ile Leu Ala Arg Val Ala Val Ile Lys Trp
                   85                  90                  95
   Gly Ala Cys Gly Leu Val Leu Ala Gly Asn Ala Val Ile Phe Leu Thr
               100                 105                 110
   Ile Gln Gly Phe Phe Leu Ile Phe Gly Arg Gly Asp Asp Phe Ser Trp
           115                 120                 125
   Glu Gln Trp
       130
<210> 5
   <211> 1359
   <212> DNA
   <213> Artificial Sequence
<220>
   <223> OB RGRP LUC
   <220>
   <221> misc_feature
   <223> OB RGRP LUC
<220>
   <221> CDS
   <222> (1)..(1359)
   <223>
<400> 5
   atg gcg ggc gtt aaa gct ctc gtg gca tta tcc ttc agt ggg gct att      48
   Met Ala Gly Val Lys Ala Leu Val Ala Leu Ser Phe Ser Gly Ala Ile
   1               5                   10                  15
   gga ctg act ttt ctt atg ctg gga tgt gcc tta gag gat tat ggc gtt      96
   Gly Leu Thr Phe Leu Met Leu Gly Cys Ala Leu Glu Asp Tyr Gly Val
               20                  25                  30
   tac tgg ccc tta ttc gtc ctg att ttc cac gcc atc tcc ccc atc ccc     144
   Tyr Trp Pro Leu Phe Val Leu Ile Phe His Ala Ile Ser Pro Ile Pro
           35                  40                  45
   cat ttc att gcc aaa aga gtc acc tat gac tca gat gca acc agt agt     192
   His Phe Ile Ala Lys Arg Val Thr Tyr Asp Ser Asp Ala Thr Ser Ser
       50                  55                  60
   gcc tgt cgg gaa ctg gca tat ttc ttc act act gga att gtt gtt tct     240
   Ala Cys Arg Glu Leu Ala Tyr Phe Phe Thr Thr Gly Ile Val Val Ser
   65                  70                  75                  80
   gcc ttt gga ttt cct gtt att ctt gct cgt gtg gct gtg atc aaa tgg     288
   Ala Phe Gly Phe Pro Val Ile Leu Ala Arg Val Ala Val Ile Lys Trp
                   85                  90                  95
   gga gcc tgc ggc ctt gtg ttg gca ggc aat gca gtc att ttc ctt aca     336
   Gly Ala Cys Gly Leu Val Leu Ala Gly Asn Ala Val Ile Phe Leu Thr
               100                 105                 110
   att caa ggg ttt ttc ctt ata ttt gga aga gga gat gat ttt agc tgg     384
   Ile Gln Gly Phe Phe Leu Ile Phe Gly Arg Gly Asp Asp Phe Ser Trp
           115                 120                 125
   gag cag tgg att ccg ggg gat cca ccg gct aga gcc acc atg acc agc     432
   Glu Gln Trp Ile Pro Gly Asp Pro Pro Ala Arg Ala Thr Met Thr Ser
       130                 135                 140
   aag gtg tac gac ccc gag cag agg aag agg atg atc acc ggc ccc cag     480
   Lys Val Tyr Asp Pro Glu Gln Arg Lys Arg Met Ile Thr Gly Pro Gln
   145                 150                 155                 160
   tgg tgg gcc agg tgc aag cag atg aac gtg ctg gac agc ttc atc aac     528
   Trp Trp Ala Arg Cys Lys Gln Met Asn Val Leu Asp Ser Phe Ile Asn
                   165                 170                 175
   tac tac gac agc gag aag cac gcc gag aac gcc gtg atc ttc ctg cac     576
   Tyr Tyr Asp Ser Glu Lys His Ala Glu Asn Ala Val Ile Phe Leu His
               180                 185                 190
   ggc aac gcc gct agc agc tac ctg tgg agg cac gtg gtg ccc cac atc      624
   Gly Asn Ala Ala Ser Ser Tyr Leu Trp Arg His Val Val Pro His Ile
           195                 200                 205
   gag ccc gtg gcc agg tgc atc atc ccc gat ctg atc ggc atg ggc aag      672
   Glu Pro Val Ala Arg Cys Ile Ile Pro Asp Leu Ile Gly Met Gly Lys
       210                 215                 220
   agc ggc aag agc ggc aac ggc agc tac agg ctg ctg gac cac tac aag      720
   Ser Gly Lys Ser Gly Asn Gly Ser Tyr Arg Leu Leu Asp His Tyr Lys
   225                 230                 235                 240
   tac ctg acc gcc tgg ttc gag ctc ctg aac ctg ccc aag aag atc atc      768
   Tyr Leu Thr Ala Trp Phe Glu Leu Leu Asn Leu Pro Lys Lys Ile Ile
                   245                 250                 255
   ttc gtg ggc cac gac tgg ggc gcc tgc ctg gcc ttc cac tac agc tac      816
   Phe Val Gly His Asp Trp Gly Ala Cys Leu Ala Phe His Tyr Ser Tyr
               260                 265                 270
   gag cac cag gac aag atc aag gcc atc gtg cac gcc gag agc gtg gtg      864
   Glu His Gln Asp Lys Ile Lys Ala Ile Val His Ala Glu Ser Val Val
           275                 280                 285
   gac gtg atc gag agc tgg gac gag tgg cca gac atc gag gag gac atc      912
   Asp Val Ile Glu Ser Trp Asp Glu Trp Pro Asp Ile Glu Glu Asp Ile
       290                 295                 300
   gcc ctg atc aag agc gag gag ggc gag aag atg gtg ctg gag aac aac      960
   Ala Leu Ile Lys Ser Glu Glu Gly Glu Lys Met Val Leu Glu Asn Asn
   305                 310                 315                 320
   ttc ttc gtg gag acc atg ctg ccc agc aag atc atg aga aag ctg gag     1008
   Phe Phe Val Glu Thr Met Leu Pro Ser Lys Ile Met Arg Lys Leu Glu
                   325                 330                 335
   ccc gag gag ttc gcc gcc tac ctg gag ccc ttc aag gag aag ggc gag     1056
   Pro Glu Glu Phe Ala Ala Tyr Leu Glu Pro Phe Lys Glu Lys Gly Glu
               340                 345                 350
   gtg aga aga ccc acc ctg agc tgg ccc aga gag atc ccc ctg gtg aag     1104
   Val Arg Arg Pro Thr Leu Ser Trp Pro Arg Glu Ile Pro Leu Val Lys
           355                 360                 365
   ggc ggc aag ccc gac gtg gtg cag atc gtg aga aac tac aac gcc tac     1152
   Gly Gly Lys Pro Asp Val Val Gln Ile Val Arg Asn Tyr Asn Ala Tyr
       370                 375                 380
   ctg aga gcc agc gac gac ctg ccc aag atg ttc atc gag agc gac ccc     1200
   Leu Arg Ala Ser Asp Asp Leu Pro Lys Met Phe Ile Glu Ser Asp Pro
   385                 390                 395                 400
   ggc ttc ttc agc aac gcc atc gtg gag ggc gcc aag aag ttc ccc aac     1248
   Gly Phe Phe Ser Asn Ala Ile Val Glu Gly Ala Lys Lys Phe Pro Asn
                   405                 410                 415
   acc gag ttc gtg aag gtg aag ggc ctg cac ttc agc cag gag gac gcc     1296
   Thr Glu Phe Val Lys Val Lys Gly Leu His Phe Ser Gln Glu Asp Ala
               420                 425                 430
   ccc gac gag atg ggc aag tac atc aag agc ttc gtg gag aga gtg ctg     1344
   Pro Asp Glu Met Gly Lys Tyr Ile Lys Ser Phe Val Glu Arg Val Leu
           435                 440                 445
   aag aac gag cag taa                                                 1359
   Lys Asn Glu Gln
   450
<210> 6
   <211> 452
   <212> PRT
   <213> Artificial Sequence
<220>
   <223> OB RGRP LUC
<220>
   <221> misc_feature
   <223> OB RGRP LUC
<400> 6
   Met Ala Gly Val Lys Ala Leu Val Ala Leu Ser Phe Ser Gly Ala Ile
   1 5 10 15
   Gly Leu Thr Phe Leu Met Leu Gly Cys Ala Leu Glu Asp Tyr Gly Val
   20 25 30
   Tyr Trp Pro Leu Phe Val Leu Ile Phe His Ala Ile Ser Pro Ile Pro
   35 40 45
   His Phe Ile Ala Lys Arg Val Thr Tyr Asp Ser Asp Ala Thr Ser Ser
   50 55 60
   Ala Cys Arg Glu Leu Ala Tyr Phe Phe Thr Thr Gly Ile Val Val Ser
   65 70 75 80
   Ala Phe Gly Phe Pro Val Ile Leu Ala Arg Val Ala Val Ile Lys Trp
   85 90 95
   Gly Ala Cys Gly Leu Val Leu Ala Gly Asn Ala Val Ile Phe Leu Thr
   100 105 110
   Ile Gln Gly Phe Phe Leu Ile Phe Gly Arg Gly Asp Asp Phe Ser Trp
   115 120 125
   Glu Gln Trp Ile Pro Gly Asp Pro Pro Ala Arg Ala Thr Met Thr Ser
   130 135 140
   Lys Val Tyr Asp Pro Glu Gln Arg Lys Arg Met Ile Thr Gly Pro Gln
   145 150 155 160
   Trp Trp Ala Arg Cys Lys Gln Met Asn Val Leu Asp Ser Phe Ile Asn
   165 170 175
   Tyr Tyr Asp Ser Glu Lys His Ala Glu Asn Ala Val Ile Phe Leu His
   180 185 190
   Gly Asn Ala Ala Ser Ser Tyr Leu Trp Arg His Val Val Pro His Ile
   195 200 205
   Glu Pro Val Ala Arg Cys Ile Ile Pro Asp Leu Ile Gly Met Gly Lys
   210 215 220
   Ser Gly Lys Ser Gly Asn Gly Ser Tyr Arg Leu Leu Asp His Tyr Lys
   225 230 235 240
   Tyr Leu Thr Ala Trp Phe Glu Leu Leu Asn Leu Pro Lys Lys Ile Ile
   245 250 255
   Phe Val Gly His Asp Trp Gly Ala Cys Leu Ala Phe His Tyr Ser Tyr
   260 265 270
   Glu His Gln Asp Lys Ile Lys Ala Ile Val His Ala Glu Ser Val Val
   275 280 285
   Asp Val Ile Glu Ser Trp Asp Glu Trp Pro Asp Ile Glu Glu Asp Ile
   290 295 300
   Ala Leu Ile Lys Ser Glu Glu Gly Glu Lys Met Val Leu Glu Asn Asn
   305 310 315 320
   Phe Phe Val Glu Thr Met Leu Pro Ser Lys Ile Met Arg Lys Leu Glu
   325 330 335
   Pro Glu Glu Phe Ala Ala Tyr Leu Glu Pro Phe Lys Glu Lys Gly Glu
   340 345 350
   Val Arg Arg Pro Thr Leu Ser Trp Pro Arg Glu Ile Pro Leu Val Lys
   355 360 365
   Gly Gly Lys Pro Asp Val Val Gln Ile Val Arg Asn Tyr Asn Ala Tyr
   370 375 380
   Leu Arg Ala Ser Asp Asp Leu Pro Lys Met Phe Ile Glu Ser Asp Pro
   385 390 395 400
   Gly Phe Phe Ser Asn Ala Ile Val Glu Gly Ala Lys Lys Phe Pro Asn
   405 410 415
   Thr Glu Phe Val Lys Val Lys Gly Leu His Phe Ser Gln Glu Asp Ala
   420 425 430
   Pro Asp Glu Met Gly Lys Tyr Ile Lys Ser Phe Val Glu Arg Val Leu
   435 440 445
   Lys Asn Glu Gln
       450
<210> 7
   <211> 1128
   <212> DNA
   <213> Artificial sequence
<220>
   <223> OB RGRP YFP
   <220>
   <221> misc_feature
   <223> OB RGRP YFP
<220>
   <221> CDS
   <222> (1)..(1128)
   <223>
<400> 7
   atg gcg ggc gtt aaa gct ctc gtg gca tta tcc ttc agt ggg gct att       48
   Met Ala Gly Val Lys Ala Leu Val Ala Leu Ser Phe Ser Gly Ala Ile
   1               5                   10                  15
   gga ctg act ttt ctt atg ctg gga tgt gcc tta gag gat tat ggc gtt       96
   Gly Leu Thr Phe Leu Met Leu Gly Cys Ala Leu Glu Asp Tyr Gly Val
               20                  25                  30
   tac tgg ccc tta ttc gtc ctg att ttc cac gcc atc tcc ccc atc ccc      144
   Tyr Trp Pro Leu Phe Val Leu Ile Phe His Ala Ile Ser Pro Ile Pro
           35                  40                  45
   cat ttc att gcc aaa aga gtc acc tat gac tca gat gca acc agt agt      192
   His Phe Ile Ala Lys Arg Val Thr Tyr Asp Ser Asp Ala Thr Ser Ser
       50                  55                  60
   gcc tgt cgg gaa ctg gca tat ttc ttc act act gga att gtt gtt tct      240
   Ala Cys Arg Glu Leu Ala Tyr Phe Phe Thr Thr Gly Ile Val Val Ser
   65                  70                  75                  80
   gcc ttt gga ttt cct gtt att ctt gct cgt gtg gct gtg atc aaa tgg      288
   Ala Phe Gly Phe Pro Val Ile Leu Ala Arg Val Ala Val Ile Lys Trp
                   85                  90                  95
   gga gcc tgc ggc ctt gtg ttg gca ggc aat gca gtc att ttc ctt aca      336
   Gly Ala Cys Gly Leu Val Leu Ala Gly Asn Ala Val Ile Phe Leu Thr
               100                 105                 110
   att caa ggg ttt ttc ctt ata ttt gga aga gga gat gat ttt agc tgg      384
   Ile Gln Gly Phe Phe Leu Ile Phe Gly Arg Gly Asp Asp Phe Ser Trp
   115 120 125
   gag cag tgg att ccg gtc gcc acc atg gtg agc aag ggc gag gag ctg 432
   Glu Gln Trp Ile Pro Val Ala Thr Met Val Ser Lys Gly Glu Glu Leu
   130 135 140
   ttc acc ggg gtg gtg ccc atc ctg gtc gag ctg gac ggc gac gta aac 480
   Phe Thr Gly Val Val Pro Ile Leu Val Glu Leu Asp Gly Asp Val Asn
   145 150 155 160
   ggc cac aag ttc agc gtg tcc ggc gag ggc gag ggc gat gcc acc tac 528
   Gly His Lys Phe Ser Val Ser Gly Glu Gly Glu Gly Asp Ala Thr Tyr
   165 170 175
   ggc aag ctg acc ctg aag ttc atc tgc acc acc ggc aag ctg ccc gtg 576
   Gly Lys Leu Thr Leu Lys Phe Ile Cys Thr Thr Gly Lys Leu Pro Val
   180 185 190
   ccc tgg ccc acc ctc gtg acc acc ttc ggc tac ggc gtg cag tgc ttc 624
   Pro Trp Pro Thr Leu Val Thr Thr Phe Gly Tyr Gly Val Gln Cys Phe
   195 200 205
   gcc cgc tac ccc gac cac atg cgc cag cac gac ttc ttc aag tcc gcc 672
   Ala Arg Tyr Pro Asp His Met Arg Gln His Asp Phe Phe Lys Ser Ala
   210 215 220
   atg ccc gaa ggc tac gtc cag gag cgc acc atc ttc ttc aag gac gac 720
   Met Pro Glu Gly Tyr Val Gln Glu Arg Thr Ile Phe Phe Lys Asp Asp
   225 230 235 240
   ggc aac tac aag acc cgc gcc gag gtg aag ttc gag ggc gac acc ctg 768
   Gly Asn Tyr Lys Thr Arg Ala Glu Val Lys Phe Glu Gly Asp Thr Leu
   245 250 255
   gtg aac cgc atc gag ctg aag ggc atc gac ttc aag gag gac ggc aac 816
   Val Asn Arg Ile Glu Leu Lys Gly Ile Asp Phe Lys Glu Asp Gly Asn
   260 265 270
   atc ctg ggg cac aag ctg gag tac aac tac aac agc cac aac gtc tat 864
   Ile Leu Gly His Lys Leu Glu Tyr Asn Tyr Asn Ser His Asn Val Tyr
   275 280 285
   atc atg gcc gac aag cag aag aac ggc atc aag gtg aac ttc aag atc 912
   Ile Met Ala Asp Lys Gln Lys Asn Gly Ile Lys Val Asn Phe Lys Ile
   290 295 300
   cgc cac aac atc gag gac ggc agc gtg cag ctc gcc gac cac tac cag 960
   Arg His Asn Ile Glu Asp Gly Ser Val Gln Leu Ala Asp His Tyr Gln
   305 310 315 320
   cag aac acc ccc atc ggc gac ggc ccc gtg ctg ctg ccc gac aac cac 1008
   Gln Asn Thr Pro Ile Gly Asp Gly Pro Val Leu Leu Pro Asp Asn His
   325 330 335
   tac ctg agc tac cag tcc gcc ctg agc aaa gac ccc aac gag aag cgc 1056
   Tyr Leu Ser Tyr Gln Ser Ala Leu Ser Lys Asp Pro Asn Glu Lys Arg
   340 345 350
   gat cac atg gtc ctg ctg gag ttc gtg acc gcc gcc ggg atc act ctc 1104
   Asp His Met Val Leu Leu Glu Phe Val Thr Ala Ala Gly Ile Thr Leu
   355 360 365
   ggc atg gac gag ctg tac aag taa 1128
   Gly Met Asp Glu Leu Tyr Lys
   370 375
<210> 8
   <211> 375
   <212> PRT
   <213> Artificial Sequence
<220>
   <223> OB RGRP YFP
<220>
   <221> misc_feature
   <223> OB RGRP YFP
<400> 8
   Met Ala Gly Val Lys Ala Leu Val Ala Leu Ser Phe Ser Gly Ala Ile
   1               5                   10                  15
   Gly Leu Thr Phe Leu Met Leu Gly Cys Ala Leu Glu Asp Tyr Gly Val
               20                  25                  30
   Tyr Trp Pro Leu Phe Val Leu Ile Phe His Ala Ile Ser Pro Ile Pro
           35                  40                  45
   His Phe Ile Ala Lys Arg Val Thr Tyr Asp Ser Asp Ala Thr Ser Ser
       50                  55                  60
   Ala Cys Arg Glu Leu Ala Tyr Phe Phe Thr Thr Gly Ile Val Val Ser
   65                  70                  75                  80
   Ala Phe Gly Phe Pro Val Ile Leu Ala Arg Val Ala Val Ile Lys Trp
                   85                  90                  95
   Gly Ala Cys Gly Leu Val Leu Ala Gly Asn Ala Val Ile Phe Leu Thr
               100                 105                 110
   Ile Gln Gly Phe Phe Leu Ile Phe Gly Arg Gly Asp Asp Phe Ser Trp
           115                 120                 125
   Glu Gln Trp Ile Pro Val Ala Thr Met Val Ser Lys Gly Glu Glu Leu
       130                 135                 140
   Phe Thr Gly Val Val Pro Ile Leu Val Glu Leu Asp Gly Asp Val Asn
   145                 150                 155                 160
   Gly His Lys Phe Ser Val Ser Gly Glu Gly Glu Gly Asp Ala Thr Tyr
                   165                 170                 175
   Gly Lys Leu Thr Leu Lys Phe Ile Cys Thr Thr Gly Lys Leu Pro Val
               180                 185                 190
   Pro Trp Pro Thr Leu Val Thr Thr Phe Gly Tyr Gly Val Gln Cys Phe
           195                 200                 205
   Ala Arg Tyr Pro Asp His Met Arg Gln His Asp Phe Phe Lys Ser Ala
       210                 215                 220
   Met Pro Glu Gly Tyr Val Gln Glu Arg Thr Ile Phe Phe Lys Asp Asp
   225                 230                 235                 240
   Gly Asn Tyr Lys Thr Arg Ala Glu Val Lys Phe Glu Gly Asp Thr Leu
                   245                 250                 255
   Val Asn Arg Ile Glu Leu Lys Gly Ile Asp Phe Lys Glu Asp Gly Asn
               260                 265                 270
   Ile Leu Gly His Lys Leu Glu Tyr Asn Tyr Asn Ser His Asn Val Tyr
           275                 280                 285
   Ile Met Ala Asp Lys Gln Lys Asn Gly Ile Lys Val Asn Phe Lys Ile
       290                 295                 300
   Arg His Asn Ile Glu Asp Gly Ser Val Gln Leu Ala Asp His Tyr Gln
   305                 310                 315                 320
   Gln Asn Thr Pro Ile Gly Asp Gly Pro Val Leu Leu Pro Asp Asn His
                   325                 330                 335
   Tyr Leu Ser Tyr Gln Ser Ala Leu Ser Lys Asp Pro Asn Glu Lys Arg
               340                 345                 350
   Asp His Met Val Leu Leu Glu Phe Val Thr Ala Ala Gly Ile Thr Leu
           355                 360                 365
   Gly Met Asp Glu Leu Tyr Lys
       370                 375
<210> 9
   <211> 2691
   <212> DNA
   <213> Homo sapiens
<220>
   <221> CDS
   <222> (1)..(2691)
   <223>
<400> 9
   atg att tgt caa aaa ttc tgt gtg gtt ttg tta cat tgg gaa ttt att      48
   Met Ile Cys Gln Lys Phe Cys Val Val Leu Leu His Trp Glu Phe Ile
   1               5                   10                  15
   tat gtg ata act gcg ttt aac ttg tca tat cca att act cct tgg aga      96
   Tyr Val Ile Thr Ala Phe Asn Leu Ser Tyr Pro Ile Thr Pro Trp Arg
               20                  25                  30
   ttt aag ttg tct tgc atg cca cca aat tca acc tat gac tac ttc ctt     144
   Phe Lys Leu Ser Cys Met Pro Pro Asn Ser Thr Tyr Asp Tyr Phe Leu
           35                  40                  45
   ttg cct gct gga ctc tca aag aat act tca aat tcg aat gga cat tat     192
   Leu Pro Ala Gly Leu Ser Lys Asn Thr Ser Asn Ser Asn Gly His Tyr
       50                  55                  60
   gag aca gct gtt gaa cct aag ttt aat tca agt ggt act cac ttt tct     240
   Glu Thr Ala Val Glu Pro Lys Phe Asn Ser Ser Gly Thr His Phe Ser
   65                  70                  75                  80
   aac tta tcc aaa aca act ttc cac tgt tgc ttt cgg agt gag caa gat     288
   Asn Leu Ser Lys Thr Thr Phe His Cys Cys Phe Arg Ser Glu Gln Asp
                   85                  90                  95
   aga aac tgc tcc tta tgt gca gac aac att gaa gga aag aca ttt gtt     336
   Arg Asn Cys Ser Leu Cys Ala Asp Asn Ile Glu Gly Lys Thr Phe Val
               100                 105                 110
   tca aca gta aat tct tta gtt ttt caa caa ata gat gca aac tgg aac     384
   Ser Thr Val Asn Ser Leu Val Phe Gln Gln Ile Asp Ala Asn Trp Asn
           115                 120                 125
   ata cag tgc tgg cta aaa gga gac tta aaa tta ttc atc tgt tat gtg     432
   Ile Gln Cys Trp Leu Lys Gly Asp Leu Lys Leu Phe Ile Cys Tyr Val
       130                 135                 140
   gag tca tta ttt aag aat cta ttc agg aat tat aac tat aag gtc cat     480
   Glu Ser Leu Phe Lys Asn Leu Phe Arg Asn Tyr Asn Tyr Lys Val His
   145                 150                 155                 160
   ctt tta tat gtt ctg cct gaa gtg tta gaa gat tca cct ctg gtt ccc     528
   Leu Leu Tyr Val Leu Pro Glu Val Leu Glu Asp Ser Pro Leu Val Pro
                   165                 170                 175
   caa aaa ggc agt ttt cag atg gtt cac tgc aat tgc agt gtt cat gaa     576
   Gln Lys Gly Ser Phe Gln Met Val His Cys Asn Cys Ser Val His Glu
               180                 185                 190
   tgt tgt gaa tgt ctt gtg cct gtg cca aca gcc aaa ctc aac gac act     624
   Cys Cys Glu Cys Leu Val Pro Val Pro Thr Ala Lys Leu Asn Asp Thr
           195                 200                 205
   ctc ctt atg tgt ttg aaa atc aca tct ggt gga gta att ttc cag tca     672
   Leu Leu Met Cys Leu Lys Ile Thr Ser Gly Gly Val Ile Phe Gln Ser
       210                 215                 220
   cct cta atg tca gtt cag ccc ata aat atg gtg aag cct gat cca cca     720
   Pro Leu Met Ser Val Gln Pro Ile Asn Met Val Lys Pro Asp Pro Pro
   225                 230                 235                 240
   tta ggt ttg cat atg gaa atc aca gat gat ggt aat tta aag att tct     768
   Leu Gly Leu His Met Glu Ile Thr Asp Asp Gly Asn Leu Lys Ile Ser
                   245                 250                 255
   tgg tcc agc cca cca ttg gta cca ttt cca ctt caa tat caa gtg aaa     816
   Trp Ser Ser Pro Pro Leu Val Pro Phe Pro Leu Gln Tyr Gln Val Lys
               260                 265                 270
   tat tca gag aat tct aca aca gtt atc aga gaa gct gac aag att gtc      864
   Tyr Ser Glu Asn Ser Thr Thr Val Ile Arg Glu Ala Asp Lys Ile Val
           275                 280                 285
   tca gct aca tcc ctg cta gta gac agt ata ctt cct ggg tct tcg tat      912
   Ser Ala Thr Ser Leu Leu Val Asp Ser Ile Leu Pro Gly Ser Ser Tyr
       290                 295                 300
   gag gtt cag gtg agg ggc aag aga ctg gat ggc cca gga atc tgg agt      960
   Glu Val Gln Val Arg Gly Lys Arg Leu Asp Gly Pro Gly Ile Trp Ser
   305                 310                 315                 320
   gac tgg agt act cct cgt gtc ttt acc aca caa gat gtc ata tac ttt     1008
   Asp Trp Ser Thr Pro Arg Val Phe Thr Thr Gln Asp Val Ile Tyr Phe
                   325                 330                 335
   cca cct aaa att ctg aca agt gtt ggg tct aat gtt tct ttt cac tgc     1056
   Pro Pro Lys Ile Leu Thr Ser Val Gly Ser Asn Val Ser Phe His Cys
               340                 345                 350
   atc tat aag aag gaa aac aag att gtt ccc tca aaa gag att gtt tgg     1104
   Ile Tyr Lys Lys Glu Asn Lys Ile Val Pro Ser Lys Glu Ile Val Trp
           355                 360                 365
   tgg atg aat tta gct gag aaa att cct caa agc cag tat gat gtt gtg     1152
   Trp Met Asn Leu Ala Glu Lys Ile Pro Gln Ser Gln Tyr Asp Val Val
       370                 375                 380
   agt gat cat gtt agc aaa gtt act ttt ttc aat ctg aat gaa acc aaa     1200
   Ser Asp His Val Ser Lys Val Thr Phe Phe Asn Leu Asn Glu Thr Lys
   385                 390                 395                 400
   cct cga gga aag ttt acc tat gat gca gtg tac tgc tgc aat gaa cat     1248
   Pro Arg Gly Lys Phe Thr Tyr Asp Ala Val Tyr Cys Cys Asn Glu His
                   405                 410                 415
   gaa tgc cat cat cgc tat gct gaa tta tat gtg att gat gtc aat atc     1296
   Glu Cys His His Arg Tyr Ala Glu Leu Tyr Val Ile Asp Val Asn Ile
               420                 425                 430
   aat atc tca tgt gaa act gat ggg tac tta act aaa atg act tgc aga     1344
   Asn Ile Ser Cys Glu Thr Asp Gly Tyr Leu Thr Lys Met Thr Cys Arg
           435                 440                 445
   tgg tca acc agt aca atc cag tca ctt gcg gaa agc act ttg caa ttg     1392
   Trp Ser Thr Ser Thr Ile Gln Ser Leu Ala Glu Ser Thr Leu Gln Leu
       450                 455                 460
   agg tat cat agg agc agc ctt tac tgt tct gat att cca tct att cat     1440
   Arg Tyr His Arg Ser Ser Leu Tyr Cys Ser Asp Ile Pro Ser Ile His
   465                 470                 475                 480
   ccc ata tct gag ccc aaa gat tgc tat ttg cag agt gat ggt ttt tat     1488
   Pro Ile Ser Glu Pro Lys Asp Cys Tyr Leu Gln Ser Asp Gly Phe Tyr
                   485                 490                 495
   gaa tgc att ttc cag cca atc ttc cta tta tct ggc tac aca atg tgg     1536
   Glu Cys Ile Phe Gln Pro Ile Phe Leu Leu Ser Gly Tyr Thr Met Trp
               500                 505                 510
   att agg atc aat cac tct cta ggt tca ctt gac tct cca cca aca tgt     1584
   Ile Arg Ile Asn His Ser Leu Gly Ser Leu Asp Ser Pro Pro Thr Cys
           515                 520                 525
   gtc ctt cct gat tct gtg gtg aag cca ctg cct cca tcc agt gtg aaa     1632
   Val Leu Pro Asp Ser Val Val Lys Pro Leu Pro Pro Ser Ser Val Lys
       530                 535                 540
   gca gaa att act ata aac att gga tta ttg aaa ata tct tgg gaa aag      1680
   Ala Glu Ile Thr Ile Asn Ile Gly Leu Leu Lys Ile Ser Trp Glu Lys
   545                 550                 555                 560
   cca gtc ttt cca gag aat aac ctt caa ttc cag att cgc tat ggt tta      1728
   Pro Val Phe Pro Glu Asn Asn Leu Gln Phe Gln Ile Arg Tyr Gly Leu
                   565                 570                 575
   agt gga aaa gaa gta caa tgg aag atg tat gag gtt tat gat gca aaa      1776
   Ser Gly Lys Glu Val Gln Trp Lys Met Tyr Glu Val Tyr Asp Ala Lys
               580                 585                 590
   tca aaa tct gtc agt ctc cca gtt cca gac ttg tgt gca gtc tat gct      1824
   Ser Lys Ser Val Ser Leu Pro Val Pro Asp Leu Cys Ala Val Tyr Ala
           595                 600                 605
   gtt cag gtg cgc tgt aag agg cta gat gga ctg gga tat tgg agt aat      1872
   Val Gln Val Arg Cys Lys Arg Leu Asp Gly Leu Gly Tyr Trp Ser Asn
       610                 615                 620
   tgg agc aat cca gcc tac aca gtt gtc atg gat ata aaa gtt cct atg      1920
   Trp Ser Asn Pro Ala Tyr Thr Val Val Met Asp Ile Lys Val Pro Met
   625                 630                 635                 640
   aga gga cct gaa ttt tgg aga ata att aat gga gat act atg aaa aag      1968
   Arg Gly Pro Glu Phe Trp Arg Ile Ile Asn Gly Asp Thr Met Lys Lys
                   645                 650                 655
   gag aaa aat gtc act tta ctt tgg aag ccc ctg atg aaa aat gac tca      2016
   Glu Lys Asn Val Thr Leu Leu Trp Lys Pro Leu Met Lys Asn Asp Ser
               660                 665                 670
   ttg tgc agt gtt cag aga tat gtg ata aac cat cat act tcc tgc aat      2064
   Leu Cys Ser Val Gln Arg Tyr Val Ile Asn His His Thr Ser Cys Asn
           675                 680                 685
   gga aca tgg tca gaa gat gtg gga aat cac acg aaa ttc act ttc ctg      2112
   Gly Thr Trp Ser Glu Asp Val Gly Asn His Thr Lys Phe Thr Phe Leu
       690                 695                 700
   tgg aca gag caa gca cat act gtt acg gtt ctg gcc atc aat tca att      2160
   Trp Thr Glu Gln Ala His Thr Val Thr Val Leu Ala Ile Asn Ser Ile
   705                 710                 715                 720
   ggt gct tct gtt gca aat ttt aat tta acc ttt tca tgg cct atg agc      2208
   Gly Ala Ser Val Ala Asn Phe Asn Leu Thr Phe Ser Trp Pro Met Ser
                   725                 730                 735
   aaa gta aat atc gtg cag tca ctc agt gct tat cct tta aac agc agt      2256
   Lys Val Asn Ile Val Gln Ser Leu Ser Ala Tyr Pro Leu Asn Ser Ser
               740                 745                 750
   tgt gtg att gtt tcc tgg ata cta tca ccc agt gat tac aag cta atg      2304
   Cys Val Ile Val Ser Trp Ile Leu Ser Pro Ser Asp Tyr Lys Leu Met
           755                 760                 765
   tat ttt att att gag tgg aaa aat ctt aat gaa gat ggt gaa ata aaa      2352
   Tyr Phe Ile Ile Glu Trp Lys Asn Leu Asn Glu Asp Gly Glu Ile Lys
       770                 775                 780
   tgg ctt aga atc tct tca tct gtt aag aag tat tat atc cat gat cat      2400
   Trp Leu Arg Ile Ser Ser Ser Val Lys Lys Tyr Tyr Ile His Asp His
   785                 790                 795                 800
   ttt atc ccc att gag aag tac cag ttc agt ctt tac cca ata ttt atg      2448
   Phe Ile Pro Ile Glu Lys Tyr Gln Phe Ser Leu Tyr Pro Ile Phe Met
                   805                 810                 815
   gaa gga gtg gga aaa cca aag ata att aat agt ttc act caa gat gat      2496
   Glu Gly Val Gly Lys Pro Lys Ile Ile Asn Ser Phe Thr Gln Asp Asp
               820                 825                 830
   att gaa aaa cac cag agt gat gca ggt tta tat gta att gtg cca gta      2544
   Ile Glu Lys His Gln Ser Asp Ala Gly Leu Tyr Val Ile Val Pro Val
           835                 840                 845
   att att tcc tct tcc atc tta ttg ctt gga aca tta tta ata tca cac      2592
   Ile Ile Ser Ser Ser Ile Leu Leu Leu Gly Thr Leu Leu Ile Ser His
       850                 855                 860
   caa aga atg aaa aag cta ttt tgg gaa gat gtt ccg aac ccc aag aat      2640
   Gln Arg Met Lys Lys Leu Phe Trp Glu Asp Val Pro Asn Pro Lys Asn
   865                 870                 875                 880
   tgt tcc tgg gca caa gga ctt aat ttt cag aag aga acg gac att ctt      2688
   Cys Ser Trp Ala Gln Gly Leu Asn Phe Gln Lys Arg Thr Asp Ile Leu
                   885                 890                 895
   tga                                                                  2691
<210> 10
   <211> 896
   <212> PRT
   <213> Homo sapiens
<400> 10
   Met Ile Cys Gln Lys Phe Cys Val Val Leu Leu His Trp Glu Phe Ile
   1               5                   10                  15
   Tyr Val Ile Thr Ala Phe Asn Leu Ser Tyr Pro Ile Thr Pro Trp Arg
               20                  25                  30
   Phe Lys Leu Ser Cys Met Pro Pro Asn Ser Thr Tyr Asp Tyr Phe Leu
           35                  40                  45
   Leu Pro Ala Gly Leu Ser Lys Asn Thr Ser Asn Ser Asn Gly His Tyr
       50                  55                  60
   Glu Thr Ala Val Glu Pro Lys Phe Asn Ser Ser Gly Thr His Phe Ser
   65                  70                  75                  80
   Asn Leu Ser Lys Thr Thr Phe His Cys Cys Phe Arg Ser Glu Gln Asp
                   85                  90                  95
   Arg Asn Cys Ser Leu Cys Ala Asp Asn Ile Glu Gly Lys Thr Phe Val
               100                 105                 110
   Ser Thr Val Asn Ser Leu Val Phe Gln Gln Ile Asp Ala Asn Trp Asn
           115                 120                 125
   Ile Gln Cys Trp Leu Lys Gly Asp Leu Lys Leu Phe Ile Cys Tyr Val
   130 135 140
   Glu Ser Leu Phe Lys Asn Leu Phe Arg Asn Tyr Asn Tyr Lys Val His
   145 150 155 160
   Leu Leu Tyr Val Leu Pro Glu Val Leu Glu Asp Ser Pro Leu Val Pro
   165 170 175
   Gln Lys Gly Ser Phe Gln Met Val His Cys Asn Cys Ser Val His Glu
   180 185 190
   Cys Cys Glu Cys Leu Val Pro Val Pro Thr Ala Lys Leu Asn Asp Thr
   195 200 205
   Leu Leu Met Cys Leu Lys Ile Thr Ser Gly Gly Val Ile Phe Gln Ser
   210 215 220
   Pro Leu Met Ser Val Gln Pro Ile Asn Met Val Lys Pro Asp Pro Pro
   225 230 235 240
   Leu Gly Leu His Met Glu Ile Thr Asp Asp Gly Asn Leu Lys Ile Ser
   245 250 255
   Trp Ser Ser Pro Pro Leu Val Pro Phe Pro Leu Gln Tyr Gln Val Lys
   260 265 270
   Tyr Ser Glu Asn Ser Thr Thr Val Ile Arg Glu Ala Asp Lys Ile Val
   275 280 285
   Ser Ala Thr Ser Leu Leu Val Asp Ser Ile Leu Pro Gly Ser Ser Tyr
   290 295 300
   Glu Val Gln Val Arg Gly Lys Arg Leu Asp Gly Pro Gly Ile Trp Ser
   305 310 315 320
   Asp Trp Ser Thr Pro Arg Val Phe Thr Thr Gln Asp Val Ile Tyr Phe
   325 330 335
   Pro Pro Lys Ile Leu Thr Ser Val Gly Ser Asn Val Ser Phe His Cys
   340 345 350
   Ile Tyr Lys Lys Glu Asn Lys Ile Val Pro Ser Lys Glu Ile Val Trp
   355 360 365
   Trp Met Asn Leu Ala Glu Lys Ile Pro Gln Ser Gln Tyr Asp Val Val
   370 375 380
   Ser Asp His Val Ser Lys Val Thr Phe Phe Asn Leu Asn Glu Thr Lys
   385 390 395 400
   Pro Arg Gly Lys Phe Thr Tyr Asp Ala Val Tyr Cys Cys Asn Glu His
   405 410 415
   Glu Cys His His Arg Tyr Ala Glu Leu Tyr Val Ile Asp Val Asn Ile
   420 425 430
   Asn Ile Ser Cys Glu Thr Asp Gly Tyr Leu Thr Lys Met Thr Cys Arg
   435 440 445
   Trp Ser Thr Ser Thr Ile Gln Ser Leu Ala Glu Ser Thr Leu Gln Leu
   450 455 460
   Arg Tyr His Arg Ser Ser Leu Tyr Cys Ser Asp Ile Pro Ser Ile His
   465 470 475 480
   Pro Ile Ser Glu Pro Lys Asp Cys Tyr Leu Gln Ser Asp Gly Phe Tyr
   485 490 495
   Glu Cys Ile Phe Gln Pro Ile Phe Leu Leu Ser Gly Tyr Thr Met Trp
   500 505 510
   Ile Arg Ile Asn His Ser Leu Gly Ser Leu Asp Ser Pro Pro Thr Cys
   515 520 525
   Val Leu Pro Asp Ser Val Val Lys Pro Leu Pro Pro Ser Ser Val Lys
   530 535 540
   Ala Glu Ile Thr Ile Asn Ile Gly Leu Leu Lys Ile Ser Trp Glu Lys
   545 550 555 560
   Pro Val Phe Pro Glu Asn Asn Leu Gln Phe Gln Ile Arg Tyr Gly Leu
   565 570 575
   Ser Gly Lys Glu Val Gln Trp Lys Met Tyr Glu Val Tyr Asp Ala Lys
   580 585 590
   Ser Lys Ser Val Ser Leu Pro Val Pro Asp Leu Cys Ala Val Tyr Ala
   595 600 605
   Val Gln Val Arg Cys Lys Arg Leu Asp Gly Leu Gly Tyr Trp Ser Asn
   610 615 620
   Trp Ser Asn Pro Ala Tyr Thr Val Val Met Asp Ile Lys Val Pro Met
   625 630 635 640
   Arg Gly Pro Glu Phe Trp Arg Ile Ile Asn Gly Asp Thr Met Lys Lys
   645 650 655
   Glu Lys Asn Val Thr Leu Leu Trp Lys Pro Leu Met Lys Asn Asp Ser
   660 665 670
   Leu Cys Ser Val Gln Arg Tyr Val Ile Asn His His Thr Ser Cys Asn
   675 680 685
   Gly Thr Trp Ser Glu Asp Val Gly Asn His Thr Lys Phe Thr Phe Leu
   690 695 700
   Trp Thr Glu Gln Ala His Thr Val Thr Val Leu Ala Ile Asn Ser Ile
   705 710 715 720
   Gly Ala Ser Val Ala Asn Phe Asn Leu Thr Phe Ser Trp Pro Met Ser
   725 730 735
   Lys Val Asn Ile Val Gln Ser Leu Ser Ala Tyr Pro Leu Asn Ser Ser
   740 745 750
   Cys Val Ile Val Ser Trp Ile Leu Ser Pro Ser Asp Tyr Lys Leu Met
   755 760 765
   Tyr Phe Ile Ile Glu Trp Lys Asn Leu Asn Glu Asp Gly Glu Ile Lys
   770 775 780
   Trp Leu Arg Ile Ser Ser Ser Val Lys Lys Tyr Tyr Ile His Asp His
   785 790 795 800
   Phe Ile Pro Ile Glu Lys Tyr Gln Phe Ser Leu Tyr Pro Ile Phe Met
   805 810 815
   Glu Gly Val Gly Lys Pro Lys Ile Ile Asn Ser Phe Thr Gln Asp Asp
   820 825 830
   Ile Glu Lys His Gln Ser Asp Ala Gly Leu Tyr Val Ile Val Pro Val
   835 840 845
   Ile Ile Ser Ser Ser Ile Leu Leu Leu Gly Thr Leu Leu Ile Ser His
   850 855 860
   Gln Arg Met Lys Lys Leu Phe Trp Glu Asp Val Pro Asn Pro Lys Asn
   865 870 875 880
   Cys Ser Trp Ala Gln Gly Leu Asn Phe Gln Lys Arg Thr Asp Ile Leu
   885 890 895
<210> 11
   <211> 3705
   <212> DNA
   <213> Artificial Sequence
<220>
   <223> OBR LUC
<220>
   <221> misc_feature
   <223> OBR LUC
<220>
   <221> CDS
   <222> (1)..(3705)
   <223>
<400> 11
   atg gtt ctt gcc agc tct acc acc agc atc cac acc atg ctg ctc ctg      48
   ctc ctg atg ctc ttc cac ctg gga ctc caa gct tca atc tcg gcg cgc      96
   cag gag cag aag ctt atc tcg gag gag gac ctg acg cgt tat cca att      144
   act cct tgg aga ttt aag ttg tct tgc atg cca cca aat tca acc tat      192
   gac tac ttc ctt ttg cct gct gga ctc tca aag aat act tca aat tcg      240
   aat gga cat tat gag aca gct gtt gaa cct aag ttt aat tca agt ggt      288
   act cac ttt tct aac tta tcc aaa aca act ttc cac tgt tgc ttt cgg      336
   agt gag caa gat aga aac tgc tcc tta tgt gca gac aac att gaa gga      384
   acg aca ttt gtt tca aca gta aat tct tta gtt ttt caa caa ata gat      432
   gca aac tgg aac ata cag tgc tgg cta aaa gga gac tta aaa tta ttc      480
   atc tgt tat gtg gag tca tta ttt aag aat cta ttc agg aat tat aac      528
   tat aag gtc cat ctt tta tat gtt ctg cct gaa gtg tta gaa gat tca      576
   cct ctg gtt ccc caa aaa ggc agt ttt cag atg gtt cac tgc aat tgc      624
   agt gtt cat gaa tgt tgt gaa tgt ctt gtg cct gtg cca aca gcc aaa      672
   ctc aac gac act ctc ctt atg tgt ttg aaa atc aca tct ggt gga gta      720
   att ttc cgg tca cct cta atg tca gtt cag ccc ata aat atg gtg aag      768
   cct gat cca cca tta ggt ttg cat atg gaa atc aca gat gat ggt aat      816
   tta aag att tct tgg tcc agc cca cca ttg gta cca ttt cca ctt caa      864
   tat caa gtg aaa tat tca gag aat tct aca aca gtt atc aga gaa gct      912
   gac aag att gtc tca gct aca tcc ctg cta gta gac agt ata ctt cct      960
   ggg tct tcg tat gag gtt cag gtg agg ggc aag aga ctg gat ggc cca      1008
   gga atc tgg agt gac tgg agt act cct cgt gtc ttt acc aca caa gat      1056
   gtc ata tac ttt cca cct aaa att ctg aca agt gtt ggg tct aat gtt      1104
   tct ttt cac tgc atc tat aag aag gaa aac aag att gtt ccc tca aaa      1152
   gag att gtt tgg tgg atg aat tta gct gag aaa att cct caa agc cag      1200
   tat gat gtt gtg agt gat cat gtt agc aaa gtt act ttt ttc aat ctg      1248
   aat gaa acc aaa cct cga gga aag ttt acc tat gat gca gtg tac tgc      1296
   tgc aat gaa cat gaa tgc cat cat cgc tat gct gaa tta tat gtg att      1344
   gat gtc aat atc aat atc tca tgt gaa act gat ggg tac tta act aaa      1392
   atg act tgc aga tgg tca acc agt aca atc cag tca ctt gcg gaa agc      1440
   act ttg caa ttg agg tat cat agg agc agc ctt tac tgt tct gat att      1488
   cca tct att cat ccc ata tct gag ccc aaa gat tgc tat ttg cag agt      1536
   gat ggt ttt tat gaa tgc att ttc cag cca atc ttc cta tta tct ggc      1584
   tac aca atg tgg att agg atc aat cac tct cta ggt tca ctt gac tct      1632
   cca cca aca tgt gtc ctt cct gat tct gtg gtg aag cca ctg cct cca      1680
   tcc agt gtg aaa gca gaa att act ata aac att gga tta ttg aaa ata      1728
   tct tgg gaa aag cca gtc ttt cca gag aat aac ctt caa ttc cag att      1776
   cgc tat ggt tta agt gga aaa gaa gta caa tgg aag atg tat gag gtt      1824
   tat gat gca aaa tca aaa tct gtc agt ctc cca gtt cca gac ttg tgt      1872
   gca gtc tat gct gtt cag gtg cgc tgt aag agg cta gat gga ctg gga      1920
   tat tgg agt aat tgg agc aat cca gcc tac aca gtt gtc atg gat ata      1968
   aaa gtt cct atg aga gga cct gaa ttt tgg aga ata att aat gga gat      2016
   act atg aaa aag gag aaa aat gtc act tta ctt tgg aag ccc ctg atg      2064
   aaa aat gac tca ttg tgc agt gtt cag aga tat gtg ata aac cat cat      2112
   act tcc tgc aat gga aca tgg tca gaa gat gtg gga aat cac acg aaa      2160
   ttc act ttc ctg tgg aca gag caa gca cat act gtt acg gtt ctg gcc      2208
   atc aat tca att ggt gct tct gtt gca aat ttt aat tta acc ttt tca     2256
   tgg cct atg agc aaa gta aat atc gtg cag tca ctc agt gct tat cct     2304
   tta aac agc agt tgt gtg att gtt tcc tgg ata cta tca ccc agt gat     2352
   tac aag cta atg tat ttt att att gag tgg aaa aat ctt aat gaa gat     2400
   ggt gaa ata aaa tgg ctt aga atc tct tca tct gtt aag aag tat tat     2448
   atc cat gat cat ttt atc ccc att gag aag tac cag ttc agt ctt tac     2496
   cca ata ttt atg gaa gga gtg gga aaa cca aag ata att aat agt ttc     2544
   act caa gat gat att gaa aaa cac cag agt gat gca ggt tta tat gta     2592
   att gtg cca gta att att tcc tct tcc atc tta ttg ctt gga aca tta     2640
   tta ata tca cac caa aga atg aaa aag cta ttt tgg gaa gat gtt ccg     2688
   aac ccc aag aat tgt tcc tgg gca caa gga ctt aat ttt cag aag aga     2736
   acg gac att ctg gat cca ccg gct aga gcc acc atg acc agc aag gtg     2784
   tac gac ccc gag cag agg aag agg atg atc acc ggc ccc cag tgg tgg     2832
   gcc agg tgc aag cag atg aac gtg ctg gac agc ttc atc aac tac tac     2880
   gac agc gag aag cac gcc gag aac gcc gtg atc ttc ctg cac ggc aac     2928
   gcc gct agc agc tac ctg tgg agg cac gtg gtg ccc cac atc gag ccc     2976
   gtg gcc agg tgc atc atc ccc gat ctg atc ggc atg ggc aag agc ggc     3024
   aag agc  ggc aac ggc agc tac  agg ctg ctg gac cac  tac aag tac      3069
   ctg acc  gcc tgg ttc gag ctc  ctg aac ctg ccc aag  aag atc atc      3114
   ttc gtg  ggc cac gac tgg ggc  gcc tgc ctg gcc ttc  cac tac agc      3159
   tac gag  cac cag gac aag atc  aag gcc atc gtg cac  gcc gag agc      3204
   gtg gtg  gac gtg atc gag agc  tgg gac gag tgg cca  gac atc gag      3249
   gag gac  atc gcc ctg atc aag  agc gag gag ggc gag  aag atg gtg      3294
   ctg gag  aac aac ttc ttc gtg  gag acc atg ctg ccc  agc aag atc      3339
   atg aga  aag ctg gag ccc gag  gag ttc gcc gcc tac  ctg gag ccc      3384
   ttc aag  gag aag ggc gag gtg  aga aga ccc acc ctg  agc tgg ccc      3429
   aga gag  atc ccc ctg gtg aag  ggc ggc aag ccc gac  gtg gtg cag      3474
   atc gtg  aga aac tac aac gcc  tac ctg aga gcc agc  gac gac ctg      3519
   ccc aag  atg ttc atc gag agc  gac ccc ggc ttc ttc  agc aac gcc      3564
   atc gtg  gag ggc gcc aag aag  ttc ccc aac acc gag  ttc gtg aag      3609
   gtg aag  ggc ctg cac ttc agc  cag gag gac gcc ccc  gac gag atg      3654
   ggc aag  tac atc aag agc ttc  gtg gag aga gtg ctg  aag aac gag      3699
   cag taa                                                             3705
<210> 12
   <211> 1234
   <212> PRT
   <213> Artificial Sequence
<220>
   <223> OBR LUC
<220>
   <221> misc_feature
   <223> OBR LUC
<400> 12
<210> 13
   <211> 3486
   <212> DNA
   <213> Artificial Sequence
<220>
   <223> OBR YFP
<220>
   <221> misc_feature
   <223> OBR YFP
<220>
   <221> CDS
   <222> (1)..(3486)
   <223>
<400> 13
   atg gtt ctt gcc agc tct acc acc agc atc cac acc atg ctg ctc ctg      48
   Met Val Leu Ala Ser Ser Thr Thr Ser Ile His Thr Met Leu Leu Leu
   1               5                   10                  15
   ctc ctg atg ctc ttc cac ctg gga ctc caa gct tca atc tcg gcg cgc      96
   Leu Leu Met Leu Phe His Leu Gly Leu Gln Ala Ser Ile Ser Ala Arg
               20                  25                  30
   cag gag cag aag ctt atc tcg gag gag gac ctg acg cgt tat cca att     144
   Gln Glu Gln Lys Leu Ile Ser Glu Glu Asp Leu Thr Arg Tyr Pro Ile
           35                  40                  45
   act cct tgg aga ttt aag ttg tct tgc atg cca cca aat tca acc tat     192
   Thr Pro Trp Arg Phe Lys Leu Ser Cys Met Pro Pro Asn Ser Thr Tyr
       50                  55                  60
   gac tac ttc ctt ttg cct gct gga ctc tca aag aat act tca aat tcg     240
   Asp Tyr Phe Leu Leu Pro Ala Gly Leu Ser Lys Asn Thr Ser Asn Ser
   65                  70                  75                  80
   aat gga cat tat gag aca gct gtt gaa cct aag ttt aat tca agt ggt     288
   Asn Gly His Tyr Glu Thr Ala Val Glu Pro Lys Phe Asn Ser Ser Gly
                   85                  90                  95
   act cac ttt tct aac tta tcc aaa aca act ttc cac tgt tgc ttt cgg     336
   Thr His Phe Ser Asn Leu Ser Lys Thr Thr Phe His Cys Cys Phe Arg
               100                 105                 110
   agt gag caa gat aga aac tgc tcc tta tgt gca gac aac att gaa gga     384
   Ser Glu Gln Asp Arg Asn Cys Ser Leu Cys Ala Asp Asn Ile Glu Gly
           115                 120                 125
   acg aca ttt gtt tca aca gta aat tct tta gtt ttt caa caa ata gat     432
   Thr Thr Phe Val Ser Thr Val Asn Ser Leu Val Phe Gln Gln Ile Asp
       130                 135                 140
   gca aac tgg aac ata cag tgc tgg cta aaa gga gac tta aaa tta ttc     480
   Ala Asn Trp Asn Ile Gln Cys Trp Leu Lys Gly Asp Leu Lys Leu Phe
   145                 150                 155                 160
   atc tgt tat gtg gag tca tta ttt aag aat cta ttc agg aat tat aac      528
   Ile Cys Tyr Val Glu Ser Leu Phe Lys Asn Leu Phe Arg Asn Tyr Asn
                   165                 170                 175
   tat aag gtc cat ctt tta tat gtt ctg cct gaa gtg tta gaa gat tca      576
   Tyr Lys Val His Leu Leu Tyr Val Leu Pro Glu Val Leu Glu Asp Ser
               180                 185                 190
   cct ctg gtt ccc caa aaa ggc agt ttt cag atg gtt cac tgc aat tgc      624
   Pro Leu Val Pro Gln Lys Gly Ser Phe Gln Met Val His Cys Asn Cys
           195                 200                 205
   agt gtt cat gaa tgt tgt gaa tgt ctt gtg cct gtg cca aca gcc aaa      672
   Ser Val His Glu Cys Cys Glu Cys Leu Val Pro Val Pro Thr Ala Lys
       210                 215                 220
   ctc aac gac act ctc ctt atg tgt ttg aaa atc aca tct ggt gga gta      720
   Leu Asn Asp Thr Leu Leu Met Cys Leu Lys Ile Thr Ser Gly Gly Val
   225                 230                 235                 240
   att ttc cgg tca cct cta atg tca gtt cag ccc ata aat atg gtg aag      768
   Ile Phe Arg Ser Pro Leu Met Ser Val Gln Pro Ile Asn Met Val Lys
                   245                 250                 255
   cct gat cca cca tta ggt ttg cat atg gaa atc aca gat gat ggt aat      816
   Pro Asp Pro Pro Leu Gly Leu His Met Glu Ile Thr Asp Asp Gly Asn
               260                 265                 270
   tta aag att tct tgg tcc agc cca cca ttg gta cca ttt cca ctt caa      864
   Leu Lys Ile Ser Trp Ser Ser Pro Pro Leu Val Pro Phe Pro Leu Gln
           275                 280                 285
   tat caa gtg aaa tat tca gag aat tct aca aca gtt atc aga gaa gct      912
   Tyr Gln Val Lys Tyr Ser Glu Asn Ser Thr Thr Val Ile Arg Glu Ala
       290                 295                 300
   gac aag att gtc tca gct aca tcc ctg cta gta gac agt ata ctt cct      960
   Asp Lys Ile Val Ser Ala Thr Ser Leu Leu Val Asp Ser Ile Leu Pro
   305                 310                 315                 320
   ggg tct tcg tat gag gtt cag gtg agg ggc aag aga ctg gat ggc cca     1008
   Gly Ser Ser Tyr Glu Val Gln Val Arg Gly Lys Arg Leu Asp Gly Pro
                   325                 330                 335
   gga atc tgg agt gac tgg agt act cct cgt gtc ttt acc aca caa gat     1056
   Gly Ile Trp Ser Asp Trp Ser Thr Pro Arg Val Phe Thr Thr Gln Asp
               340                 345                 350
   gtc ata tac ttt cca cct aaa att ctg aca agt gtt ggg tct aat gtt     1104
   Val Ile Tyr Phe Pro Pro Lys Ile Leu Thr Ser Val Gly Ser Asn Val
           355                 360                 365
   tct ttt cac tgc atc tat aag aag gaa aac aag att gtt ccc tca aaa     1152
   Ser Phe His Cys Ile Tyr Lys Lys Glu Asn Lys Ile Val Pro Ser Lys
       370                 375                 380
   gag att gtt tgg tgg atg aat tta gct gag aaa att cct caa agc cag     1200
   Glu Ile Val Trp Trp Met Asn Leu Ala Glu Lys Ile Pro Gln Ser Gln
   385                 390                 395                 400
   tat gat gtt gtg agt gat cat gtt agc aaa gtt act ttt ttc aat ctg     1248
   Tyr Asp Val Val Ser Asp His Val Ser Lys Val Thr Phe Phe Asn Leu
                   405                 410                 415
   aat gaa acc aaa cct cga gga aag ttt acc tat gat gca gtg tac tgc     1296
   Asn Glu Thr Lys Pro Arg Gly Lys Phe Thr Tyr Asp Ala Val Tyr Cys
               420                 425                 430
   tgc aat gaa cat gaa tgc cat cat cgc tat gct gaa tta tat gtg att      1344
   Cys Asn Glu His Glu Cys His His Arg Tyr Ala Glu Leu Tyr Val Ile
           435                 440                 445
   gat gtc aat atc aat atc tca tgt gaa act gat ggg tac tta act aaa      1392
   Asp Val Asn Ile Asn Ile Ser Cys Glu Thr Asp Gly Tyr Leu Thr Lys
       450                 455                 460
   atg act tgc aga tgg tca acc agt aca atc cag tca ctt gcg gaa agc      1440
   Met Thr Cys Arg Trp Ser Thr Ser Thr Ile Gln Ser Leu Ala Glu Ser
   465                 470                 475                 480
   act ttg caa ttg agg tat cat agg agc agc ctt tac tgt tct gat att      1488
   Thr Leu Gln Leu Arg Tyr His Arg Ser Ser Leu Tyr Cys Ser Asp Ile
                   485                 490                 495
   cca tct att cat ccc ata tct gag ccc aaa gat tgc tat ttg cag agt      1536
   Pro Ser Ile His Pro Ile Ser Glu Pro Lys Asp Cys Tyr Leu Gln Ser
               500                 505                 510
   gat ggt ttt tat gaa tgc att ttc cag cca atc ttc cta tta tct ggc      1584
   Asp Gly Phe Tyr Glu Cys Ile Phe Gln Pro Ile Phe Leu Leu Ser Gly
           515                 520                 525
   tac aca atg tgg att agg atc aat cac tct cta ggt tca ctt gac tct      1632
   Tyr Thr Met Trp Ile Arg Ile Asn His Ser Leu Gly Ser Leu Asp Ser
       530                 535                 540
   cca cca aca tgt gtc ctt cct gat tct gtg gtg aag cca ctg cct cca      1680
   Pro Pro Thr Cys Val Leu Pro Asp Ser Val Val Lys Pro Leu Pro Pro
   545                 550                 555                 560
   tcc agt gtg aaa gca gaa att act ata aac att gga tta ttg aaa ata      1728
   Ser Ser Val Lys Ala Glu Ile Thr Ile Asn Ile Gly Leu Leu Lys Ile
                   565                 570                 575
   tct tgg gaa aag cca gtc ttt cca gag aat aac ctt caa ttc cag att      1776
   Ser Trp Glu Lys Pro Val Phe Pro Glu Asn Asn Leu Gln Phe Gln Ile
               580                 585                 590
   cgc tat ggt tta agt gga aaa gaa gta caa tgg aag atg tat gag gtt      1824
   Arg Tyr Gly Leu Ser Gly Lys Glu Val Gln Trp Lys Met Tyr Glu Val
           595                 600                 605
   tat gat gca aaa tca aaa tct gtc agt ctc cca gtt cca gac ttg tgt      1872
   Tyr Asp Ala Lys Ser Lys Ser Val Ser Leu Pro Val Pro Asp Leu Cys
       610                 615                 620
   gca gtc tat gct gtt cag gtg cgc tgt aag agg cta gat gga ctg gga      1920
   Ala Val Tyr Ala Val Gln Val Arg Cys Lys Arg Leu Asp Gly Leu Gly
   625                 630                 635                 640
   tat tgg agt aat tgg agc aat cca gcc tac aca gtt gtc atg gat ata      1968
   Tyr Trp Ser Asn Trp Ser Asn Pro Ala Tyr Thr Val Val Met Asp Ile
                   645                 650                 655
   aaa gtt cct atg aga gga cct gaa ttt tgg aga ata att aat gga gat      2016
   Lys Val Pro Met Arg Gly Pro Glu Phe Trp Arg Ile Ile Asn Gly Asp
               660                 665                 670
   act atg aaa aag gag aaa aat gtc act tta ctt tgg aag ccc ctg atg      2064
   Thr Met Lys Lys Glu Lys Asn Val Thr Leu Leu Trp Lys Pro Leu Met
           675                 680                 685
   aaa aat gac tca ttg tgc agt gtt cag aga tat gtg ata aac cat cat      2112
   Lys Asn Asp Ser Leu Cys Ser Val Gln Arg Tyr Val Ile Asn His His
   690 695 700
   act tcc tgc aat gga aca tgg tca gaa gat gtg gga aat cac acg aaa 2160
   Thr Ser Cys Asn Gly Thr Trp Ser Glu Asp Val Gly Asn His Thr Lys
   705 710 715 720
   ttc act ttc ctg tgg aca gag caa gca cat act gtt acg gtt ctg gcc 2208
   Phe Thr Phe Leu Trp Thr Glu Gln Ala His Thr Val Thr Val Leu Ala
   725 730 735
   atc aat tca att ggt gct tct gtt gca aat ttt aat tta acc ttt tca 2256
   Ile Asn Ser Ile Gly Ala Ser Val Ala Asn Phe Asn Leu Thr Phe Ser
   740 745 750
   tgg cct atg agc aaa gta aat atc gtg cag tca ctc agt gct tat cct 2304
   Trp Pro Met Ser Lys Val Asn Ile Val Gln Ser Leu Ser Ala Tyr Pro
   755 760 765
   tta aac agc agt tgt gtg att gtt tcc tgg ata cta tca ccc agt gat 2352
   Leu Asn Ser Ser Cys Val Ile Val Ser Trp Ile Leu Ser Pro Ser Asp
   770 775 780
   tac aag cta atg tat ttt att att gag tgg aaa aat ctt aat gaa gat 2400
   Tyr Lys Leu Met Tyr Phe Ile Ile Glu Trp Lys Asn Leu Asn Glu Asp
   785 790 795 800
   ggt gaa ata aaa tgg ctt aga atc tct tca tct gtt aag aag tat tat 2448
   Gly Glu Ile Lys Trp Leu Arg Ile Ser Ser Ser Val Lys Lys Tyr Tyr
   805 810 815
   atc cat gat cat ttt atc ccc att gag aag tac cag ttc agt ctt tac 2496
   Ile His Asp His Phe Ile Pro Ile Glu Lys Tyr Gln Phe Ser Leu Tyr
   820 825 830
   cca ata ttt atg gaa gga gtg gga aaa cca aag ata att aat agt ttc 2544
   Pro Ile Phe Met Glu Gly Val Gly Lys Pro Lys Ile Ile Asn Ser Phe
   835 840 845
   act caa gat gat att gaa aaa cac cag agt gat gca ggt tta tat gta 2592
   Thr Gln Asp Asp Ile Glu Lys His Gln Ser Asp Ala Gly Leu Tyr Val
   850 855 860
   att gtg cca gta att att tcc tct tcc atc tta ttg ctt gga aca tta 2640
   Ile Val Pro Val Ile Ile Ser Ser Ser Ile Leu Leu Leu Gly Thr Leu
   865 870 875 880
   tta ata tca cac caa aga atg aaa aag cta ttt tgg gaa gat gtt ccg 2688
   Leu Ile Ser His Gln Arg Met Lys Lys Leu Phe Trp Glu Asp Val Pro
   885 890 895
   aac ccc aag aat tgt tcc tgg gca caa gga ctt aat ttt cag aag aga 2736
   Asn Pro Lys Asn Cys Ser Trp Ala Gln Gly Leu Asn Phe Gln Lys Arg
   900 905 910
   acg gac att ctg gat cca ccg gtc gcc acc atg gtg agc aag ggc gag 2784
   Thr Asp Ile Leu Asp Pro Pro Val Ala Thr Met Val Ser Lys Gly Glu
   915 920 925
   gag ctg ttc acc ggg gtg gtg ccc atc ctg gtc gag ctg gac ggc gac 2832
   Glu Leu Phe Thr Gly Val Val Pro Ile Leu Val Glu Leu Asp Gly Asp
   930 935 940
   gta aac ggc cac aag ttc agc gtg tcc ggc gag ggc gag ggc gat gcc 2880
   Val Asn Gly His Lys Phe Ser Val Ser Gly Glu Gly Glu Gly Asp Ala
   945 950 955 960
   acc tac ggc aag ctg acc ctg aag ttc atc tgc acc acc ggc aag ctg 2928
   Thr Tyr Gly Lys Leu Thr Leu Lys Phe Ile Cys Thr Thr Gly Lys Leu
                   965                 970                 975
   ccc gtg ccc tgg ccc acc ctc gtg acc acc ttc ggc tac ggc gtg cag     2976
   Pro Val Pro Trp Pro Thr Leu Val Thr Thr Phe Gly Tyr Gly Val Gln
               980                 985                 990
   tgc ttc gcc cgc tac ccc gac cac  atg cgc cag cac gac  ttc ttc aag   3024
   Cys Phe Ala Arg Tyr Pro Asp His  Met Arg Gln His Asp  Phe Phe Lys
           995                 1000                 1005
   tcc gcc  atg ccc gaa ggc tac  gtc cag gag cgc acc  atc ttc ttc      3069
   Ser Ala  Met Pro Glu Gly Tyr  Val Gln Glu Arg Thr  Ile Phe Phe
       1010                 1015                 1020
   aag gac  gac ggc aac tac aag  acc cgc gcc gag gtg  aag ttc gag      3114
   Lys Asp  Asp Gly Asn Tyr Lys  Thr Arg Ala Glu Val  Lys Phe Glu
       1025                 1030                 1035
   ggc gac  acc ctg gtg aac cgc  atc gag ctg aag ggc  atc gac ttc      3159
   Gly Asp  Thr Leu Val Asn Arg  Ile Glu Leu Lys Gly  Ile Asp Phe
       1040                 1045                 1050
   aag gag  gac ggc aac atc ctg  ggg cac aag ctg gag  tac aac tac      3204
   Lys Glu  Asp Gly Asn Ile Leu  Gly His Lys Leu Glu  Tyr Asn Tyr
       1055                 1060                 1065
   aac agc  cac aac gtc tat atc  atg gcc gac aag cag  aag aac ggc      3249
   Asn Ser  His Asn Val Tyr Ile  Met Ala Asp Lys Gln  Lys Asn Gly
       1070                 1075                 1080
   atc aag  gtg aac ttc aag atc  cgc cac aac atc gag  gac ggc agc      3294
   Ile Lys  Val Asn Phe Lys Ile  Arg His Asn Ile Glu  Asp Gly Ser
       1085                 1090                 1095
   gtg cag  ctc gcc gac cac tac  cag cag aac acc ccc  atc ggc gac      3339
   Val Gln  Leu Ala Asp His Tyr  Gln Gln Asn Thr Pro  Ile Gly Asp
       1100                 1105                 1110
   ggc ccc  gtg ctg ctg ccc gac  aac cac tac ctg agc  tac cag tcc      3384
   Gly Pro  Val Leu Leu Pro Asp  Asn His Tyr Leu Ser  Tyr Gln Ser
       1115                 1120                 1125
   gcc ctg  agc aaa gac ccc aac  gag aag cgc gat cac  atg gtc ctg      3429
   Ala Leu  Ser Lys Asp Pro Asn  Glu Lys Arg Asp His  Met Val Leu
       1130                 1135                 1140
   ctg gag  ttc gtg acc gcc gcc  ggg atc act ctc ggc  atg gac gag      3474
   Leu Glu  Phe Val Thr Ala Ala  Gly Ile Thr Leu Gly  Met Asp Glu
       1145                 1150                 1155
   ctg tac  aag taa                                                    3486
   Leu Tyr  Lys
       1160
<210> 14
   <211> 1161
   <212> PRT
   <213> Artificial Sequence
<220>
   <223> OBR YFP
<220>
   <221> misc_feature
   <223> OBR YFP
<400> 14
   Met Val Leu Ala Ser Ser Thr Thr Ser Ile His Thr Met Leu Leu Leu
   1               5                   10                  15
   Leu Leu Met Leu Phe His Leu Gly Leu Gln Ala Ser Ile Ser Ala Arg
               20                  25                  30
   Gln Glu Gln Lys Leu Ile Ser Glu Glu Asp Leu Thr Arg Tyr Pro Ile
           35                  40                  45
   Thr Pro Trp Arg Phe Lys Leu Ser Cys Met Pro Pro Asn Ser Thr Tyr
       50                  55                  60
   Asp Tyr Phe Leu Leu Pro Ala Gly Leu Ser Lys Asn Thr Ser Asn Ser
   65                  70                  75                  80
   Asn Gly His Tyr Glu Thr Ala Val Glu Pro Lys Phe Asn Ser Ser Gly
                   85                  90                  95
   Thr His Phe Ser Asn Leu Ser Lys Thr Thr Phe His Cys Cys Phe Arg
               100                 105                 110
   Ser Glu Gln Asp Arg Asn Cys Ser Leu Cys Ala Asp Asn Ile Glu Gly
           115                 120                 125
   Thr Thr Phe Val Ser Thr Val Asn Ser Leu Val Phe Gln Gln Ile Asp
       130                 135                 140
   Ala Asn Trp Asn Ile Gln Cys Trp Leu Lys Gly Asp Leu Lys Leu Phe
   145                 150                 155                 160
   Ile Cys Tyr Val Glu Ser Leu Phe Lys Asn Leu Phe Arg Asn Tyr Asn
                   165                 170                 175
   Tyr Lys Val His Leu Leu Tyr Val Leu Pro Glu Val Leu Glu Asp Ser
               180                 185                 190
   Pro Leu Val Pro Gln Lys Gly Ser Phe Gln Met Val His Cys Asn Cys
           195                 200                 205
   Ser Val His Glu Cys Cys Glu Cys Leu Val Pro Val Pro Thr Ala Lys
       210                 215                 220
   Leu Asn Asp Thr Leu Leu Met Cys Leu Lys Ile Thr Ser Gly Gly Val
   225                 230                 235                 240
   Ile Phe Arg Ser Pro Leu Met Ser Val Gln Pro Ile Asn Met Val Lys
   245 250 255
   Pro Asp Pro Pro Leu Gly Leu His Met Glu Ile Thr Asp Asp Gly Asn
   260 265 270
   Leu Lys Ile Ser Trp Ser Ser Pro Pro Leu Val Pro Phe Pro Leu Gln
   275 280 285
   Tyr Gln Val Lys Tyr Ser Glu Asn Ser Thr Thr Val Ile Arg Glu Ala
   290 295 300
   Asp Lys Ile Val Ser Ala Thr Ser Leu Leu Val Asp Ser Ile Leu Pro
   305 310 315 320
   Gly Ser Ser Tyr Glu Val Gln Val Arg Gly Lys Arg Leu Asp Gly Pro
   325 330 335
   Gly Ile Trp Ser Asp Trp Ser Thr Pro Arg Val Phe Thr Thr Gln Asp
   340 345 350
   Val Ile Tyr Phe Pro Pro Lys Ile Leu Thr Ser Val Gly Ser Asn Val
   355 360 365
   Ser Phe His Cys Ile Tyr Lys Lys Glu Asn Lys Ile Val Pro Ser Lys
   370 375 380
   Glu Ile Val Trp Trp Met Asn Leu Ala Glu Lys Ile Pro Gln Ser Gln
   385 390 395 400
   Tyr Asp Val Val Ser Asp His Val Ser Lys Val Thr Phe Phe Asn Leu
   405 410 415
   Asn Glu Thr Lys Pro Arg Gly Lys Phe Thr Tyr Asp Ala Val Tyr Cys
   420 425 430
   Cys Asn Glu His Glu Cys His His Arg Tyr Ala Glu Leu Tyr Val Ile
   435 440 445
   Asp Val Asn Ile Asn Ile Ser Cys Glu Thr Asp Gly Tyr Leu Thr Lys
   450 455 460
   Met Thr Cys Arg Trp Ser Thr Ser Thr Ile Gln Ser Leu Ala Glu Ser
   465 470 475 480
   Thr Leu Gln Leu Arg Tyr His Arg Ser Ser Leu Tyr Cys Ser Asp Ile
   485 490 495
   Pro Ser Ile His Pro Ile Ser Glu Pro Lys Asp Cys Tyr Leu Gln Ser
   500 505 510
   Asp Gly Phe Tyr Glu Cys Ile Phe Gln Pro Ile Phe Leu Leu Ser Gly
   515 520 525
   Tyr Thr Met Trp Ile Arg Ile Asn His Ser Leu Gly Ser Leu Asp Ser
   530 535 540
   Pro Pro Thr Cys Val Leu Pro Asp Ser Val Val Lys Pro Leu Pro Pro
   545 550 555 560
   Ser Ser Val Lys Ala Glu Ile Thr Ile Asn Ile Gly Leu Leu Lys Ile
   565 570 575
   Ser Trp Glu Lys Pro Val Phe Pro Glu Asn Asn Leu Gln Phe Gln Ile
   580 585 590
   Arg Tyr Gly Leu Ser Gly Lys Glu Val Gln Trp Lys Met Tyr Glu Val
   595 600 605
   Tyr Asp Ala Lys Ser Lys Ser Val Ser Leu Pro Val Pro Asp Leu Cys
   610 615 620
   Ala Val Tyr Ala Val Gln Val Arg Cys Lys Arg Leu Asp Gly Leu Gly
   625 630 635 640
   Tyr Trp Ser Asn Trp Ser Asn Pro Ala Tyr Thr Val Val Met Asp Ile
   645 650 655
   Lys Val Pro Met Arg Gly Pro Glu Phe Trp Arg Ile Ile Asn Gly Asp
   660 665 670
   Thr Met Lys Lys Glu Lys Asn Val Thr Leu Leu Trp Lys Pro Leu Met
   675 680 685
   Lys Asn Asp Ser Leu Cys Ser Val Gln Arg Tyr Val Ile Asn His His
   690 695 700
   Thr Ser Cys Asn Gly Thr Trp Ser Glu Asp Val Gly Asn His Thr Lys
   705 710 715 720
   Phe Thr Phe Leu Trp Thr Glu Gln Ala His Thr Val Thr Val Leu Ala
   725 730 735
   Ile Asn Ser Ile Gly Ala Ser Val Ala Asn Phe Asn Leu Thr Phe Ser
   740 745 750
   Trp Pro Met Ser Lys Val Asn Ile Val Gln Ser Leu Ser Ala Tyr Pro
   755 760 765
   Leu Asn Ser Ser Cys Val Ile Val Ser Trp Ile Leu Ser Pro Ser Asp
   770 775 780
   Tyr Lys Leu Met Tyr Phe Ile Ile Glu Trp Lys Asn Leu Asn Glu Asp
   785                 790                 795                 800
   Gly Glu Ile Lys Trp Leu Arg Ile Ser Ser Ser Val Lys Lys Tyr Tyr
                   805                 810                 815
   Ile His Asp His Phe Ile Pro Ile Glu Lys Tyr Gln Phe Ser Leu Tyr
               820                 825                 830
   Pro Ile Phe Met Glu Gly Val Gly Lys Pro Lys Ile Ile Asn Ser Phe
           835                 840                 845
   Thr Gln Asp Asp Ile Glu Lys His Gln Ser Asp Ala Gly Leu Tyr Val
       850                 855                 860
   Ile Val Pro Val Ile Ile Ser Ser Ser Ile Leu Leu Leu Gly Thr Leu
   865                 870                 875                 880
   Leu Ile Ser His Gln Arg Met Lys Lys Leu Phe Trp Glu Asp Val Pro
                   885                 890                 895
   Asn Pro Lys Asn Cys Ser Trp Ala Gln Gly Leu Asn Phe Gln Lys Arg
               900                 905                 910
   Thr Asp Ile Leu Asp Pro Pro Val Ala Thr Met Val Ser Lys Gly Glu
           915                 920                 925
   Glu Leu Phe Thr Gly Val Val Pro Ile Leu Val Glu Leu Asp Gly Asp
       930                 935                 940
   Val Asn Gly His Lys Phe Ser Val Ser Gly Glu Gly Glu Gly Asp Ala
   945                 950                 955                 960
   Thr Tyr Gly Lys Leu Thr Leu Lys Phe Ile Cys Thr Thr Gly Lys Leu
                   965                 970                 975
   Pro Val Pro Trp Pro Thr Leu Val Thr Thr Phe Gly Tyr Gly Val Gln
               980                 985                 990
   Cys Phe Ala Arg Tyr Pro Asp His  Met Arg Gln His Asp  Phe Phe Lys
           995                 1000                 1005
   Ser Ala  Met Pro Glu Gly Tyr  Val Gln Glu Arg Thr  Ile Phe Phe
       1010                 1015                 1020
   Lys Asp  Asp Gly Asn Tyr Lys  Thr Arg Ala Glu Val  Lys Phe Glu
       1025                 1030                 1035
   Gly Asp  Thr Leu Val Asn Arg  Ile Glu Leu Lys Gly  Ile Asp Phe
       1040                 1045                 1050
   Lys Glu Asp Gly Asn Ile Leu Gly His Lys Leu Glu Tyr Asn Tyr
   1055 1060 1065
   Asn Ser His Asn Val Tyr Ile Met Ala Asp Lys Gln Lys Asn Gly
   1070 1075 1080
   Ile Lys Val Asn Phe Lys Ile Arg His Asn Ile Glu Asp Gly Ser
   1085 1090 1095
   Val Gln Leu Ala Asp His Tyr Gln Gln Asn Thr Pro Ile Gly Asp
   1100 1105 1110
   Gly Pro Val Leu Leu Pro Asp Asn His Tyr Leu Ser Tyr Gln Ser
   1115 1120 1125
   Ala Leu Ser Lys Asp Pro Asn Glu Lys Arg Asp His Met Val Leu
   1130 1135 1140
   Leu Glu Phe Val Thr Ala Ala Gly Ile Thr Leu Gly Met Asp Glu
   1145 1150 1155
   Leu Tyr Lys
   1160
<210> 15
   <211> 396
   <212> DNA
   <213> Homo sapiens
<220>
   <221> CDS
   <222> (1)..(396)
   <223>
<400> 15
   atg gca ggc atc aaa gct ttg att agt ttg tcc ttt gga gga gca atc 48
   Met Ala Gly Ile Lys Ala Leu Ile Ser Leu Ser Phe Gly Gly Ala Ile
   1 5 10 15
   gga ctg atg ttt ttg atg ctt gga tgt gcc ctt cca ata tac aac aaa 96
   Gly Leu Met Phe Leu Met Leu Gly Cys Ala Leu Pro Ile Tyr Asn Lys
   20 25 30
   tac tgg ccc ctc ttt gtt cta ttt ttt tac atc ctt tca cct att cca 144
   Tyr Trp Pro Leu Phe Val Leu Phe Phe Tyr Ile Leu Ser Pro Ile Pro
   35 40 45
   tac tgc ata gca aga aga tta gtg gat gat aca gat gct atg agt aac 192
   Tyr Cys Ile Ala Arg Arg Leu Val Asp Asp Thr Asp Ala Met Ser Asn
       50                  55                  60
   gct tgt aag gaa ctt gcc atc ttt ctt aca acg ggc att gtc gtg tca      240
   Ala Cys Lys Glu Leu Ala Ile Phe Leu Thr Thr Gly Ile Val Val Ser
   65                  70                  75                  80
   gct ttt gga ctc cct att gta ttt gcc aga gca cat ctg att gag tgg      288
   Ala Phe Gly Leu Pro Ile Val Phe Ala Arg Ala His Leu Ile Glu Trp
                   85                  90                  95
   gga gct tgt gca ctt gtt ctc aca gga aac aca gtc atc ttt gca act      336
   Gly Ala Cys Ala Leu Val Leu Thr Gly Asn Thr Val Ile Phe Ala Thr
               100                 105                 110
   ata cta ggc ttt ttc ttg gtc ttt gga agc aat gac gac ttc agc tgg      384
   Ile Leu Gly Phe Phe Leu Val Phe Gly Ser Asn Asp Asp Phe Ser Trp
           115                 120                 125
   cag cag tgg tga                                                      396
   Gln Gln Trp
       130
<210> 16
   <211> 131
   <212> PRT
   <213> Homo sapiens
<400> 16
   Met Ala Gly Ile Lys Ala Leu Ile Ser Leu Ser Phe Gly Gly Ala Ile
   1               5                   10                  15
   Gly Leu Met Phe Leu Met Leu Gly Cys Ala Leu Pro Ile Tyr Asn Lys
               20                  25                  30
   Tyr Trp Pro Leu Phe Val Leu Phe Phe Tyr Ile Leu Ser Pro Ile Pro
           35                  40                  45
   Tyr Cys Ile Ala Arg Arg Leu Val Asp Asp Thr Asp Ala Met Ser Asn
       50                  55                  60
   Ala Cys Lys Glu Leu Ala Ile Phe Leu Thr Thr Gly Ile Val Val Ser
   65                  70                  75                  80
   Ala Phe Gly Leu Pro Ile Val Phe Ala Arg Ala His Leu Ile Glu Trp
                   85                  90                  95
   Gly Ala Cys Ala Leu Val Leu Thr Gly Asn Thr Val Ile Phe Ala Thr
               100                 105                 110
   Ile Leu Gly Phe Phe Leu Val Phe Gly Ser Asn Asp Asp Phe Ser Trp
           115                 120                 125
   Gln Gln Trp
   130
<210> 17
   <211> 1359
   <212> DNA
   <213> Homo sapiens
<220>
   <221> misc_feature
   <223> MY47 LUC
<220>
   <221> CDS
   <222> (1)..(1359)
   <223>
<400> 17
   atg gca ggc atc aaa gct ttg att agt ttg tcc ttt gga gga gca atc      48
   Met Ala Gly Ile Lys Ala Leu Ile Ser Leu Ser Phe Gly Gly Ala Ile
   1               5                   10                  15
   gga ctg atg ttt ttg atg ctt gga tgt gcc ctt cca ata tac aac aaa      96
   Gly Leu Met Phe Leu Met Leu Gly Cys Ala Leu Pro Ile Tyr Asn Lys
               20                  25                  30
   tac tgg ccc ctc ttt gtt cta ttt ttt tac atc ctt tca cct att cca      144
   Tyr Trp Pro Leu Phe Val Leu Phe Phe Tyr Ile Leu Ser Pro Ile Pro
           35                  40                  45
   tac tgc ata gca aga aga tta gtg gat gat aca gat gct atg agt aac      192
   Tyr Cys Ile Ala Arg Arg Leu Val Asp Asp Thr Asp Ala Met Ser Asn
       50                  55                  60
   gct tgt aag gaa ctt gcc atc ttt ctt aca acg ggc att gtc gtg tca      240
   Ala Cys Lys Glu Leu Ala Ile Phe Leu Thr Thr Gly Ile Val Val Ser
   65                  70                  75                  80
   gct ttt gga ctc cct att gta ttt gcc aga gca cat ctg att gag tgg      288
   Ala Phe Gly Leu Pro Ile Val Phe Ala Arg Ala His Leu Ile Glu Trp
                   85                  90                  95
   gga gct tgt gca ctt gtt ctc aca gga aac aca gtc atc ttt gca act      336
   Gly Ala Cys Ala Leu Val Leu Thr Gly Asn Thr Val Ile Phe Ala Thr
               100                 105                 110
   ata cta ggc ttt ttc ttg gtc ttt gga agc aat gac gac ttc agc tgg      384
   Ile Leu Gly Phe Phe Leu Val Phe Gly Ser Asn Asp Asp Phe Ser Trp
           115                 120                 125
   cag cag tgg cga ccg gtg gat cca ccg gct aga gcc acc atg acc agc      432
   Gln Gln Trp Arg Pro Val Asp Pro Pro Ala Arg Ala Thr Met Thr Ser
       130                 135                 140
   aag gtg tac gac ccc gag cag agg aag agg atg atc acc ggc ccc cag       480
   Lys Val Tyr Asp Pro Glu Gln Arg Lys Arg Met Ile Thr Gly Pro Gln
   145                 150                 155                 160
   tgg tgg gcc agg tgc aag cag atg aac gtg ctg gac agc ttc atc aac       528
   Trp Trp Ala Arg Cys Lys Gln Met Asn Val Leu Asp Ser Phe Ile Asn
                   165                 170                 175
   tac tac gac agc gag aag cac gcc gag aac gcc gtg atc ttc ctg cac       576
   Tyr Tyr Asp Ser Glu Lys His Ala Glu Asn Ala Val Ile Phe Leu His
               180                 185                 190
   ggc aac gcc gct agc agc tac ctg tgg agg cac gtg gtg ccc cac atc       624
   Gly Asn Ala Ala Ser Ser Tyr Leu Trp Arg His Val Val Pro His Ile
           195                 200                 205
   gag ccc gtg gcc agg tgc atc atc ccc gat ctg atc ggc atg ggc aag       672
   Glu Pro Val Ala Arg Cys Ile Ile Pro Asp Leu Ile Gly Met Gly Lys
       210                 215                 220
   agc ggc aag agc ggc aac ggc agc tac agg ctg ctg gac cac tac aag       720
   Ser Gly Lys Ser Gly Asn Gly Ser Tyr Arg Leu Leu Asp His Tyr Lys
   225                 230                 235                 240
   tac ctg acc gcc tgg ttc gag ctc ctg aac ctg ccc aag aag atc atc       768
   Tyr Leu Thr Ala Trp Phe Glu Leu Leu Asn Leu Pro Lys Lys Ile Ile
                   245                 250                 255
   ttc gtg ggc cac gac tgg ggc gcc tgc ctg gcc ttc cac tac agc tac       816
   Phe Val Gly His Asp Trp Gly Ala Cys Leu Ala Phe His Tyr Ser Tyr
               260                 265                 270
   gag cac cag gac aag atc aag gcc atc gtg cac gcc gag agc gtg gtg       864
   Glu His Gln Asp Lys Ile Lys Ala Ile Val His Ala Glu Ser Val Val
           275                 280                 285
   gac gtg atc gag agc tgg gac gag tgg cca gac atc gag gag gac atc       912
   Asp Val Ile Glu Ser Trp Asp Glu Trp Pro Asp Ile Glu Glu Asp Ile
       290                 295                 300
   gcc ctg atc aag agc gag gag ggc gag aag atg gtg ctg gag aac aac       960
   Ala Leu Ile Lys Ser Glu Glu Gly Glu Lys Met Val Leu Glu Asn Asn
   305                 310                 315                 320
   ttc ttc gtg gag acc atg ctg ccc agc aag atc atg aga aag ctg gag      1008
   Phe Phe Val Glu Thr Met Leu Pro Ser Lys Ile Met Arg Lys Leu Glu
                   325                 330                 335
   ccc gag gag ttc gcc gcc tac ctg gag ccc ttc aag gag aag ggc gag      1056
   Pro Glu Glu Phe Ala Ala Tyr Leu Glu Pro Phe Lys Glu Lys Gly Glu
               340                 345                 350
   gtg aga aga ccc acc ctg agc tgg ccc aga gag atc ccc ctg gtg aag      1104
   Val Arg Arg Pro Thr Leu Ser Trp Pro Arg Glu Ile Pro Leu Val Lys
           355                 360                 365
   ggc ggc aag ccc gac gtg gtg cag atc gtg aga aac tac aac gcc tac      1152
   Gly Gly Lys Pro Asp Val Val Gln Ile Val Arg Asn Tyr Asn Ala Tyr
       370                 375                 380
   ctg aga gcc agc gac gac ctg ccc aag atg ttc atc gag agc gac ccc      1200
   Leu Arg Ala Ser Asp Asp Leu Pro Lys Met Phe Ile Glu Ser Asp Pro
   385                 390                 395                 400
   ggc ttc ttc agc aac gcc atc gtg gag ggc gcc aag aag ttc ccc aac      1248
   Gly Phe Phe Ser Asn Ala Ile Val Glu Gly Ala Lys Lys Phe Pro Asn
                   405                 410                 415
   acc gag ttc gtg aag gtg aag ggc ctg cac ttc agc cag gag gac gcc      1296
   Thr Glu Phe Val Lys Val Lys Gly Leu His Phe Ser Gln Glu Asp Ala
               420                 425                     430
   ccc gac gag atg ggc aag tac atc aag agc ttc gtg gag aga gtg ctg      1344
   Pro Asp Glu Met Gly Lys Tyr Ile Lys Ser Phe Val Glu Arg Val Leu
           435                     440                 445
   aag aac gag cag taa                                                  1359
   Lys Asn Glu Gln
       450
<210> 18
   <211> 452
   <212> PRT
   <213> Homo sapiens
<220>
   <221> misc_feature
   <223> MY47 LUC
<400> 18
   Met Ala Gly Ile Lys Ala Leu Ile Ser Leu Ser Phe Gly Gly Ala Ile
   1               5                   10                  15
   Gly Leu Met Phe Leu Met Leu Gly Cys Ala Leu Pro Ile Tyr Asn Lys
               20                  25                  30
   Tyr Trp Pro Leu Phe Val Leu Phe Phe Tyr Ile Leu Ser Pro Ile Pro
           35                  40                  45
   Tyr Cys Ile Ala Arg Arg Leu Val Asp Asp Thr Asp Ala Met Ser Asn
       50                  55                  60
   Ala Cys Lys Glu Leu Ala Ile Phe Leu Thr Thr Gly Ile Val Val Ser
   65                  70                  75                  80
   Ala Phe Gly Leu Pro Ile Val Phe Ala Arg Ala His Leu Ile Glu Trp
                   85                  90                  95
   Gly Ala Cys Ala Leu Val Leu Thr Gly Asn Thr Val Ile Phe Ala Thr
               100                 105                 110
   Ile Leu Gly Phe Phe Leu Val Phe Gly Ser Asn Asp Asp Phe Ser Trp
           115                 120                 125
   Gln Gln Trp Arg Pro Val Asp Pro Pro Ala Arg Ala Thr Met Thr Ser
       130                 135                 140
   Lys Val Tyr Asp Pro Glu Gln Arg Lys Arg Met Ile Thr Gly Pro Gln
   145 150 155 160
   Trp Trp Ala Arg Cys Lys Gln Met Asn Val Leu Asp Ser Phe Ile Asn
   165 170 175
   Tyr Tyr Asp Ser Glu Lys His Ala Glu Asn Ala Val Ile Phe Leu His
   180 185 190
   Gly Asn Ala Ala Ser Ser Tyr Leu Trp Arg His Val Val Pro His Ile
   195 200 205
   Glu Pro Val Ala Arg Cys Ile Ile Pro Asp Leu Ile Gly Met Gly Lys
   210 215 220
   Ser Gly Lys Ser Gly Asn Gly Ser Tyr Arg Leu Leu Asp His Tyr Lys
   225 230 235 240
   Tyr Leu Thr Ala Trp Phe Glu Leu Leu Asn Leu Pro Lys Lys Ile Ile
   245 250 255
   Phe Val Gly His Asp Trp Gly Ala Cys Leu Ala Phe His Tyr Ser Tyr
   260 265 270
   Glu His Gln Asp Lys Ile Lys Ala Ile Val His Ala Glu Ser Val Val
   275 280 285
   Asp Val Ile Glu Ser Trp Asp Glu Trp Pro Asp Ile Glu Glu Asp Ile
   290 295 300
   Ala Leu Ile Lys Ser Glu Glu Gly Glu Lys Met Val Leu Glu Asn Asn
   305 310 315 320
   Phe Phe Val Glu Thr Met Leu Pro Ser Lys Ile Met Arg Lys Leu Glu
   325 330 335
   Pro Glu Glu Phe Ala Ala Tyr Leu Glu Pro Phe Lys Glu Lys Gly Glu
   340 345 350
   Val Arg Arg Pro Thr Leu Ser Trp Pro Arg Glu Ile Pro Leu Val Lys
   355 360 365
   Gly Gly Lys Pro Asp Val Val Gln Ile Val Arg Asn Tyr Asn Ala Tyr
   370 375 380
   Leu Arg Ala Ser Asp Asp Leu Pro Lys Met Phe Ile Glu Ser Asp Pro
   385 390 395 400
   Gly Phe Phe Ser Asn Ala Ile Val Glu Gly Ala Lys Lys Phe Pro Asn
   405 410 415
   Thr Glu Phe Val Lys Val Lys Gly Leu His Phe Ser Gln Glu Asp Ala
               420                 425                 430
   Pro Asp Glu Met Gly Lys Tyr Ile Lys Ser Phe Val Glu Arg Val Leu
           435                 440                 445
   Lys Asn Glu Gln
       450
<210> 19
   <211> 1140
   <212> DNA
   <213> Artificial Sequence
<220>
   <223> MYO47 YFP
<220>
   <221> misc_feature
   <223> MY47 YFP
<220>
   <221> CDS
   <222> (1)..(1140)
   <223>
<400> 19
   atg gca ggc atc aaa gct ttg att agt ttg tcc ttt gga gga gca atc      48
   Met Ala Gly Ile Lys Ala Leu Ile Ser Leu Ser Phe Gly Gly Ala Ile
   1               5                   10                  15
   gga ctg atg ttt ttg atg ctt gga tgt gcc ctt cca ata tac aac aaa      96
   Gly Leu Met Phe Leu Met Leu Gly Cys Ala Leu Pro Ile Tyr Asn Lys
               20                  25                  30
   tac tgg ccc ctc ttt gtt cta ttt ttt tac atc ctt tca cct att cca     144
   Tyr Trp Pro Leu Phe Val Leu Phe Phe Tyr Ile Leu Ser Pro Ile Pro
           35                  40                  45
   tac tgc ata gca aga aga tta gtg gat gat aca gat gct atg agt aac     192
   Tyr Cys Ile Ala Arg Arg Leu Val Asp Asp Thr Asp Ala Met Ser Asn
       50                  55                  60
   gct tgt aag gaa ctt gcc atc ttt ctt aca acg ggc att gtc gtg tca     240
   Ala Cys Lys Glu Leu Ala Ile Phe Leu Thr Thr Gly Ile Val Val Ser
   65                  70                  75                  80
   gct ttt gga ctc cct att gta ttt gcc aga gca cat ctg att gag tgg     288
   Ala Phe Gly Leu Pro Ile Val Phe Ala Arg Ala His Leu Ile Glu Trp
                   85                  90                  95
   gga gct tgt gca ctt gtt ctc aca gga aac aca gtc atc ttt gca act      336
   Gly Ala Cys Ala Leu Val Leu Thr Gly Asn Thr Val Ile Phe Ala Thr
               100                 105                 110
   ata cta ggc ttt ttc ttg gtc ttt gga agc aat gac gac ttc agc tgg      384
   Ile Leu Gly Phe Phe Leu Val Phe Gly Ser Asn Asp Asp Phe Ser Trp
           115                 120                 125
   cag cag tgg cga ccg gtg gat cca ccg gtc gcc acc atg gtg agc aag      432
   Gln Gln Trp Arg Pro Val Asp Pro Pro Val Ala Thr Met Val Ser Lys
       130                 135                 140
   ggc gag gag ctg ttc acc ggg gtg gtg ccc atc ctg gtc gag ctg gac      480
   Gly Glu Glu Leu Phe Thr Gly Val Val Pro Ile Leu Val Glu Leu Asp
   145                 150                 155                 160
   ggc gac gta aac ggc cac aag ttc agc gtg tcc ggc gag ggc gag ggc      528
   Gly Asp Val Asn Gly His Lys Phe Ser Val Ser Gly Glu Gly Glu Gly
                   165                 170                 175
   gat gcc acc tac ggc aag ctg acc ctg aag ttc atc tgc acc acc ggc      576
   Asp Ala Thr Tyr Gly Lys Leu Thr Leu Lys Phe Ile Cys Thr Thr Gly
               180                 185                 190
   aag ctg ccc gtg ccc tgg ccc acc ctc gtg acc acc ttc ggc tac ggc      624
   Lys Leu Pro Val Pro Trp Pro Thr Leu Val Thr Thr Phe Gly Tyr Gly
           195                 200                 205
   gtg cag tgc ttc gcc cgc tac ccc gac cac atg cgc cag cac gac ttc      672
   Val Gln Cys Phe Ala Arg Tyr Pro Asp His Met Arg Gln His Asp Phe
       210                 215                 220
   ttc aag tcc gcc atg ccc gaa ggc tac gtc cag gag cgc acc atc ttc      720
   Phe Lys Ser Ala Met Pro Glu Gly Tyr Val Gln Glu Arg Thr Ile Phe
   225                 230                 235                 240
   ttc aag gac gac ggc aac tac aag acc cgc gcc gag gtg aag ttc gag      768
   Phe Lys Asp Asp Gly Asn Tyr Lys Thr Arg Ala Glu Val Lys Phe Glu
                   245                 250                 255
   ggc gac acc ctg gtg aac cgc atc gag ctg aag ggc atc gac ttc aag      816
   Gly Asp Thr Leu Val Asn Arg Ile Glu Leu Lys Gly Ile Asp Phe Lys
               260                 265                 270
   gag gac ggc aac atc ctg ggg cac aag ctg gag tac aac tac aac agc      864
   Glu Asp Gly Asn Ile Leu Gly His Lys Leu Glu Tyr Asn Tyr Asn Ser
           275                 280                 285
   cac aac gtc tat atc atg gcc gac aag cag aag aac ggc atc aag gtg      912
   His Asn Val Tyr Ile Met Ala Asp Lys Gln Lys Asn Gly Ile Lys Val
       290                 295                 300
   aac ttc aag atc cgc cac aac atc gag gac ggc agc gtg cag ctc gcc      960
   Asn Phe Lys Ile Arg His Asn Ile Glu Asp Gly Ser Val Gln Leu Ala
   305                 310                 315                 320
   gac cac tac cag cag aac acc ccc atc ggc gac ggc ccc gtg ctg ctg     1008
   Asp His Tyr Gln Gln Asn Thr Pro Ile Gly Asp Gly Pro Val Leu Leu
                   325                 330                 335
   ccc gac aac cac tac ctg agc tac cag tcc gcc ctg agc aaa gac ccc     1056
   Pro Asp Asn His Tyr Leu Ser Tyr Gln Ser Ala Leu Ser Lys Asp Pro
               340                 345                 350
   aac gag aag cgc gat cac atg gtc ctg ctg gag ttc gtg acc gcc gcc     1104
   Asn Glu Lys Arg Asp His Met Val Leu Leu Glu Phe Val Thr Ala Ala
           355                 360                 365
   ggg atc act ctc ggc atg gac gag ctg tac aag taa                    1140
   Gly Ile Thr Leu Gly Met Asp Glu Leu Tyr Lys
       370                 375
<210> 20
   <211> 379
   <212> PRT
   <213> Artificial Sequence
<220>
   <223> MYO47 YFP
<220>
   <221> misc_feature
   <223> MY47 YFP
<400> 20
   Met Ala Gly Ile Lys Ala Leu Ile Ser Leu Ser Phe Gly Gly Ala Ile
   1               5                   10                  15
   Gly Leu Met Phe Leu Met Leu Gly Cys Ala Leu Pro Ile Tyr Asn Lys
               20                  25                  30
   Tyr Trp Pro Leu Phe Val Leu Phe Phe Tyr Ile Leu Ser Pro Ile Pro
           35                  40                  45
   Tyr Cys Ile Ala Arg Arg Leu Val Asp Asp Thr Asp Ala Met Ser Asn
       50                  55                  60
   Ala Cys Lys Glu Leu Ala Ile Phe Leu Thr Thr Gly Ile Val Val Ser
   65                  70                  75                  80
   Ala Phe Gly Leu Pro Ile Val Phe Ala Arg Ala His Leu Ile Glu Trp
                   85                  90                  95
   Gly Ala Cys Ala Leu Val Leu Thr Gly Asn Thr Val Ile Phe Ala Thr
               100                 105                 110
   Ile Leu Gly Phe Phe Leu Val Phe Gly Ser Asn Asp Asp Phe Ser Trp
           115                 120                 125
   Gln Gln Trp Arg Pro Val Asp Pro Pro Val Ala Thr Met Val Ser Lys
       130                 135                 140
   Gly Glu Glu Leu Phe Thr Gly Val Val Pro Ile Leu Val Glu Leu Asp
   145                 150                 155                 160
   Gly Asp Val Asn Gly His Lys Phe Ser Val Ser Gly Glu Gly Glu Gly
                   165                 170                 175
   Asp Ala Thr Tyr Gly Lys Leu Thr Leu Lys Phe Ile Cys Thr Thr Gly
               180                 185                 190
   Lys Leu Pro Val Pro Trp Pro Thr Leu Val Thr Thr Phe Gly Tyr Gly
           195                 200                 205
   Val Gln Cys Phe Ala Arg Tyr Pro Asp His Met Arg Gln His Asp Phe
       210                 215                 220
   Phe Lys Ser Ala Met Pro Glu Gly Tyr Val Gln Glu Arg Thr Ile Phe
   225                 230                 235                 240
   Phe Lys Asp Asp Gly Asn Tyr Lys Thr Arg Ala Glu Val Lys Phe Glu
                   245                 250                 255
   Gly Asp Thr Leu Val Asn Arg Ile Glu Leu Lys Gly Ile Asp Phe Lys
               260                 265                 270
   Glu Asp Gly Asn Ile Leu Gly His Lys Leu Glu Tyr Asn Tyr Asn Ser
           275                 280                 285
   His Asn Val Tyr Ile Met Ala Asp Lys Gln Lys Asn Gly Ile Lys Val
       290                 295                 300
   Asn Phe Lys Ile Arg His Asn Ile Glu Asp Gly Ser Val Gln Leu Ala
   305                 310                 315                 320
   Asp His Tyr Gln Gln Asn Thr Pro Ile Gly Asp Gly Pro Val Leu Leu
                   325                 330                 335
   Pro Asp Asn His Tyr Leu Ser Tyr Gln Ser Ala Leu Ser Lys Asp Pro
               340                 345                 350
   Asn Glu Lys Arg Asp His Met Val Leu Leu Glu Phe Val Thr Ala Ala
           355                 360                 365
   Gly Ile Thr Leu Gly Met Asp Glu Leu Tyr Lys
       370                 375
<210> 21
   <211> 1114
   <212> DNA
   <213> Homo sapiens
<400> 21
   gtctggcttg ggcaggctgc ccgggccgtg gcaggaagcc ggaagcagcc gcggccccag      60
   ttcgggagac atggcgggcg ttaaagctct cgtggcatta tccttcagtg gggctattgg 120
   actgactttt cttatgctgg gatgtgcctt agaggattat ggcgtttact ggcccttatt 180
   cgtcctgatt ttccacgcca tctcccccat cccccatttc attgccaaaa gagtcaccta 240
   tgactcagat gcaaccagta gtgcctgtcg ggaactggca tatttcttca ctactggaat 300
   tgttgtttct gcctttggat ttcctgttat tcttgctcgt gtggctgtga tcaaatgggg 360
   agcctgcggc cttgtgttgg caggcaatgc agtcattttc cttacaattc aagggttttt 420
   ccttatattt ggaagaggag atgattttag ctgggagcag tggtagcact ttattctgat 480
   tacagtgcat tgaatttctt agaactcata ctatctgtat acatgtgcac atgcggcatt     540
   ttactatgaa atttaatatg ctgggttttt taataccttt atatatcatg ttcactttaa     600
   gaaagacttc ataagtagga gatgagtttt attctcagca aatagacctg tcaaatttag 660
   attatgttac tcaaattatg ttacttgttt ggctgttcat gtagtcacgg tgctctcaga 720
   aaatatatta acgcagtctt gtaggcagct gccaccttat gcagtgcatc gaaacctttt 780
   gcttggggat gtgcttggag aggcagataa cgctgaagca ggcctctcat gacccaggaa 840
   ggccggggtg gatccctctt tgtgttgtag tccatgctat taaaagtgtg gcccacagac 900
   caagagcctc aacatttcct agagccttat tagaaatgca gaatctgaag ccccactctg 960
   gacccaggac attttgatga gatccaaagg agttgtatgc acatgaaagt ttgagaagca 1020
   tcatcataga gaagtaaaca tcacacccaa cttccttatc tttccagtgg ctaaaccact 1080
   taacctctct gggtgttacc tgctcatttg ttta 1114
<210> 22
   <211> 20
   <212> DNA
   <213> Artificial Sequence
<220>
   <223> AS01
<400> 22
   gcttcctgcc acggcccggg 20
<210> 23
   <211> 20
   <212> DNA
   <213> Artificial sequence
<220>
   <223> AS02
<400> 23
   gccatgtctc ccgaactggg 20
<210> 24
   <211> 20
   <212> DNA
   <213> Artificial Sequence
<220>
   <223> AS03
<400> 24
   aacgcccgcc atgtctcccg 20
<210> 25
   <211> 20
   <212> DNA
   <213> Artificial Sequence
<220>
   <223> AS04
<400> 25
   agagctttaa cgcccgccat 20
<210> 26
   <211> 20
   <212> DNA
   <213> Artificial Sequence
<220>
   <223> AS05
<400> 26
   ggataatgcc acgagagctt 20
<210> 27
   <211> 20
   <212> DNA
   <213> Artificial Sequence
<220>
   <223> AS06
<400> 27
   aaggcacatc ccagcataag 20
<210> 28
   <211> 20
   <212> DNA
   <213> Artificial Sequence
<220>
   <223> AS07
<400> 28
   ccataatcct ctaaggcaca 20
<210> 29
   <211> 20
   <212> DNA
   <213> Artificial Sequence
<220>
   <223> AS08
<400> 29
   cagtaaacgc cataatcctc 20
<210> 30
   <211> 20
   <212> DNA
   <213> Artificial Sequence
<220>
   <223> AS09
<400> 30
   aataagggcc agtaaacgcc 20
<210> 31
   <211> 20
   <212> DNA
   <213> Artificial Sequence
<220>
   <223> AS10
<400> 31
   atgccagttc ccgacaggca 20
<210> 32
   <211> 22
   <212> DNA
   <213> Artificial Sequence
<220>
   <223> AS11
<400> 32
   aaatatgcca gttcccgaca gg 22
<210> 33
   <211> 20
   <212> DNA
   <213> Artificial Sequence
<220>
   <223> AS12
<400> 33
   aagaaatatg ccagttcccg 20
<210> 34
   <211> 20
   <212> DNA
   <213> Artificial Sequence
<220>
   <223> AS13
<400> 34
   cattgcctgc caacacaagg 20
<210> 35
   <211> 20
   <212> DNA
   <213> Artificial Sequence
<220>
   <223> AS15
<400> 35
   aaccgccgac ctgtcctccg 20
<210> 36
   <211> 22
   <212> DNA
   <213> Artificial Sequence
<220>
   <223> AS16
<400> 36
   aaatctgcac gttacagcca gg 22
<210> 37
   <211> 21
   <212> DNA
   <213> Artificial Sequence
<220>
   <223> Artificial
<400> 37
   gugccugucg ggaacuggct t 21
<210> 38
   <211> 21
   <212> DNA
   <213> Artificial Sequence
<220>
   <223> Artificial
<400> 38
   gccaguuccc gacaggcact t 21
<210> 39
   <211> 38
   <212> DNA
   <213> Artificial Sequence
<220>
   <223> Artificial
<400> 39
   tctcttgaat gaaacacatg tgcacacaca aacaaggc 38
<210> 40
   <211> 40 <212> DNA
   <213> Artificial Sequence
<220>
   <223> Artificial
<400> 40
   gctctagaaa aatgtgtgca catgtgtttc atctcttgaa 40
<210> 41
   <211> 35
   <212> DNA
   <213> Artificial Sequence
<220>
   <223> Artificial
<400> 41
   ccatctaggc caagcttatc cgacgccgcc atctc 35
<210> 42
   <211> 47
   <212> DNA
   <213> Artificial Sequence
<220>
   <223> Artificial
<400> 42
   tgtgtgcaca tgtgtttcat tcaagagatg aaacacatgt gcacaca 47
<210> 43
   <211> 14
   <212> DNA
   <213> Artificial Sequence
<220>
   <223> Artificial
<400> 43
   ccgtggcagg aagc 14
<210> 44
   <211> 20
   <212> DNA
   <213> Artificial Sequence
<220>
   <223> Artificial
<400> 44
   gcagcgacag ccccagctcc 20
<210> 45
   <211> 16
   <212> DNA
   <213> Artificial Sequence
<220>
   <223> Artificial
<400> 45
   cagccacacg agcaag 16
<210> 46
   <211> 15
   <212> DNA
   <213> Artificial Sequence
<220>
   <223> Artificial
<400> 46
   ggagaaggct ggggc 15
<210> 47
   <211> 17
   <212> DNA
   <213> Artificial Sequence
<220>
   <223> Artificial
<400> 47
   gatggcatgg actgtgg 17

## Description

La présente demande a pour objet des oligonucléotides inhibant l'expression de la protéine OB-RGRP

L'invention est définie par les revendications.

La leptine est une protéine de 16 kDa secrétée principalement par le tissu adipeux, et se liant à un récepteur (OB-R) appartenant à la famille des récepteurs aux cytokines. Cinq isoformes membranaires de ce récepteur ont été identifiéès, et dérivent de l'épissage alternatif d'un même gène. Ces isoformes qui possèdent le même domaine extracellulaire et transmembranaire, sont caractérisés par des domaines intracellulaires de tailles variables(Tartaglia et al. (1995) Cell 83, 1263-1271). Une forme soluble du récepteur à aussi été identifiée et provient d'un épissage alternatif au d'un clivage protéolytique du domaine extracellulaire des formes membranaires. La forme courte du récepteur (OB-Rs) qui semble impliquée dans le transport de la leptine au travers de la barrière hémato-encéphalique est l'isoforme la plus exprimée. La forme longue (OB-RI) est seulement exprimée dans quelques tissus comme l'hypothalamus et semble responsable de la plupart des effets biologiques de la leptine (Sweeney, G. (2002) Cell Signal 14, 655-663). La leptine et son récepteur ont été l'objet d'une attention particulière à cause de leur implication dans la régulation de la balance énergétique, du métabolisme, et dans la réponse neuroendocrine à la prise alimentaire. Récemment, il a été montré que la leptine est aussi impliquée dans des fonctions additionnelles importantes comme la régulation de la masse osseuse, l'angiogénèse, la cicatrisation, la formation de thrombus, la maturation sexuelle, l'hématopoïèse, la régulation de l'immunité et de l'inflammation, le développement foetal et le cancer. L'administration de leptine chez des organismes déficients en leptine comme les souris (ob/ob) et certains individus humains provoque une diminution de la masse de lipide dans divers tissus comme le foie et le tissu adipeux (Halaas et al. (1995) Science 269, 543-546, Pelleymounter et al. (1995) Science 269, 540-543, Campfield et al. (1995) Science 269, 546-549, Farooqi et al. (1999) N Engl J Med 341, 879-884). Ce traitement par la leptine améliore aussi la sensibilité à l'insuline et diminue la masse grasse chez la souris et l'homme présentant une lipodistrophie(Shimomura et al. (1999) Nature 401, 73-76, Oral et al. (2002) New England Journal of Medicine 346, 570-578, Petersen et al. (2002) J Clin Invest 109, 1345-1350 . Les personnes obèses sont généralement résistantes à la leptine. Les raisons de cette résistance sont encore mal comprises mais plusieurs mécanismes ont été suggérés : un défaut de transport de la leptine au travers de la barrière hémato-encéphalique, un défaut d'activation des OB-R ou de la signalisation de ces récepteurs, et la surexpression de régulateurs négatifs comme SOCS3 et PTP-1B Bjorbaek et al. (2000) J Biol Chem 275, 40649-40657, Cheng et al. (2002) Developmental Cell 2, 497-503, Cook et Unger (2002) Developmental Cell 2, 385-387. La compréhension des mécanismes de la résistance à la leptine requiert une caractérisation plus détaillée des mécanismes impliqués dans l'activation des OB-R.

OB-R est constitutivement associé à la janus kinase 2 (JAK2). La liaison de JAK2 au récepteur est critique pour la signalisation par les OB-R et a été proposée comme étant impliquée dans la stabilisation des dimères de récepteurs OB-R. L'activation par des agonistes provoquerait un changement de conformation dans la région juxta membranaire de la queue cytoplasmique des OB-R. JAK2, qui est constitutivement reliée au motif box1 dans cette région est activée par autophosphorylation puis phosphoryle le récepteur OB-RI mais pas OB-Rs. La phosphorylation d'OB-RI permet l'ancrage de protéines STAT qui se lient au récepteur et son activées par phosphorylation sur tyrosine. Les protéines STAT activées dimérisent et transloquent dans le noyau pour stimuler la transcription de gènes via des élément de réponse aux STAT (Tartaglia (1997) J Biol Chem 272, 6093-6096).

Récemment, un second promoteur pour le récepteur de la leptine a été découvert. De façon intéressante, un second transcrit est co-exprimé avec les messagers des OB-R depuis ce promoteur. Ce transcrit a été observé dans plusieurs espèces comme la souris, le rat, l'homme, la levure et C. *elegans* (Bailleul et al. (1997) Nucleic Acids Res 25, 2752-2758). Des expériences d'hybridation *in situ* confirment la co-expression des OB-R et du gène associé dans le cerveau de la souris y compris les régions hypothalamiques impliquées dans la régulation du poids corporel (Mercer et al., J Neuroendocrinol 2000 Jul;12(7):649-55). La protéine correspondante est composée de 131 acides aminés et est appelée OB-R-gene related protein (OB-RGRP). Cette protéine a fait l'objet de la demande de brevet WO98/05792.

Le fait qu'OB-RGRP soit exprimée chez la levure et le nématode, organismes dépourvus de récepteurs de la leptine, Indique un rôle plus général pour OB-RGRP, supporté par la délétion de cette protéine chez la levure qui provoque un défaut de transport des protéines du golgi vers les vacuoles (Belgareh-Touze et al. (2002) Molecular Biology Of The Cell 13, 1694-1708).

En 2001, un ADNc appelé MY047, a été cloné à partir d'une banque d'ADNc de cerveau humain (Huang et al. (2001) Blochimica et Biophysica acta. Gene structure and expression 327-331). Cette protéine a en outre fait l'objet de la demande EP 0 969 091. La fonction de la protéine correspondante est encore inconnue. MY047 a 68 % d'homologie avec OB-RGRP suggérant que ces deux protéines appartiennent à la même famille. L'analyse des séquences
Par ailleurs, la demande de brevet EP0969091A2 enseigne un polypeptide dénommé LEPRGRP. Cette demande suggère que l'expression du gène codant pour le polypeptide LEPRGRP endogène pourrait être inhibée par le biais de techniques bloquant l'expression. disponibles du projet de séquençage du génome humain montre qu'il n'existe aucun autre homologue.

Les demandeurs se sont attachés à déterminer le rôle de la OB-RGRP et ses relations avec les récepteurs de la leptine.

Ils ont ainsi montré la spécificité des interactions entre la OB-RGRP et le récepteur OBRs.

Ils ont en outre montré qu'il était possible de modifier spécifiquement l'expression des récepteurs de la leptine à la surface cellulaire à l'aide d'oligonucléotides antisens dirigés contre la protéine associée au gène des récepteurs de la leptine (OB-RGRP).

La présente a donc pour objet des oligonucléotides éventuellement modifiés comprenant de 8 à 50 nucléotides qui hybrident de manière spécifique à la séquence SEQ ID N° 1 et inhibent l'expression de OB-RGRP, caractérisés en ce qu'ils comprennent une séquence présentant une identité d'au moins 90% avec la séquence SEQ ID NO:2. Avantageusement, ces oligonucléotides favorisent l'expression des récepteurs de la leptine à la surface cellulaire.

Préférentiellement ces oilgonucléotides sont des antisens.

Selon un mode de mise en oeuvre avantageux ces oligonucléotides sont caractérisés en ce que des nucléotides sont thioestérifiés.

Selon un autre mode de mise en oeuvre avantageux ces oligonucléotides sont caractérisés en ce que des nucléotides sont 2' O-méthylés.

Selon un autre mode de mise en oeuvre avantageux ces oligonucléotides sont caractérisés en ce qu'il présentent un résidu triéthylèneglycol à leurs extrémités 3'.

Bien que la forme la plus usitée de composés antisens se présente sous la forme d'oligonucléotides antisens, la présente invention inclut les dérivés d'oligonucléotides et les composés mimant leur structure tels que ceux décrits
ci-après, sans que cette liste soit limitative. Les composés antisens en accord avec cette invention comprennent de préférence, de 8 à 50 nucléobases (c'est-à-dire sont des oligomères formés de 8 à 50 unités nucléotidiques). Les composés antisens particulièrement visés sont les oligonucléotides antisens, plus spécialement ceux qui sont formés d'environ 12 à 30 nucléobases. Les composés antisens comprennent les ribozymes, les oligozymes et autres ARN catalytiques courts ou oligonucléotides catalytiques qui s'hybrident avec l'acide nucléique cible et modulent son expression. Un nucléoside est une combinaison d'une base azotée et d'un sucre. La base d'un nucléoside est généralement une base azotée hétérocyclique. Les deux types de base hétérocycliques les plus communs sont les bases puriques et pyrimidiques. Les nucléotides sont des nucléosides qui portent un groupement phosphate lié de manière covalente au sucre du nucléoside. Pour les nucléosides comportant un pentanofurannose, le phosphate peut être lié à l'hydroxyle en position 2', 3' ou 5' du sucre. La formation de nucléotides provient de l'attachement covalent du groupement phosphate à deux nucléosides adjacents ce qui permet de proche en proche l'obtention d'un oligomère linéaire. Les deux extrémités d'un tel polymère linéaire peuvent à leur tour se joindre pour former une structure circulaire, mais la structure ouverte est généralement préférée. Dans la structure nucléotidique, les groupements phosphate sont considérés comme formant le squelette internucléosidique de l'oligonucléotide. La liaison normale dans le squelette d'ARN ou d'ADN est un lien phosphodiester 3'-5'. Des exemples spécifiques de composés antisens utilisables dans cette invention incluent des oligonucléotides contenant une ossature modifiée ou des liaisons internucléosidiques non-naturelles. Ainsi des oligonucléotides à ossature modifiée comprennent ceux qui conservent un atome de phosphate dans leur squelette et ceux qui en sont dépourvus. Pour les besoins de la présente invention, des oligonucléotides modifiés ne possédant pas d'atome de phosphore dans leur liaison internucléosidique peuvent malgré tout être considérés comme des oligonucléotides. L'ossature de ces oligonucléotides modifiés peut comprendre par exemple des groupements phosphorothioates, phosphorothioates chiraux, phosphorodithioates, phosphotriesters, aminoalkylphosphotriesters, méthyl et autres alkyl phosphonates y compris les 3'-alkylène phosphonates, 5'-alkylène phosphonates et phosphonates chiraux, des groupements phosphinates, phosphoramidates y compris 3'-amino phosphoramidate and aminoalkylphosphoramidates, thionophosphoramidates, thionoalkylphosphonates, thionoalkylphosphotriesters, sélénophosphates and borophosphates formant des liaisons normales, 3'-5', et leurs analogues formant des liasons 2'-5', ainsi que ceux qui présentent une polarité inversée, c'est-à-dire comprenant au moins une liaison internucléosidique de type 3'-3', 5'-5' ou 2'-2'. La forme d'oligonucléotides possédant une polarité inversée préférentiellement utilisée est celle possédant la première liaison internucléosidique en 3' est de type 3'-3'. Ceci correspond à un seul résidu nucléosidique inversé, qui peut par ailleurs être abasique, c'est-à-dire dans lequel la base azotée hétérocyclique est manquante ou remplacée par un groupement hydroxyle. Les diverses formes (salines ou acide libre) sont incluses dans le champ de cette invention.

L'ossature des oligonucléotides modifiés dépourvus d'atome de phosphore est préférentiellement formée de courtes chaines alkyles ou cycloalkyles, y compris leurs dérivés comportant un ou plusieurs hétéroatomes, servant de liaison internucléosidique. Ce type d'ossature peut être à base de liaison morpholino (constituée en partie par le sucre du nucléoside), de siloxane, de formacétyle et thioformacétyle, de méthylène formacétyle et méthylène thioformacétyle, de riboacétyle, d'alcènes, de sulfamates, de sulfonate et sulfonamide, de méthylène imine et méthylène hydrazine, d'amide, et de tout autre groupement comportant divers atomes d'azote, de soufre et d'oxygène ou des groupes méthyle.

Pour d'autres analogues d'oligonucléotides, le sucre et la liaison internucléosidique (c'est-à-dire l'ossature) sont à la fois remplacés dans la structure nucléotidique par de nouveaux groupes. La base azotée hétérocyclique est conservée pour assurer l'hybridation avec l'acide nucléique cible. De tels composés oligomères, les PNA (pour Peptide Nucleic Acid), ont montré une excellente capacité d'hybridation. Dans ces composés, le squelette de l'oligonucléotide est remplacé par une ossature à base d'amide, en particulier par l'aminoéthyl glycine, greffée directement ou indirectement sur les bases azotées. De plus amples renseignement sur ces PNA peuvent être trouvés dans Nielsen et al., Science, 1991, 254, 1497.

L'invention incorpore plus particulièrement des oligonucléotides à ossature phosphorothioate, amide et morpholine, et les oligonucléosides à squelette à hétéroatomes, plus précisément :

-CH2-NH-O-CH2-

-CH2-N(CH3)-O-CH2- (dénommé squelette méthylène-(méthylimino) ou MMI)

-CH2-O-N(CH3)-CH2-

-CH2-N(CH3)-N(CH3)-CH2-

-O-N(CH3)-CH2-CH2- (dans lequel le pont phosphodiester est : O-P-O-CH2).

La modification des oligonucléotides peut également porter sur les sucres : les substitutions préférées sont en position 2' (F; dérivés O-, N- ou S-alcanes, O-, N- ou S-alcènes ou O-, N- ou S-alcynes de longueur C1 à C11, substitués ou non) en particulier, les dérivés préférés sont :

O-[(CH2)nO]m-CH3

O-(CH2)n-O-CH3

O-(CH2)n-NH2

O-(CH2)n-CH3

O-(CH2)n-O-NH2

O-(CH2)n-O-N[(CH2)n-CH3]2

où n et m varient de 1 à 10.

D'autres modifications de la position 2' incluent les groupes suivants : chaînes aliphatiques substituées ou non de longueur C1 à C10, aryles, aryles-alkyles et alkyles-aryles; -SH, -SCH3, -OCN, -Cl, -Br, -CN, -CF3, -OCF3, -SO2CH3, - ONO2, -NO2, -N3, -NH2; silyles substitués; groupement "rapporteur"; groupement intercaleur; groupement de clivage de l'ARN; groupement pour améliorer les capacités pharmacodynamiques d'un oligonucléotide. Les modifications préférées incluent les groupements :
2'-méthoxyéthoxy (2'-O-CH2CH2OCH3, également appelé 2'-O-(2-méthoxyéthyl) ou 2'-MOE) (Martin et al., Helv. Chim. Acta, 1995, 78f 486-504) 2'-diméthylaminooxyéthoxy (O(CH2)2ON(CH3)2, également appelé 2'-DMAOE
2'-diméthylaminoéthoxyéthoxy (2'-O-CH2OCH2-N(CH2)2, également appelé 2'-diméthylaminoéthoxyéthyl or 2'-DMAEOE).

Une autre modification intéressante conduit à la formation de LNA (Locked Nucleic Acids) dans lesquels l'hydroxyle en position 2' est lié au carbone en position 3' ou 4' du sucre, formant alors un sucre à structure bicyclique. Le pontage préféré se fait par un lien méthyle ou éthyle entre l'oxygène 2' et le carbone en 4'.

D autres substitutions préférées en position 2' incluent :

-O-CH3 (2'-méthoxy)

-O-(CH2)3-NH2 (2'-aminopropoxy)

-CH2-CH=CH2 (2'-allyle)

-O-CH2-CH=CH2 (2'-O-allyle)

-F (2' fluoro).

Ces modifications en 2' peuvent se présenter en position ribo (bas) ou arabino (haut). Le substituant 2' fluoro est le préféré en position arabino.

Des modifications similaires peuvent être faite sur d'autres positions, en particulier en position 3' du sucre du nucléotide en extrémité 3'-terminale ou dans les oligonucléotides à ossature 2'-5', et en position 5' du sucre en extrémité 5'-terminale. Les sucres des oligonucléotides peuvent également être remplacés par des analogues (par exemple un cyclobutyl peut être substitué à un pentofurranyl).

Les oligonucléotides peuvent aussi comporter des modifications ou substitutions au niveau des nucléobases (bases hétérocycliques azotées appelées "bases" par l'homme de l'art). Les bases naturelles (non modifiées) sont les purines (adénine A et guanine G) et les pyrimidines (cytosine C, thymine T et uracile U). Parmi les bases modifiées sont incluses des molécules naturelles ou synthétiques telles que 5-méthylcytosine, 5-hydroxyméthylcytosine, xanthine, hypoxanthine, 2-aminoadénine; 6-méthyl, 2-méthyl et autres dérivés alkylés des bases puriques (A et G); dérivé 2-thio (C, T et U); dérivé 5-halo (U, C); dérivé 5-propynyl (U et C) cytosine; dérivé 6-azo (U, Tet C); 5-uracile; 4-thiouracile; 8-halo, 8- amino, 8-thiol, 8-thioalkyl, 8-hydroxyl autres adénines et guanines substituées en position 8; 5-halo (en particulier 5-bromo), 5- trifluoromethyl et autres uraciles and cytosines substituées en position 5; 7-méthylguanine and 7-méthyladénine; 2-fluoroadénine; 2-amino-adénine; 8-azaguanine et 8-azaadénine; 7-déazaguanine et 7-déazaadénine; 3-déazaguanine et 3-déazaadénine. Dans les autres bases modifiées, on trouve les pyrimidines tricycliques telles que phénoxazine cytidine(IH-pyrimido[5,4-b][ 1,4]benzoxazin-2(3H)- one), phénothiazine cytidine (1H-pyrimido[5,4- b][1,4]benzothiazin-2(3H)-one), phénoxazine cytidine substituées (comme la 9-(2-aminoethoxy)-H-pyrimido[5,4-b][1,4]benzoxazin-2(3H)-one), carbazole cytidine (2H-pyrimido[4,5-b]-indol-'2-one).

Les bases modifiées comprennent les composés dont l'hétérocycle purique ou pyrimidique est remplacé par un autre hétérocycle par example 7-déazaadénine, 7-déazaguanosine, 2-aminopyridine ou 2-pyridone (The Concise Encyclopedia Of Polymer Science And Engineering, pages 858-859" Kroschwitz, J.I., ed. John Wiley & Sons, 1990; Englisch et al., Angewandte Chemie, International Edition, 1991, 30, 613; Sanghvi, Y.S., Chapter 15, Antisense Research and Applications, pages 289-302, Crooke, S.T. and Lebleu, B., ed., CRC Press, 1993). Certaines de ces bases modifiées peuvent être d'un grand intérêt pour augmenter l'affinité des composés oligomériques de l'invention, comme les pyrimidines substituées en position 5, les azapyrimidines, les purines N- et O- substituées (telles que la 2-aminopropyladénine, la 5-propynyl uracile, la 5-propynyl cytosine). Les 5-méthylcytosines substituées ont un effet positif sur la stabilité des duplex oligoméres-acides nucléiques (Sanghvif Y.S.f Crookef S.T, and Lebleu, B.,r eds.f Antisense Research and Applications, CRC Press, Boca Raton, 1993, pp. 276-278) et sont la substitution préférée, en particulier en combinaison avec les modifications 2'-méthoxyéthyl des sucres.

Préférentiellement ces oligonucléotides sont sous la forme simple brin.

Selon un mode de mise en oeuvre particulièrement avantageux ces oligonucléotides comprennent une séquence présentant une identité d'au moins 90 %, avec la séquence SEQ ID N°2, dans laquelle les nucléotides en positions 2, 4, 6, 7, 9, 11, 13, 15, 17, 19 et 20, dans le sens 5' vers 3', sont thioestérifiés.

Selon un mode de mise en oeuvre particulièrement avantageux ces oligonucléotides comprennent une séquence présentant une identité d'au moins 90% avec la séquence SEQ ID N°2, dans laquelle les nucléotides en positions 1, 2, 3, 4, 5, 16, 17, 18, 19 et/ou 20, dans le sens 5' vers 3', sont 2' O-méthylés.

Préférentiellement les oligonucléotides selon la présente invention sont des ADN.

La présente invention a encore pour objet des oligonucléotides de type ARNi (Acide Ribonucléique Interférant) comprenant de 15 à 60 nucléotides, caractérisés en ce qu'ils hybrident de manière spécifique à la séquence SEQ ID N° 21 et inhibent l'expression de OB-RGRP, et caractérisés en ce qu'ils sont des ARN double-brins dont au moins l'un des deux brins comprend une séquence présentant une identité d'au moins 90 %, avec l'une des séquences SEQ ID N°37 ou SEQ ID N°38.

La présente invention a également pour objet des oligonucléotides de type ARNi comprenant de 15 à 60 nucléotides qui hybrident de manière spécifique à la séquence SEQ ID NO: 21 et inhibent l'expression de OB-RGRP, caractérisés en ce qu'ils sont sous forme simple brin et qu'ils comprennent une séquence présentant une identité d'au moins 90%, avec la séquence SEQ ID N°42.

Préférentiellement de tels ARNi comprennent 17 ou 19 nucléotides pris en continu dans la séquence SEQ ID NO : 21, ou dans sa séquence complémentaire.

Des nucléotides A(A/G) et (C/T)T peuvent être ajoutés respectivement en 5' et en 3' de cette séquence de 17 ou 19 nucléotides. D'autres types de résidus ou groupes chimiques peuvent néanmoins être ajoutés à ces deux extrémités, pour autant qu'ils ne diminuent pas l'activité des antisens.

Les modifications de nucléotides décrites pour les antisens sont aussi possibles pour ceux entrant dans la composition des sARNi.

La présente invention inclut également toutes modifications des antisens ou des ARNi, visant à augmenter la résistance de ces composés aux nucléases cellulaires, ou leur pénétration dans les cellules et/ou leur efficacité dans le ciblage de la séquence d'OB-RGRP.

Lorsqu'ils sont des ADN, les oligonucléotides selon la présente invention peuvent être réalisés commodément et en routine par la technique bien connue de la synthése en phase solide. L'équipement pour une telle synthèse est vendu par différentes sociétés spécialisées telles que Applied Biosystems (Foster City, CA). La synthèse des antisens dans la présente invention a fait appel à une synthèse chimique sur un support adapté selon les méthodes connues de l'homme du métier, en particulier décrites par E. Uhlmann, A. Peyman, A. Ryte, A. Schmidt and E. Buddecke (1999, Methods in Enzymology 313: 268-284)et par E. Uhlmann (Recent advances in the medicinal chemistry of antisense oligonucleotides,Current Opinion of Drug Discovery and Develepment 3: 203-213, 2000). Tout autre procédé de synthèse connu de l'homme du métier peut aussi être employé.

Lorsqu'ils sont des ARNi les oligonucléotides selon la présente invention peuvent être synthétisés par synthèse chimique, lorsqu'il s'agit de ARNi synthétiques, exprimés in situ à l'aide de vecteurs permettant la synthèse de tels oligonucléotides ou obtenus par clivage in vitro d'un ARN double brin par la RNAse III ou l'enzyme DICER.

Les ARNis (ARNi small ou petits ARNi) peuvent être obtenus auprès de différents fournisseurs comme Proligo (Proligo France SAS 1 rue Robert et Sonia Delaunay 75011 Paris) Dharmacon (Dharmacon, Inc. 1376 Miners Drive #101 Lafayette, CO 80026) et Ambion (Ambion (Europe) Ltd. Ermine Business Park Spitfire Close Huntingdon, Cambridgeshire PE29 6XY United Kingdom), ou peuvent être synthétisés à partir de kits commercialisés par différentes sociétés comme Dharmacon et Ambion.

Préférentiellement les ARNi selon la présente invention sont sous forme double brin.

Après synthèse les ARNi sont tout d'abord repris dans de l'eau dépourvue de RNAses. L'appariement des deux molécules simple brin peut être réalisé comme suit : 20 µmol.L-1 de chaque brin est mélangé dans le tampon d'appariement (100 mmol.L-1 d'acétate de potassium, 30 mmol.L-1 d'HEPES-KOH pH 7,4, 2 mmol.L-1 d'acétate de magnésium) puis chauffé à 90°C pendant 1 min suivit par une incubation d'1 h à 37°C.

La transfection des ARNis peut se faire par le même protocole que pour la transfection des antisens.

Une alternative pour le ARNi est l'utilisation de vecteurs permettant la synthèse d'ARN antisens spécifiques du gène à éteindre et qui vont s'apparier dans les cellules transfectées pour donner un ARNis. Un premier système de vecteur permet l'expression d'une séquence antisens par deux promoteurs en sens inverse, de chaque coté de cette séquence, donnant naissance à deux ARN complémentaires qui vont s'apparier dans les cellules transfectées et donner un ARNis. Un autre système de vecteur met en jeu la synthèse d'un ARN présentant la séquence de l'antisens suivie de la séquence sens, espacée par quelques nucléotides, qui va créer une structure d'ARN en épingle a cheveu, qui va être clivée dans les cellules transfectées pour donner un ARNis. Encore un autre systeme de vecteur met en jeu l'expression d'un ARN double brin, long jusqu'a 600 paires de bases qui ne peut sortir du noyau faute des séquences nécessaires : pas de coiffe en 3' (site ribozyme), ni de queue poly-A en 5' (site de fixation de la protéine à doigt de zing MAZ). Cet ARN long est clivé dans le noyau pour donner des ARNis fonctionnels qui vont sortir dans le cytoplasme et provoquer la dégradation de l'ARN cible.

La transfection de ces vecteurs s'effectue de manière classique comme décrit ci-avant pour les différents ADN. L'obtention de lignées stables qui présentent une extinction du gène cible est réalisable par une sélection par antibiotique classiquement utilisée pour l'obtention de lignées.

De manière générale, l'homme du métier peut se référer pour les ARNI aux publications suivantes : Elbashir S.M. et al. (2001, Nature 411: 494-498), Elbashir S.M. Lendeckel W. and Tuschl T. (2001, Genes & Dev. 15:188-200) et Masters J.R., et al. (2001, Proc. Natl. Acad. Sci. USA 98: 8012-8017).

Des vecteurs permettant l'expression des ARNi peuvent être obtenus comme décrit par Brummelkamp T.R., Bernards R., Agami R. (2002. Science 296: 550-553), Yu J.Y., DeRuiter S.L., and Turner D. (2002, Proc. Natl. Acad. Sci. USA 99: 6047-6052) et Shinagawa T. et Ishii S. (2003, Genes & Dev. 17:1340-1345),

De tels vecteurs, ainsi que des cellules contenant de tels vecteurs sont des objets de la présente demande.

Par ailleurs, la présente invention divulgue des médicaments contenant de tels oligonucléolides, vecteurs et cellules et des compositions pharmaceutiques contenant une quantité pharmacologiquement active de tels oligonucléotides, vecteurs et cellules et des excipients pharmaceutiquement acceptables.

La présente invention divulgue également l'utilisation de tels oligonucléotides, vecteurs et cellules pour la fabrication d'un médicament pour la prévention et /ou le traitement de pathologies liées à la leptine.

Elle divulgue de plus un procédé de traitement curatif ou préventif de maladies liées à la leptine consistant à administrer de tels oligonucléotides, vecteurs et cellules à un patient atteint par ladite maladie.

L'invention divulgue également un procédé de détermination de la modification de l'interaction entre la OB-RGRP ou la protéine MYO47, ou une protéine présentant une identité d'au moins 65 % avec cette protéine ou avec la protéine MYO47, et le récepteur de la leptine par un composé.

Elle divulgue en outre des protéines de fusion pour la mise en oeuvre de ce procédé, ainsi des acides nucléiques codant ces protéines.

Elle divulgue de plus un procédé de traitement curatif ou préventif de maladies liées à la leptine consistant à administrer un ligand sélectionné par le procédé défini ci-dessus à un patient atteint par la dite maladie.

Une protéine de fusion caractérisée en ce qu'elle est composée d'une séquence présentant une identité d'au moins 65 % avec la séquence SEQ ID N°4, ou la séquence SEQ ID N°16, ou d'une partie substantielle de la séquence SEQ ID N°4 ou de la séquence SEQ ID N°16, et d'une protéine donneur d'énergie ou accepteur d'énergie, ou d'une partie substantielle et active d'une protéine donneur d'énergie ou accepteur d'énergie, est également divulguée.

De telles protéines de fusion sont composées en substance d'une partie correspondant à une partie ou la totalité d'une séquence présentant une identité d'au moins 65 % préférentiellement d'au moins 75 %, et encore plus préférentiellement d'au moins 85 % ou 95 %, avec la séquence SEQ ID N°4 ou la séquence SEQ ID N°16, ou d'une partie substantielle de la séquence SEQ ID N°4 ou de la séquence SEQ ID N°16, et d'une partie correspondant à une protéine donneur ou accepteur d'énergie. Elles peuvent néanmoins comprendre d'autres séquences d'acides aminés, issues d'autres protéines, telles que des séquences signal.

De manière avantageuse la protéine donneur d'énergie est la luciférase de Renilla (Rluc). Elle peut néanmoins être toute autre protéine donneur d'énergie telle que le spectre d'émission du donneur chevauche suffisamment le spectre d'excitation de l'accepteur pour permettre un transfert d'énergie efficace entre les deux partenaires. Elle peut ainsi être la GFP, si le transfert d'énergie est le FRET, ou encore l'aequorine si le transfert d'énergie est le CRET. L'aequorine peut être obtenue et utilisée comme décrit dans la demande de brevet EP0 187 519, ou dans l'article de Inouye et al. (PNAS USA 82 : 3154-3158 (1985)).

La protéine fluorescente accepteur d'énergie est quant à elle préférentiellement la DsRed, la GFP ou un mutant de cette protéine, tel que la YFP, l'EYFP, la GFP sauvage, la GFPS65T, la Topaz, ou la GFP₁₀.

Elle peut néanmoins être toute autre protéine fluorescente accepteur d'énergie telle que le spectre d'excitation de l'accepteur et le spectre d'émission du donneur se chevauchent suffisamment pour permettre un transfert d 'énergie efficace entre les deux partenaires.

Ces protéines sont connues de l'homme du métier qui peut trouver leurs séquences dans la littérature, notamment dans le revue de Blinks et al. (Pharmacol. Rev. 28 : 1-93 (1976)). En particulier la GFP est décrite par Tsien (Annu. Rev. Biochem. 67 : 509-544 (1998)) et son clonage par Prasher et al. (Gene 111 : 229-233 (1992)). Le clonage de la DsRed est quant à lui décrit par Matz et al. (Nat. Biotechnol. 17 : 969-973 (1999)). Pour la Rluc, l'homme du métier peut se référer à Blinks et al. (Pharmacol. Rev. 28 : 1-93 (1976)) ou encore à Lorenz et al. (PNAS 88: 4438-4442 (1991)).

De manière particulièrement avantageuse les protéines de fusion donneur et accepteur présentent l'une des séquences SEQID N°6, SEQID N°8, SEQID N°12, SEQ ID N°14, SEQID N°18 ou SEQID N°20 ou un variant de cette séquence présentant une identité d'au moins 65%.

La présente invention divulgue aussi des acides nucléiques codant pour ces protéines. De tels acides nucléiques peuvent être des ADN complémentaires ou génomiques, ou des ARN. Ces acides nucléiques ou polynucléotides peuvent être sous forme simple chaîne ou sous la forme de duplex.

Ils sont de manière particulièrement avantageuse des ADN complémentaires. De manière préférentielle un tel acide nucléique a une identité d'au moins 65 %, préférentiellement d'au moins 75 %, et encore plus préférentiellement d'au moins 85 % ou 95 %, d'identité en nucléotides avec un acide nucléique de séquence SEQ ID N°5, SEQ ID N°7, SEQ ID N°11, SEQ ID N°13, SEQ ID N°17 ou SEQ ID N°19.

L'invention divulgue encore un acide nucléique hybridant, dans des conditions d'hybridation de forte stringence, avec un acide nucléique tel que défini ci-avant, et plus particulièrement un acide nucléique de séquences nucléotidiques SEQ ID N°5, SEQ ID N°7, SEQ ID N°11, SEQ ID N°13, SEQ ID N°17 ou SEQ ID N°19, ou un acide nucléique de séquence complémentaire.

Le « pourcentage d'identité » entre deux séquences de nucléotides ou d'acides aminés, au sens de la présente invention, peut être déterminé en comparant deux séquences alignées de manière optimale, à travers une fenêtre de comparaison.

La partie de la séquence nucléotidique ou polypeptide dans la fenêtre de comparaison peut ainsi comprendre des additions ou des délétions (par exemple des « gaps ») par rapport à la séquence de référence (qui ne comprend pas ces additions ou ces délétions) de manière à obtenir un alignement optimal des deux séquences.

Le pourcentage est calculé en déterminant le nombre de positions auxquelles une base nucléique ou un résidu d'aminoacide identique est observé pour les deux séquences (nucléique ou peptidique) comparées, puis en divisant le nombre de positions auquel il y a identité entre les deux bases ou résidus d'aminoacides par le nombre total de positions dans la fenêtre de comparaison, puis en multipliant le résultat par 100 afin d'obtenir le pourcentage d'identité de séquence.

L'alignement optimal des séquences pour la comparaison peut être réalisé de manière informatique à l'aide d'algorithmes connus contenus dans le package de la Société WISCONSIN GENETICS SOFTWARE PACKAGE, GENETICS COMPUTER GROUP (GCG), 575 Science Doctor , Madison, WISCONSIN. A titre d'illustration, le pourcentage d'identité de séquence pourra être effectué à l'aide du logiciel BLAST (versions BLAST 1.4.9 de mars 1996, BLAST 2.0.4 de février 1998 et BLAST 2.0.6 de septembre 1998), en utilisant exclusivement les paramètres par défaut (S. F Altschul et al, J. Mol. Biol. 1990 215 : 403-410, S. F Altschul et al, Nucleic Acids Res. 1997 25 : 3389-3402). Blast recherche des séquences similaires/homologues à une séquence « requête » de référence, à l'aide de l'algorithme d'Altschul et al. La séquence requête et les bases de données utilisées peuvent être peptidiques ou nucléiques, toute combinaison étant possible.

Par « conditions d'hybridation de forte stringence » au sens de la présente invention, on entendra les conditions suivantes :

### 1- Compétition des membranes et PRE HYBRIDATION :

- Mélanger : 40µl ADN sperme de saumon (10mg/ml)+ 40 µl ADN placentaire humain (10mg/ml)
- Dénaturer 5 min à 96°C, puis plonger dans la glace le mélange.
- Oter le SSC 2X et verser 4 ml de mix formamide dans le tube d'hybridation contenant les membranes.
- Ajouter le mélange des deux ADNs dénaturés.
- Incubation à 42°C pendant 5 à 6 heures, avec rotation.

### 2- Compétition de la sonde marquée :

- Ajouter à la sonde marquée et purifiée 10 à 50 µl ADN Cot I, selon la quantité de répétitions.
- Dénaturer 7 à 10 mn à 95°C.
- Incuber à 65°C pendant 2 à 5 heures.

### 3- Hybridation:

- Oter le mix de pré hybridation.
- Mélanger 40 µl ADN sperme de saumon + 40 µl ADN placentaire humain ; dénaturer 5 mn à 96°C, puis plonger dans la glace.
- Ajouter dans le tube d'hybridation 4 ml de mix formamide, le mélange des deux ADN et la sonde marquée/ADN Cot I dénaturée.
- Incuber 15 à 20 heures à 42°C, avec rotation.

### 4- Lavages :

- Un lavage à température ambiante dans du SSC 2X, pour rincer.
- 2 fois 5 minutes à température ambiante SSC 2X et SDS 0,1% à 65°C.
- 2 fois 15 minutes à 65°C SSC 1X et SDS 0,1% à 65°C.

Envelopper les membranes dans du Saran et exposer.

Les conditions d'hybridation décrites plus haut sont adaptées à l'hybridation dans des conditions de forte stringence, d'une molécule d'acide nucléique d'une longueur variable de 20 nucléotides à plusieurs centaines de nucléotides.

Il va sans dire que les conditions d'hybridation ci-dessus décrites peuvent être adaptées en fonction de la longueur de l'acide nucléique dont l'hybridation est recherchée ou du type de marquage choisi, selon des techniques connues de l'homme du métier.

Les conditions convenables d'hybridation peuvent par exemple être adaptées selon l'enseignement contenu dans l'ouvrage de HAMES et HIGGINS (1985, "Nucleic acid hybridiation ; a practical approach", Hames and Higgins Ed., IRL Press, Oxford) ou encore dans l'ouvrage de F.AUSUBEL et al (1989, Current Protocols in Molecular Biology, Green Publishing Associates and Wiley Interscience, N.Y).

Les protéines divulguées dans la présente invention peuvent être obtenues par tous moyens connus de l'homme du métier. Elles sont néanmoins avantageusement obtenues par expression des acides nucléiques tels que décrits ci-dessus, codant pour ces protéines, éventuellement insérés dans des vecteurs d'expression, dans des cellules avantageusement choisies, suivie éventuellement par une extraction et une purification qui peut être totale ou partielle.

L'invention divulgue également un vecteur recombinant comprenant un acide nucléique décrit ci-dessus.

Avantageusement, un tel vecteur recombinant comprendra un acide nucléique choisi parmi les acides nucléiques suivants :
a) un acide nucléique codant pour une protéine ayant au moins 65 % d'identité en acides aminés avec une séquence SEQ ID N°6, SEQ ID N°8, SEQ ID N°18 ou SEQ ID N°20 ou un fragment peptidique ou un variant de cette dernière ;
b) un acide nucléique comprenant un polynucléotide ayant une séquence SEQ ID N°5, SEQ ID N°7, SEQ ID N°17 ou SEQ ID N°19, ou un fragment ou un variant de ce denier;
c) un acide ayant au moins 65% d'identité en nucléotides avec un acide nucléique ayant une séquence SEQ ID N°5, SEQ ID N°7, SEQ ID N°17 ou SEQ ID N°19 ou un fragment ou un variant de ce dernier ;
d) un acide nucléique hybridant, dans des conditions d'hybridation de forte stringence, avec un acide nucléique de séquences SEQ ID N°5, SEQ ID N°7, SEQ ID N°17 ou SEQ ID N°19, ou un fragment ou un variant de ce dernier.

Par « vecteur » au sens de la présente invention on entendra une molécule d'ADN ou d'ARN circulaire ou linéaire qui est indifféremment sous forme de simple brin ou double brin.

Le vecteur d'expression peut comprendre outre un acide nucléique conforme à l'invention, des séquences régulatrides permettant d'en diriger la transcription et/ou la traduction.

Un tel vecteur recombinant comprendra notamment les éléments suivants :
(1) des éléments de régulation de l'expression de l'acide nucléique à insérer, tels que des promoteurs et des enhanceurs ;
(2) la séquence codante comprise dans l'acide nucléique conforme à l'invention à insérer dans un tel vecteur, ladite séquence codante étant placée en phase avec les signaux de régulation décrits aux (1) ; et
(3) des séquence d'initiation et d'arrêt de la transcription appropriées.

En outre, les vecteurs recombinants pourront inclure une ou plusieurs origines de réplication chez les hôtes cellulaires dans lesquels leur amplification ou leur expression est recherchée, des marqueurs ou des marqueurs de sélection.

A titre d'exemples, les promoteurs pour cellules eucaryotes comprendront le promoteur de la thymidine kinase du virus HSV ou encore le promoteur de la métallothionéine-L de souris.

De manière générale, pour le choix d'un promoteur adapté, l'homme du métier pourra avantageusement se référer à l'ouvrage de SAMBROOK et al. (1989, "Molecular Cloning: A Laboratory Manual," 2nd ed., Cold Spring Harbor Laboratory Press, Cold Spring Harbor, NY.) ou encore aux techniques décrites par FULLER et al. (1996, Immunology in Current Protocols in Molecular Biology, Ausubel et al).

Les vecteurs préférés sont des plasmides, tels que par exemple les vecteurs pcDNA3 (Invitrogen), pQE70, pQE60, pQE9 (Qiagen), psiX174, pBluescript SA, pNH8A, pNH16A, pNH18A, pNH46A, pWLNEO, pSV2CAT, pOG44, pXTI, pSG(Stragene).

Il peut s'agir également de vecteurs de type *baculovirus* tel que le vecteur pVL1392/1393 (Pharmingen) utilisé pour transfecter les cellules de la lignée Sf9 (ATCC N°CRL 1711) dérivées de *Spodoptera frugiperda.*

Il peut encore s'agir de vecteurs adénoviraux tels que l'adénovirus humain de type 2 ou 5.

Un vecteur recombinant peut aussi être un vecteur rétroviral ou encore un vecteur adéno-associé (AAV). De tels vecteurs adéno-associés sont par exemple décrits par FLOTTE et al. (1992, Am. J, Respir. Ce/l Mol. Biol., 7 : 349-356.

La présente invention divulgue en outre des cellules comprenant une protéine, un acide nucléique ou un vecteur tels que décrits ci dessus, ou des fragments de ces cellules, des lysats de ces cellules ou encore des membranes de ces cellules.

De telles cellules peuvent être cellules isolées d'un organisme et cultivées dans un milieu de croissance adéquat. Elles sont néanmoins préférentiellement des lignées cellulaires. Ainsi de telles lignées sont de manière particulièrement avantageuse les lignées cellulaires HEK 293, COS (ATCC N°CRL 1650), COS-M6 et HeLa (ATCC N°CCL2), ou encore Cv 1 (ATCC N°CCL70), Sf-9 (ATCC N°CRL 1711), CHO (ATCC N°CCL-61) ou 3T3 (ATCC N°CRL-6361).

Les membranes de ces cellules peuvent être préparées par toute méthode connue de l'homme du métier.

Préférentiellement elles seront préparées par broyage mécanique des cellules puis centrifugation des suspensions obtenues, comme illustré dans les exemples qui suivent.

La présente invention divulgue en outre des compostions comprenant des cellules telles que décrites ci dessus et de la saponine.

La présente invention divulgue en outre un procédé de détermination de la modification de l'interaction entre la OB-RGRP, la protéine MY047, ou une protéine présentant une identité d'au moins 65 % avec la séquence SEQ ID N°4 ou la séquence SEQ ID N°16, et le récepteur de la leptine par un composé comprenant les étapes consistant à :
- mettre en contact ledit composé avec une protéine présentant une identité d'au moins 65 % avec la séquence SEQ ID N°4 ou la séquence SEQ ID N°16, et le récepteur de la leptine, ou des cellules, ou des fragments, ou des lysats, ou des membranes de cellules comprenant de telles protéines, et éventuellement un substrat enzymatique adéquat, et
- à mesurer l'interaction entre une protéine présentant une identité d'au moins 65% avec la séquence SEQ ID N°4 ou la séquence SEQ ID N°16, et le récepteur de la leptine.

De manière préférentielle, ledit composé est mis en contact avec une protéine de fusion donneur d'énergie, et une protéine de fusion accepteur d'énergie, ou des cellules, ou des fragments, ou des lysats, ou des membranes de cellules comprenant une telle protéine, et éventuellement un substrat enzymatique adéquat

Préférentiellement ledit procédé est mis en oeuvre avec des cellules traitées par un agent perméabilisant les cellules tel que la saponine.

Les protéines de fusion donneur d'énergie et les protéines de fusion accepteur d'énergie sont choisies de manière à ce que l'énergie résultant de l'activation du donneur puisse être transférée de manière efficace à l'accepteur.

Dans un mode de mise en oeuvre avantageux dudit procédé, la protéine de fusion donneur d'énergie est une protéine de fusion avec la luciférase ou une partie substantielle de la luciférase, auquel cas le substrat est avantageusement la coelenterazine.

Dans un mode de mise en oeuvre préférentiel dudit procédé, la protéine de fusion accepteur d'énergie est une protéine de fusion avec la YFP ou une partie substantielle de la YFP.

Dans un mode de mise en oeuvre avantageux dudit procédé, le transfert d'énergie mesuré en présence du composé à tester est comparé à celui mesuré en absence du composé à tester.

Dans un autre mode de mise en oeuvre avantageux dudit procédé le transfert d'énergie mesuré en présence du composé à tester et de la leptine (ou un ligand du récepteur), est comparé à celui mesuré en présence du composé en absence de leptine (ou un ligand du récepteur).

Préférentiellement le procédé est mis en oeuvre sur des membranes des cellules, telles que décrites ci dessus.

De manière préférentielle les protéines donneur et accepteur décrites ci-dessus sont choisies afin que le transfert d'énergie se fasse par BRET (pour Bioluminescence Résonance Energy Transfer ou ou transfert d'énergie de bioluminescence par résonance) de première ou deuxième génération, ou LRET (pour Luminescence Resonance Energy Transfer ou ou transfert d'énergie de luminescence par résonance). Néanmoins un tel transfert d'énergie peut être effectué par FRET (pour Fluorescence Résonance Energy Transfer ou transfert d'énergie de fluorescence par résonance) ou encore par CRET (pour Chemioluminescence Resonance Energy Transfer ou transfert d'énergie de chimioluminescence par résonance).

Quelque soit le type de transfert d'énergie les couples protéine de fusion donneur protéines de fusion accepteur d'énergie sont choisis afin de permettre un tel transfert,

Le BRET2 (2éme génération) consiste en un transfert d'énergie entre la luciférase de *Rénilla,* et une GFP mutante, la GFP₁₀, utilisant un substrat adéquate, la coelantérazine DeepblueC^{™} (Biosignal Packard).

Le CRET consiste en un transfert d'énergie entre l'aequorine, qui est une luciférase, et la GFP.

Le FRET consiste en un transfert d'énergie entre deux protéines de la famille des GFP ayant des spectres différents.

Pour la mise en oeuvre de ces transferts l'homme du métier peut se référer à Ramsay D et al. (Biochem J 365: 429-40 (2002)) et à Yoshioka K et al. (FEBS Lett 523: 147-151 (2002)) pour le BRET2, à Baubet et al. (PNAS USA 97 : 7260-7265 (2000)) pour le CRET, à Matyus (J Photochem Photobiol B 12: 323-337 (1992)) et Pollok et Heim (Trends Cell Biol 9:57-60 (1999)) pour le FRET.

La présente invention divulgue aussi un procédé de criblage ou de détection de composés destinées à la prévention et / ou au traitement de pathologies liées à la leptine comprenant les étapes consistant à :
mettre en contact ledit composé avec une protéine présentant une identité d'au moins 65 % avec la séquence SEQ ID N°4 ou la séquence SEQ ID N°16, et le récepteur de la leptine, ou des cellules, ou des fragments, ou des lysats, ou des membranes de cellules comprenant de telles protéines, et éventuellement un substrat enzymatique adéquat, et
-à mesurer l'interaction entre une protéine présentant une identité d'au moins 65 % avec la séquence SEQ ID N°4 ou la séquence SEQ ID N°16, et le récepteur de la leptine.

Préférentiellement la protéine présentant une identité d'au moins 65 avec la séquence SEQ ID N°4 ou la séquence SEQ ID N°16 est la OB-RGRP ou MY047.

Ce procédé est compatible avec les plaques à 96 ou 384 puits généralement utilisées, Il ne nécessite pas l'utilisation de molécules radioactives, est sensible, reproductible, rapide et le résultat est facile à lire. Cette caractéristique est particulièrement intéressante pour la mise en oeuvre de criblage à grande échelle.

La présente invention divulgue en outre l'utilisation de composés sélectionnés par un procédé consistant à :
mettre en contact ledit composé avec une protéine présentant une identité d'au moins 65 % avec la séquence SEQ ID N°4 ou la séquence SEQ ID N°16, et le récepteur de la leptine, ou des cellules, ou des fragments, ou des lysats, ou des membranes de cellules comprenant de telles protéines, et éventuellement un substrat enzymatique adéquat, et
-à mesurer l'interaction entre une protéine présentant une identité d'au moins 65 % avec la séquence SEQ ID N°4 ou la séquence SEQ ID N°16, et le récepteur de la leptine.

Elle divulgue enfin un procédé de traitement curatif ou préventif de maladies liées à la leptine ou à son récepteur comprenant les étapes de :
- sélection dudit composé par un procédé consistant à :
   +mettre en contact ledit composé avec une protéine présentant une identité d'au moins 65 % avec la séquence SEQ ID N°4 ou la séquence SEQ ID N°16, et le récepteur de la leptine, ou des cellules, ou des fragments, ou des lysats, ou des membranes de cellules comprenant de telles protéines, et éventuellement un substrat enzymatique adéquat, et
   +à mesurer l'interaction entre une protéine présentant une identité d'au moins 65 % avec la séquence SEQ ID N°4 ou la séquence SEQ ID N°16 et le récepteur de la leptine
- d'administration dudit composé à un patient atteint par la dite maladie.

Des pathologies liées à la leptine peuvent être des maladies liées à une diminution de la densité osseuse comme par exemple l'ostéoporose ou à l'inverse celles liées à une calcification importante.

Elles peuvent aussi être des maladies ayant un effet sur le poids, telles que l'obésité, le diabète ou l'anorexie.

Elles peuvent aussi être des maladies ayant un effet sur la maturation sexuelle l'hématopoïèse, l'angiogenèse, la formation de thrombus, la régulation de l'immunité et de l'inflammation, le développement foetal, la cicatrication et le cancer.

Les composés de l'invention, oligonucléotides et ARNi, peuvent être formulés dans des compositions pharmaceutiques en vue d'une administration par voie topique, orale, parentérale, intranasale, intraveineuse, intramusculaire, sous-cutanée, intraoculaire, etc. Préférentiellement, les compositions pharmaceutiques contiennent des véhicules pharmaceutiquement acceptables pour une formulation injectable. Il peut s'agir en particulier de solutions salines (phosphate monosodique, disodique, chlorure de sodium, potassium, calcium ou magnésium, etc, ou des mélanges de tels sels), stériles, isotoniques, ou de compositions sèches, notamment lyophilisées, qui, par addition selon le cas d'eau stérilisée ou de sérum physiologique, permettent la constitution de solutés injectables.

La formulation de compositions thérapeutiques et leur administration est dans les compétences de l'homme du métier.

La formulation des composés peut inclure différents produits connus de l'homme du métier. Préférentiellement, les composés peuvent être par exemple additionnés de sels, comme le sodium, le potassium, l'ammonium, le magnésium, le calcium, les polyamines, l'acide hydrochlorique, hydrobromique, sulfurique, phosphorique, ou nitrique. D'autres sels sont également utilisables, comme ceux provenant de l'acide acétique, oxalique, tartrique, succinique, maléique, fumarique, gluconique, citrique, malique, ascorbique, benzoïque, tannique, palmitique, alginique, polyglutamique, naphtalènesulfonique, methanesulfonique, p-toluenesulfonique, naphtalènedisulfonique, polygalacturonique. Enfin, on peut aussi préférentiellement utiliser des sels de chlorine, bromine et iodine.

La composition et la formulation pour l'administration topique peuvent inclure des patches transdermiques, des pommades, des lotions, des crèmes, des gels, des gouttes, des suppositoires, des sprays, des liquides et des poudres.

La composition et la formulation pour l'administration orale peuvent inclure des poudres, des granules, des microparticules, des nano particules, des suspensions, des solutions aqueuses ou non, des capsules, des capsules de gel, des sachets, des tablettes ou mini tablettes. Des épaississants, des arômes, des diluants, des emulsifieurs, des aides à la dispersion ou des liants peuvent être ajoutés.

La composition et la formulation pour l'administration parentérale, intrathécale ou intra ventriculaire, peut inclure des solutions aqueuses stériles qui peuvent aussi contenir des tampons, des diluants et autres additifs comme, mais non limité à des agents augmentant la pénétration, des produits transporteurs et des excipients.

La composition peut-être formulée et utilisée comme une mousse, une émulsion, une microémulsion, des liposomes cationique, sensibles au pH ou négativement chargés, et des transféromes.

D'une façon générale, les différentes formulations peuvent contenir un mélange d'un ou plusieurs agents, comme mais non limité à des agents augmentant la pénétration du composé (surfactants, sels biliaires, agents chélateurs, surfactant non chélateurs), des excipients (liants, remplisseurs, lubrifiants, désintégrants, agents mouillant), des transporteurs (eau, solutions salines, alcools, polyéthylène glycol, gélatine, lactose, amylose, stéarate de magnésium, talc, acide silicique, paraffine visqueuse, hydroxyméthylcellulose, polyvinylpyrrolidone). D'autres composants peuvent être ajoutés, comme des colorants, des arômes, des conservateurs, des antioxydants, des opacifieurs, des agents épaississeurs et des stabilisateurs.

Le dosage est dépendant de la sévérité et de la sensibilité de l'état de la maladie a traiter, avec une durée de traitement pouvant aller de quelques jours à quelques mois, ou jusqu'à ce que la cure soit efficace ou qu'une diminution de la maladie soit observée. Le dosage optimal peut être calculé à partir de mesures d'accumulation de l'agent thérapeutique dans le corps du patient. L'homme du métier peut facilement déterminer les dosages optimum, les méthodes de dosage et les taux de répétitions de ces dosages. Les dosages optimum peuvent varier en fonction de l'efficacité relative de chaque oligonucléotide ou ARNi, et peuvent en général être estimés par la mesure des EC50 des doses utilisées in vitro et in vivo dans des modèles animaux. En général, le dosage est compris entre 0,01µg et 100 g par kilo du poids corporel et peut être administré une fois ou plus, de façon journalière, hebdomadaire, mensuelle ou annuelle, ou même une fois toutes les 2 à 20 années.

Des personnes compétentes peuvent facilement déterminer le taux de répétition des dosages basé sur le temps de présence du composé dans les fluides corporels ou les tissus. Suite à un traitement réussit, il peut être désirable que le patient continue une thérapie de maintenance pour prévenir la réapparition de la maladie, pour ce faire l'oligonucléotide ou l'ARNi sont administrés à des doses de maintenance allant de 0,01 µg à 100 g par kilo du poids corporel, une fois ou plus par jour jusqu'à une fois tous les 20 ans.

L'administration d'antisens in vivo a été réalisée avec succès par divers auteurs, utilisant des protocoles d'injection simple d'antisens par voie intraveineuse (He et al. (1998) Zhonghua Shi Yan He Lin Chuang Bing Du Xue Za Zhi 12:1-4) ou intracérébrale (Yoburn et al. (2003) Synapse 47: 109-116, Tischkau et al. (2003) J. Biol. Chem. 278: 718-723). Ces deux dernières années, des systèmes plus complexes permettant le ciblage d'antisens dans l'organisme ont été développés et utilisés avec succès (Morishita et al. (2002) J. Endocrinol. 175: 475-485, Bartsch et al. (2002) Pharm. Res. 19: 676-680), permettant chez la souris et le rat, de traiter divers cancers (Rait et al. (2002) Mol. Med. 8: 475-486, Ochietti et al. (2002) J. Drug. Target 10: 113-121, Eder et al. (2002) Cancer Gene Ther. 9:117-125). La transfection des antisens met en jeu les mêmes procédés que pour la transfection des ARNi, laissant envisager les mêmes applications in vivo pour les ARNi. Dans cette optique, nous pouvons imaginer un ciblage de l'antisens ou des ARNi au niveau du système nerveux central, pour traiter des troubles dont l'origine est centrale (obésité), mais aussi ceux obtenus par une action périphérique des récepteurs de la leptine. Plus particulièrement, une action des antisens ou des ARNi au niveau du transport de la leptine au travers de la barrière hémato-encéphalique et impliquant les OB-R peut être envisagée. D'ailleurs, les cellules endothéliales ont déjà été ciblées avec succès par une stratégie antisens in vivo (Bartsch et al. (2002) Pharm. Res. 19: 676-680).

### Figures :

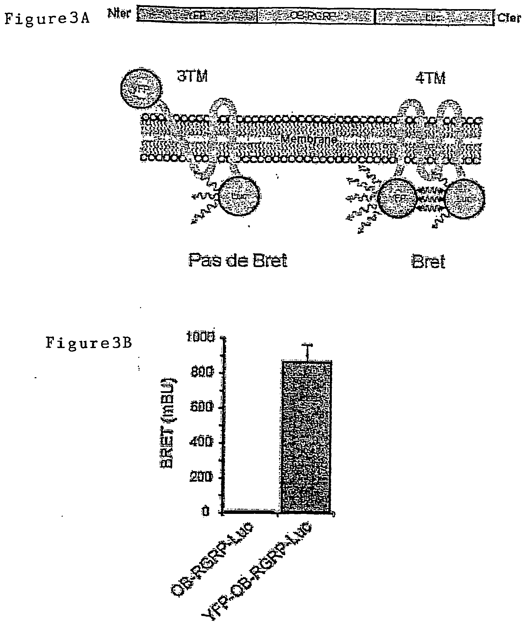

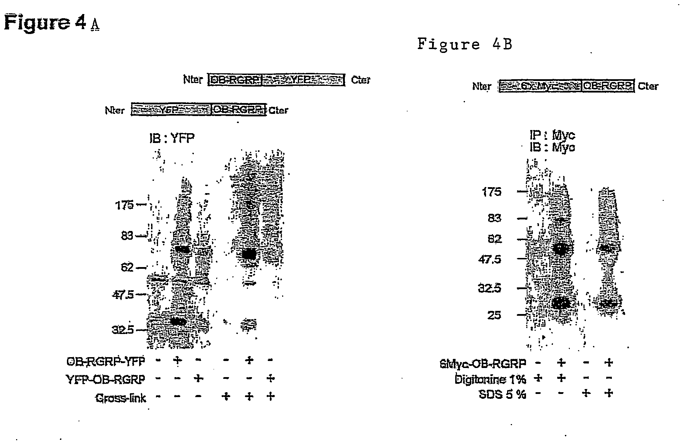

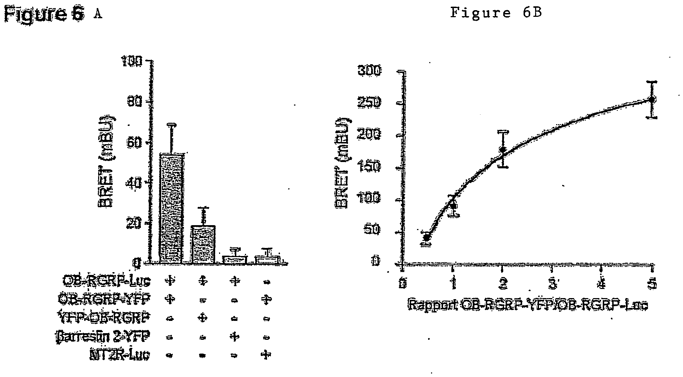

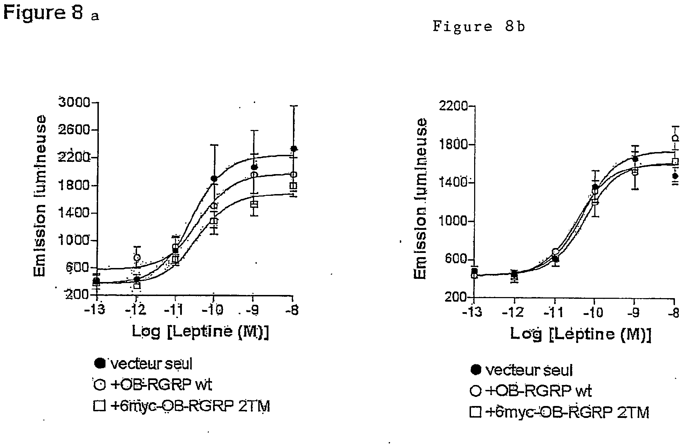

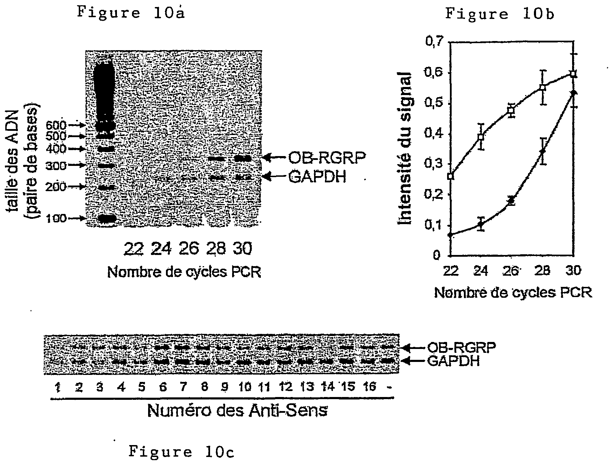

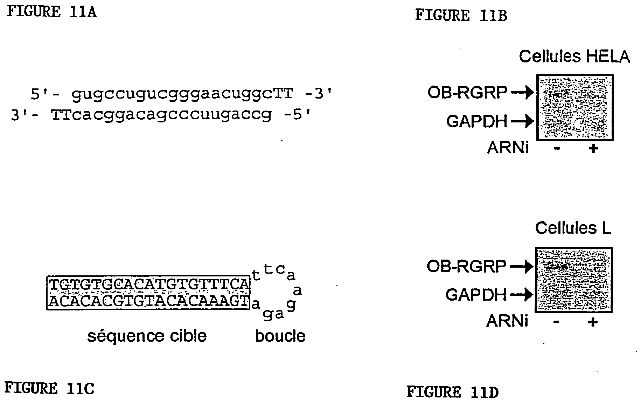

**Figure 1**
   Séquences des différents ODN antisens utilisés AS 01 à AS 16.
**Figure 2**
   Alignement des séquences protéiques d'OB-RGRP de différentes espèces et de la séquence protéique humaine de MY047. Les domaines transmembranaires potentiels ont été déterminés par différentes méthodes (HMMTOP, TMHMM, TopPred2, TMpred) et sont écrites en gras.
**Figure 3**
   Topologie d'OB-RGRP étudiée par BRET, par l'utilisation de la double protéine de fusion YFP-OB-RGRP-Luc. Figure 3a: représentation schématique de la topologie d'OB-RGRP pour le modèles 3 et 4TM. Figure 3b : Résultats des expériences de BRET utilisant les protéines indiquées. Les données sont exprimées en mBU.
**Figure 4**
   Etude de l'oligomérisation d'OB-RGRP par des expériences de SDS-PAGE et immunoprécipitations. Figure 4a: Les cellules exprimant les protéines de fusion indiquées ont été traitées ou non avec du Dithiobis(succinimidyl propionate) (DSP) 2 mmol.L-1 dans du PBS (1X pH7,4) pour cross-linker les complexes protéiques. Les protéines ont été séparées par SDS-PAGE et les protéines de fusions avec la YFP ont été détectées par l'utilisation d'un anticorps spécifique anti-YFP. Figure 4b: Les cellules exprimant la construction 6Myc-OB-RGRP ont été solubilisées avec 1 % de digitonine ou 5 % de SDS et le solubilisat a été immunoprécipité avec un anticorps anti-myc. Les précipitats ont été soumis à une séparation par SDS-PAGE et les protéines étiquetées avec myc ont été détectées avec un anticorps anti-myc.
**Figure 5**
   Identification des déterminants moléculaires impliqués dans l'oligomérisation d'OB-RGRP. Les protéines de fusions avec les troncations d'OB-RGRP ont été traitées comme décrit Fig. 4b. TM, domaine transmembranaire.
**Figure 6**
   Etude de l'oligomérisation d'OB-RGRP dans les cellules HEK vivantes par la technologie de BRET. Figure 6a: Les protéines de fusion indiquées ont été co-exprimées à un rapport équimolaire, et des expériences de mesures de BRET ont été réalisées. Figure 6b: Des quantités constantes du plasmide OB-RGRP-Luc ont été co-exprimées avec des quantités croissantes du plasmide d'OB-RGRP-YFP et des mesures de BRET ont été réalisées. MT2R-Luc, protéine de fusion du récepteur MT2 de la mélatonine avec la luciférase.
**Figure 7**
   Interaction d'OB-Rₛ et d'OB-RGRP et MY047 étudiées par BRET.Les protéines de fusions indiquées ont été co-exprimées à un rapport équimolaire et des mesures de BRET ont été réalisées. IR-YFP, protéine de fusion du récepteur de l'insuline avec la YFP.
**Figure 8**
   Activation dose dépendante des gènes rapporteurs pour STAT3 (Figure 8a) et STAT5 (Figure 8b) dans des cellules HeLa, par OB-R_{I} en présence d'une sur-expression des constructions de la protéine OB-RGRP comme indiquées.
**Figure 9**
   Effet de la surexpression d'OB-RGRP sur l'expression des OB-R à la surface des cellules. Des cellules HEK 293 transfectées ou non avec le vecteur d'expression d'OB-RGRP, et des cellules COS transfectées avec les vecteurs d'expression d'OB-Rᵢ ou OB-Rₛ et +/- les vecteurs d'OB-RGRP, ont été utilisées pour déterminer la quantité de récepteurs exprimés à la surface et le total exprimé dans les cellules par des expériences de liaison de ¹²⁵I-leptine.
**Figure 10**
   Effet des différents oligodésoxynucléotides (ODN) antisens sur le niveau des messagers d'OB-RGRP observés par RT-PCR semi-quantitative. Figure 10a: détermination de la zone linéaire d'amplification des transcrits d'OB-RGRP et de la GAPDH, en fonction du nombre de cycles PCR. Figure 10b: quantification des résultats montrés dans le panneau a. Figure 10c: Détermination des niveaux d'expression relatifs des ARNm d'OB-RGRP à 26 cycles PCR, dans les cellules incubées avec les différents ODN antisens.
**Figures 11**
   Effet des différents ARN interférant sur le niveau des messagers d'OB-RGRP observés par RT-PCR semi-quantitative. **Figure 11a**: séquence SEQ ID N°37 /SEQ ID N°38 de l'ARNi synthétique utilisé (homologue pour l'homme et la souris). **Figure 11**b: détermination des niveaux d'expression relatifs des ARNm d'OB-RGRP à 26 cycles PCR, dans des cellules HELA transfectées ou non avec l'ARNi synthétique. **Figure 11**c: séquence SEQ ID N°42 de l'ARNi en épingle à cheveux synthétisé à partir du vecteur PCR3.1-RNAi 14. **Figure 11**d: détermination des niveaux d'expression relatifs des ARNm d'OB-RGRP à 26 cycles PCR, dans des cellules Ltk transfectées ou non avec le vecteur PCR3.1-RNAi 14.
**Figure 12**
   Effet des ODN antisens spécifiques d'OB-RGRP sur l'activation d'un gène rapporteur STAT3. Les cellules HeLa ont été co-transfectées, d'abord avec le vecteur d'expression OB-RI et les constructions des gènes rapporteurs pour STAT3 ou 5, puis avec les ODN antisens indiqués. Après 48 heures de stimulation ou non avec 10 nmol.L-1 de leptine.
**Figure 13**
   Effet des ODN antisens spécifiques d'OB-RGRP sur l'expression en surface des OB-R. Les cellules HeLa ont été transfectées ou non avec les plasmides d'expression d' OB-R_{I} ou d'OB-Rₛ avant une seconde transfectioh ou non avec les ODN antisens indiqués. 48 h post-transfection, la quantités d'OB-R totale et la fraction exposée à la surface a été déterminée dans des expériences de liaison avec de la ¹²⁵I-leptine.

### Matériels et Méthodes utilisés dans les exemples

### Construction des plasmides.

Les protéines de fusions des OB-R avec la YFP et la luciférase ont été construites par ligation de la YFP et de la Luciférase à la partie C-terminale des récepteurs d'OB-R, par des techniques standards de biologie moléculaire. La région codante de la YFP a été obtenue à partir du vecteur Cytogem®-Topaze (pGFPtpz-N1) (Packard, Meriden, CT) et fut insérée dans le site EcoRV du vecteur pcDNA3/CMV (Invitrogen, Groningen, Pays-bas) contenant un polylinker modifié. La région codante de la *Renilla* luciferase a été obtenue à partir du vecteur pRL-CMV (Promega, Madison, WI) et insérée dans le site EcoRV du vecteur pcDNA3 modifié. Les régions codantes d'OB-Rₗ et d'OB-Rₛ (gift of Dr. Gainsford, Royal Melbourne Hospital, Victoria, Australie) ont été insérée dans les deux vecteurs décrits ci-dessus, respectivement dans les sites EcoR1/BamH1 et Nhe1. Les codons stop ont été supprimés par mutagenèse dirigée et la phase des protéines de fusion a été ajustée en même temps.

Le vecteur pcDNA3-OB-RGRP a été obtenu par insertion de la région codante d'OB-RGRP, obtenue depuis le vecteur pCDNA3-Di1, dans les sites EcoR1 et Xba1 du vecteur pcDNA3/CMV (Invitrogen, Groningen, Pays-bas). Le codon stop d'OB-RGRP a été supprimé par mutagenèse dirigée. Le vecteur pcDNA3-OB-RGRP-Luc a été obtenu par digestion du vecteur pRL-CMV N3 (Promega, Madison, WI) avec Sma1 et Hpa1 et par insertion du fragment, correspondant à la région codante de la *Renilla* luciferase, après la région codante d'OB-RGRP dans le site BspE1 rempli du vecteur pcDNA3-OB-RGRP.

Le vecteur pcDNA3-YFP a été obtenu par sous-clonage de la région codante de la YFP à partir du vecteur pGFPtpz-N1 (Packard, Meriden, CT) inséré dans le site EcoRV du vecteur pcDNA3/CMV. Le vecteur pcDNA3-OB-RGRP-YFP a été obtenu par insertion du fragment BamH1/BspE1 du vecteur pCDNA3-OB-RGRP non-stop dans le vecteur pcDNA3-YFP digéré avec les enzymes BamH1 et Age1.

La construction pcDNA3-GFP-OB-RGRP-Luc a été obtenue par insertion du fragment OB-RGRP-Luc du vecteur pcDNA3-OB-RGRP-Rluc coupé par EcoR1, dans le site EcoR1 du vecteur pcDNA3-YFP. Le codon stop de la YFP a été éliminé par mutagenèse dirigée.

Le vecteur 6Myc-OBR-GRP (4TM) a été obtenu par insertion du fragment 6myc du vecteur pCDNA3-RSV-6Myc, dans les sites BamH1 et EcoR1 du vecteur pCDNA3-OBRGRP. Les différentes délétions d'OB-RGRP (2 et 3 TM) ont été obtenus par PCR et insertion dans le vecteur pcDNA3 dans les sites EcoR1 et Xba1. La séquence codante de MY047 a été obtenue par RT-PCR sur des ARNm d'origine humaine. Le fragment PCR a été digéré par les enzymes de restriction EcoR1/Xba1 et inséré dans le vecteur pcDNA3-Topaze coupé par les mêmes enzymes. Le codon stop de la YFP a ensuite été éliminé par mutagénèse dirigée pour obtenir le vecteur pcDNA3-YFP-MY047. Le vecteur pcDNA3-MY047-YFP à été obtenu par insertion du fragment d'ADN obtenu par PCR sur le vecteur pcDNA3-YFP-MY047 et coupé par BamH1, puis inséré dans le vecteur pcDNA3-YFP coupé par la même enzyme. L'insertion du même fragment dans le vecteur pcDNA3-Rluc coupé par BamH1 a permit d'obtenir le vecteur pcDNA3-MY047-Rluc. Deux réactions de PCR consécutives ont étés réalisées pour obtenir une amplification du promoteur U6 de souris suivi de la séquence ARNi en épingle à cheveux de séquence SEQ ID N°42. Dans la première réaction, un premier couple d'amorces : U6 sens, 5'-CCATCTAGGCCAAGCTTATCCGACGCCGCCATCTC-3' SEQ ID N°41 et celle correspondant à la séquence sens de la cible suivie d'une séquence formant la boucle (SEQ ID N°39). Ce produit de PCR a ensuite été utilisé dans une seconde réaction avec le même primer sens (U6 sens) et avec une seconde amorce correspondant à la séquence anti-sens de la cible précédée de la même séquence formant la boucle (SEQ ID N°40). Les différents produits de PCR correspondant aux différents ARNi en épingle à cheveu ont été inséré dans le vecteur PCR3.1 à l'aide du kit TA-cloning (INVITROGEN Groningen, Pays-bas), pour donner les plasmides PCR3.1-RNAi 14. Toutes les constructions ont été vérifiées par séquençage.

### Culture cellulaire et transfection.

Les cellules HEK 293, COS-7- et HeLa ont été cultivées dans du DMEM supplémenté avec 10 % (v/v) de SVF, 4,5 g/litre de glucose, 100 U/ml de pénicilline, 0,1 mg/ml de streptomycine, 1 mmol.L-1 de glutamine (tous de Life Technologies, Gaithersburg, MD). Les transfections transitoires ont été réalisées avec le réactif FuGene 6 (Roche, Basel, Suisse) selon les instructions du fournisseur, sauf pour les cellules Ltk. Ces dernières ont été transfectées par la technique de DEAE Dextran : les cellules sont rincées deux fois au PBS puis 1 ml d'un mélange de 2µg d'ADN, DMEM, Hepes 20 mM, 4,5 g/litre de glucose et 200µg de DEAE-dextran est ajouté sur les cellules. Après une incubation de 8 heures, le milieu est retiré puis les cellules sont incubées pendant 1'30"avec 1 ml de DMEM, 4,5 g/litre de glucose et 10 % DMSO. Les cellules sont finalement rincées puis incubées avec le milieu de culture.

### Préparation des membranes et solubilisation.

Les membranes ont été préparées comme décrit précédemment (19), et resuspendues dans du Tris 75 mmol.L-1 (pH 7,4), du MgCl₂ 12,5 mmol.L-1, et EDTA 5 mmol.L-1 et immédiatement utilisées dans des expériences de BRET.

### SDS PAGE et Western blot.

Les lysats totaux ont étés préparés par lavage des cellules une fois au PBS froid (pH 7,4) et dénaturés par l'ajout de tampon de dépôt (30mmol.L-1 Tris HCl pH 6,8, 1 % glycérol 5 % SDS, 50 mmol.L-1 DTT et 0,05 % bromophenol blue). Les lysats totaux ou les immunoprécipités ont été incubés pendant 10 minutes à 90°C puis déposés sur gel d'acrylamide 10 % pour une séparation par électrophorèse (SDS-PAGE). Les protéines ont alors été transférées sur une membrane de nitrocellulose et révélées par des anticorps primaires spécifiques : anti-YFP (8367-1 Living Colors) au 1/200ème, anti-myc A14 (sc-789 TEBU Peprotech Santa Cruz Biotechnology) au 1/500éme, puis un anticorps secondaire couplé à la péroxydase (IgG de chèvre anti-lapin ; Jackson Immunoresearch Laboratories, Inc., West Baltimore Pike) au 1/10000éme. Les bandes immuno-réactives ont été révélées par un kit ECL (Pharmacia Biotech).

### Immunoprecipitation.

Deux jours après la transfection, les cellules ont été lavées une fois au PBS froid, et les protéines ont été extraites par une incubation pendant 15 minutes dans du tampon de lyse (PBS 1X, Nonidet P40 1 %, Sodium Desoxycholate 0,5 %, SDS 0,1 %, NaN3 0,02 %, benzamidine 10 mg.L-1 et des inhibiteurs de trypsine 5 mg.L-1). Le lysat a été centrifugé à 18000 g pendant 15 min et le surnageant a ensuite été incubé pendant 3 heures à 4°C avec un anticorps anti-myc couplé à des billes d'agarose (*sc*-*40AC* TEBU preprotech, Santa CRUZ Biotechnology). Les précipités ont été lavés trois fois avec du tampon de lyse froid puis dénaturés avec du tampon de dépôt pour SDS-PAGE.

### Expériences de radioliaison.

Les expériences de radioliaison ont été réalisées comme décrit précédemment (Barr et al. (1999) J Biol Chem, 274, 21416-21424), avec de légères modifications. Pour déterminer la liaison de la leptine en surface, les cellules cultivées en plaques 6 puits ont été lavées deux fois au PBS froid et incubées dans le tampon de liaison (DMEM, 25 mmol.L-1 Hepes pH 7,4, 1 % BSA) contenant 100000 cpm/puit de ¹²⁵I-leptin (PerkinElmer life sciences, Paris, France) en présence ou non de 200 nmol.L-1 de leptine (PeproTech Inc, USA) pendant 4 h à 4°C. Les cellules ont été lavées deux fois au PBS froid, puis lysées dans du NaOH 1 N, et la radioactivité a été déterminée dans un compteur γ. Pour déterminer la liaison totale de la leptine, les cellules cultivées dans des boites de 10 cm de diamètre ont été solubilisées dans 1,5 ml de tampon de liaison contenant 0,15% de digitonine pendant 2 h à 4°C. Les extraits ont été centrifugés 30 min à vitesse maximale et à 4°C. Les surnageants (0,2ml) ont été incubés avec 100000 cpm de ¹²⁵I-leptine en présence ou non de 200 nmol.L-1 de leptine, dans un volume total de 0,25 ml en rotation constante à 4°C pendant une nuit. 0,5 ml de γ-globuline (1,25 mg/ml) et 0,5 ml de polyéthylène glycol 6000 (25 % p/v) ont été ajoutés pour précipiter les complexes récepteurs-ligands, qui on été centrifugés à 17000 g pendant 3 min. Le culot a été lavé une fois avec 1ml de polyéthylène glycol 6000 (12 % p/v) et la radioactivité a été déterminée dans un compteur γ.

### Test d'activation des gènes rapporteurs.

Les cellules HeLa cultivées dans des puits de plaques 6 puits ont été co-transfectées avec 500 ng d'un plasmide rapporteur exprimant la luciférase de firefly sous le contrôle d'éléments de réponse pour les facteurs STAT3 ou STAT5, (gift of Dr. Levy, Université de New York, New York, USA), 250 pg du vecteur d'expression pcDNA3-luciférase de *Renilla* (utilisé comme standard interne entre les échantillons) et avec 500 ng des différents vecteurs d'expression des OB-R ou le vecteur seul. 48 h après la transfection, les cellules ont été mises à jeun pendant une nuit dans du milieu Otpimem (Invitrogen, Groningen, Pays-bas) contenant 1 % de BSA, avant stimulation ou non par 10 nmol.L-1 de leptine pendant 48 h. Les cellules ont alors été lavées une fois au PBS puis lysées dans du tampon de lyse passif (Promega Corporation, Madison, WI) pendant 15 min à température ambiante. Les lysats totaux ont été centrifugés pendant 2 min à 15000 g et les surnageant ont été utilisées dans un test de mesure de luciférase (Dual Luciferase Assay System de Promega Corporation, Madison, WI) en utilisant un luminomètre Berthold (Lumat LB 9507). Les résultats sont exprimés en rapport des activités de la luciférase de *firefly* sur la luciférase de *Renilla.*

### Mesures de BRET en microplaques.

48 h après transfection, les cellules COS-7, HeLa ou HEK 293 exprimant les protéines de fusions des OB-R ont été détachées et lavées dans du PBS. 1-2x10⁵ cellules ont été distribuées dans des puits de plaques optiplate (96 puits, Packard Instrument Company, Meriden, CT) en présence ou non des ligands et incubées à 25°C. De façon alternative, nous avons procédé de la même façon avec des membranes préparées à partir des cellules exprimant les différentes constructions. Le substrat, la Coelenterazine h (Molecular Probes, Eugene, OR) a été ajouté à une concentration finale de 5 µmol.L-1 et les lectures ont été réalisées avec un lumino/fluorimetre Fusion^{™} (Packard Instrument Company, Meriden, CT) qui permet la mesure de la luminescence à travers deux filtres (filtre Luciférase: 485 ± 10 nm; filtre YFP: 530 ± 12.5 nm). Le rapport de BRET a été défini comme la différence d'émission à 530 nm/485 nm des cellules co-transfectées avec les protéines de fusion Luc et YFP et l'émission à 530 nm/485 nm de la protéine de fusion Luc transfectée seule dans les cellules. Les résultats sont exprimés en unités de milliBRET (mBU), 1 mBRET correspond aux valeurs des différences des ratios multipliées par 1000.

### RT-PCR.

Les ARN totaux ont été extraits par la méthode de Chomczynski et Sacchi (Chomcynzki P., et Sacchi N. (1987) Anal. Biochem. 162, 156-159). 1 µg d'ARN est dénaturé pendant 5 minutes à 68°C puis refroidit brutalement pendant 5 min à 4°C. L'échantillon dénaturé est reverse-transcrit pendant 1 h à 37°C dans 20 µl de milieu réactionnel RT (5µmol.L-1 PdN6, 10µmol.L-1 DTT, 50mmol.L-1 Tris-HCl pH=8,3, 75mmol.L-1 KCl, 5mmol.L-1 MgCl2, 500 µmol.L-1 dNTP, 200U RT-MMLV). Un aliquote de 2,5 µl de cette réaction est utilisé pour une réaction de PCR dans un volume final de 25µl (40mmol.L-1 Tris-HCl pH 8,4; 100 mmol.L-1 KCl; 1,5 mmol.L-1 MgCl2; 0,2mmol.L-1 de chaque dNTP; 0,141 mmol. L-1 d'amorces spécifiques d'OB-RGRP (sens humain: CCGTGGCAGGAAGC **SEQ ID N°43,** sens murin: GCAGCGACAGCCCCAGCTCC **SEQ ID N°44** antisens: CAGCCACACGAGCAAG **SEQ ID N°45**), et 0,035mmol.L-1 d'amorces spécifiques de la glyceraldéhyde phosphate déshydrogénase (GAPDH) (sens: GGAGAAGGCTGGGGC **SEQ ID N°46**, antisens: GATGGCATGGACTGTGG **SEQ ID N°47**) et 2,5U de TAQ DNA polymérase). Le protocole suivant a été utilisé pour la réaction de PCR : Dénaturation initiale de 3 min à 94°C, puis 22 à 30 cycles de dénaturation (20 sec à 94°C), hybridation (20 sec à 59°C), élongation (20 sec à 72°C) suivie par une élongation finale de 7 min à 72°C.

Un aliquote de la réaction de PCR est déposé sur un gel d'agarose à 2% pour séparer les produits de la réaction par électrophorèse. Les tailles attendues des fragments de la GAPDH et d'OBR-GRP sont respectivement de 229 pb et 334 pb.

### Synthèse d'oligonucléotides

Les oligonucléotides ont été synthétisés sur synthétiseur automatique d'ADN (modèle 8909 "Expedite MOSS" d'Applied Biosystems) par chimie standard de phosphoramidites et oxydation à l'iode. La déméthylation a été effectuée par une solution à 0,2 mol.L-1de 3H-1,2-benzodithiole-3-one 1,1-dioxide dans l'acétonitrile pendant 120 s. Le détachement du support et la déprotection ont été réalisés en ammoniac concentré (18 h à 55°C), puis les oligonucléotides ont étés purifiés par précipitation. Le produit de déprotection a été précipité par 10 volumes de 1-butanol; le culot repris dans un volume de NaCl à 0,3 mol.L-1a été reprécipité par l'addition de 4 volumes d'éthanol.

L'analyse par gel de polyacrylamide à 20 % (en tampon urée 8 mol.L-1 et Tris-borate 454 mmol.L-1 à pH 7,0) a montré une proportion supérieure à 80 % de produit de longueur attendue.

### Transfection des OligoDesoxyNucleotides antisens et du duplex d'ARN interférant synthétiques.

Pour la transfection de 300 000 cellules cultivées dans un puit de plaque 6 puits, 10 µL d'ODN antisens à 20 µmol.L-1 ou 10 µL du duplex d'ARN interférant à 20 µM ont été ont été dilués dans 175 µL de DMEM. 3 µL d'oligofectamine (Invitrogen, Groningen, Pays-bas) et 12 µL de DMEM ont été incubés dans un second tube pendant 10 min à température ambiante. Le mélange oligofectamine / DMEM est alors ajouté à l'ODN antisens dilué, vortexé et incubé 20 min à température ambiante. Pendant ce temps, les cellules sont lavées une fois au PBS, une fois au DMEM, puis recouvertes de 800 µl de DMEM. Le mélange ODN / oligofectamine a alors été ajouté goutte à goutte sur les cellules et incubé 4h à 37°C avant l'ajout de 500 µl de DMEM supplémenté avec 30 % de sérum.

### Exemple 1 : Topologie et localisation cellulaire d'OB-RGRP.

Pour étudier la topologie et la localisation subcellulaire d'OB-RGRP, nous avons étiqueté la protéine avec le variant jaune de la Green Fluorescent Protéine (YFP) à l'extrémité de sa queue C-terminale. La protéine de fusion a été exprimée dans les cellules HeLa et sa localisation a été déterminée par microscopie de fluorescence.

Les résultats montrent que la protéine de fusion est ciblée préférentiellement au niveau des membranes périnucléaires et dans des vésicules intracellulaires. Des résultats similaires ont été observés dans les cellules HEK. Aucune co-localisation avec des protéines cytoplasmique et nucléaires n'ont été observées, confirmant la localisation d'OB-RGRP au niveau de membranes (non montré). La nature exacte du compartiment membranaire a été déterminé par des études de co-localisation avec des marqueurs spécifiques de compartiment subcellulaires. Une forte co-localisation a été observée avec la chaîne invariante des protéines de classe Il du CMH, un marqueur du compartiment endocytique.

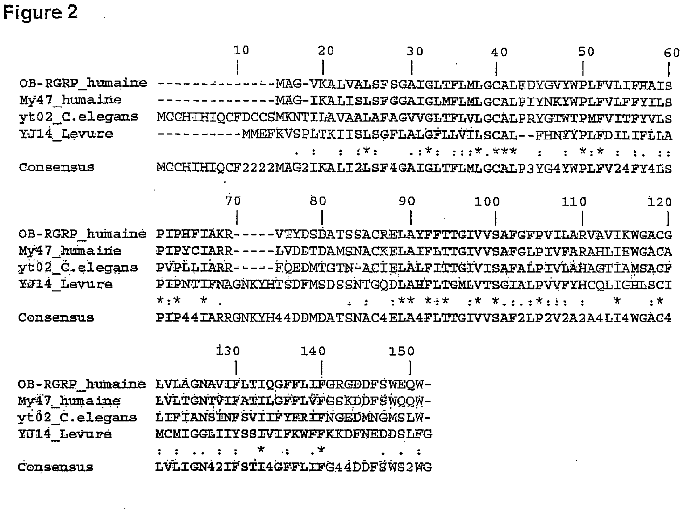

L'analyse initiale de la topologie d'OB-RGRP suggérait une organisation en 3 domaines transmembranaires (TM) Bailleul et al (1997) Nucleic Acids Research 25, 2752-2758). Une organisation similaire a été proposée pour MY047 (Huang et al. 2001) Biochimica et Biophysica acta. Gene structure and expression 327-331). Cependant, une nouvelle analyse du profil d'hydrophobicité des différentes séquences protéiques disponibles pour OB-RGRP et MY047 est aussi compatible avec un modèle à 4 TM (Fig. 2). La topologie diffère profondément entre ces deux modèles. Dans le modèle à 3 TM, les extrémités N- et C-terminales sont localisées de chaque coté de la membrane, alors que dans le modèle à 4 TM, les deux queues sont orientées du même coté de la membrane (Fig. 3a). Pour déterminer le bon modèle, nous avons utilisé la méthode de transfert d'énergie par résonance (BRET), qui a récemment été développée pour suivre les interactions protéine-protéine dans les cellules vivantes(Xu et al. (1999) Proc Natl Acad Sci U S A 96, 151-156). Dans le cas d'une proximité physique (< 100 Å) entre les deux protéines interagissant, un transfert d'énergie peut avoir lieu entre le donneur d'énergie (Luc) et l'accepteur d'énergie (YFP), fusionnés aux deux protéines d'intérêt. Nous avons étiqueté la queue N-terminale d'OB-RGRP avec la YFP, la queue C-terminale avec la luciférase, et nous avons observé le transfert d'énergie par des mesures de BRET avec cette double protéine de fusion. Le modèle à 3 TM ne permet pas de transfert puisque les deux partenaires de BRET sont séparés par la bicouche lipidique. Le modèle à 4 TM au contraire prévoit un fort transfert d'énergie puisque les deux partenaires sont localisés du même coté de la membrane. Comme montré dans la figure 3b, un très fort transfert d'énergie à été détecté pour la double protéine de fusion dans les cellules intactes, indiquant qu'OB-RGRP possède 4 TM.

L'ensemble de ces résultats suggère qu'OB-RGRP est une protéine membranaire à 4 domaines transmembranaires, possédant 3 courtes boucles et des extrémités N- et C-terminales courtes orientées du même coté de la membrane. OB-RGRP est majoritairement localisée dans des compartiments intracellulaires.

### Exemple 2: Olinomérisation d'OB-RGRP.

L'oligomérisation est une propriété commune à différentes protéines y compris des protéines membranaires comme les récepteurs tyrosine kinase, les récepteurs aux cytokines et les phosphotyrosines phosphatase. Il a été montré que cette oligomérisation joue un rôle important dans la fonction de ces protéines. Pour obtenir des éléments dans la fonction d'OB-RGRP, nous avons voulu savoir si cette protéine oligomérise.

OB-RGRP a été étiquetée avec la YFP à sa queue C-terminale et exprimée dans des cellules HeLa. Les protéines ont été séparées par électrophorèse sur gel de polyacrylamide en condition dénaturante (PAGE-SDS) et des expériences d'immunoblot ont été réalisées avec un anticorps anti-YFP. La figure. 4a révèle plusieurs bandes spécifiques d'OB-RGRP-YFP, correspondant à des formes monomériques, dimériques et des complexes oligomériques. Des résultats similaires ont été obtenus avec OB-RGRP étiquetée en N-terminal soit avec la YFP ou un épitope myc (Fig. 4 a,b). La formation des oligomères d'OB-RGRP a été observée sur des extraits cellulaires totaux après immunoprécipitaion. L'utilisation d'un cross-linker sur les cellules entières, stabilise les complexes dimériques, indiquant que la forme dimérique est la forme prédominante d'OB-RGRP dans les cellules intactes (Fig. 4a).

De façon surprenante, OB-RGRP a des propriétés inattendues puisque les oligomères sont stables en présence de différents agents dénaturant et/ou dissociant comme le SDS 5 %, le Triton X-100 1 %, le Nonidet P40 1 %, la digitonin1 %, le DTT 50 mmol.L⁻¹ et le β-mercaptoethanol 2 %. Cependant, des observations similaires ont été obtenues pour d'autres protéines membranaires comme la glycophorine A et les récepteurs β2-adrenergiques couplés aux protéines G. Des études sur ces protéines montrent respectivement que des motifs LIXXGVXXG et LXXXGXXXGXXXL dans les domaines transmembranaires sont essentiels pour la formation des oligomères. Des motifs similaire ont été identifiés dans les régions membranaires d'OB-RGRP.

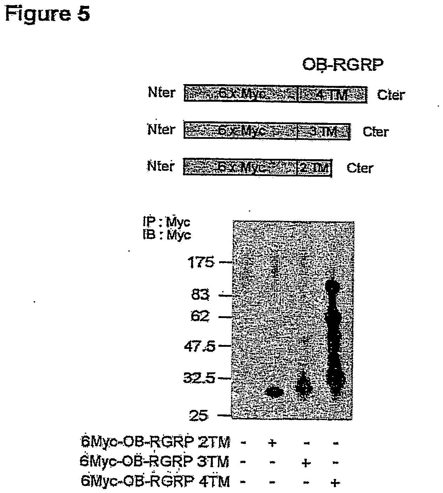

Pour identifier les déterminants moléculaires impliqués dans la dimérisation, nous avons réalisé des constructions d'OB-RGRP présentant des délétions progressives de la queue C-terminale (Fig. 5). Une construction contenant les deux premiers TM potentiels perd la capacité de former des oligomères. L'addition du 3^{ème} TM restaure la possibilité de former des dimères. Cependant, le profil complet d'oligomérisation a seulement été observé en présence des 4 TM potentiels.

Les oligomères de protéines membranaires peuvent être des artéfacts induit durant la préparation des échantillons (solubilisation, dénaturation etc.). C'est pourquoi il est important de vérifier l'oligomérisation des protéines dans des cellules vivantes. Les techniques de transfert d'énergie développées récemment comme le BRET permettent de suivre de telles interactions protéines-protéines dans les cellules vivantes. Des protéines de fusions d'OB-RGRP avec la luciférase et la YFP ont été utilisées pour suivre l'oligomérisation d'OB-RGRP dans les cellules vivantes. La co-expression des constructions OB-RGRP-YFP ou YFP-OB-RGRP avec la construction OB-RGRP-Luc induit un transfert d'énergie (Fig. 6a). La spécificité de cette interaction a été montrée par l'absence de transfert d'énergie lors de la co-expression avec deux protéines de fusions différentes : la βarrestine2-YFP(Angers et al. (2000) Proc Natl Acad Sci U S A 97, 3684-3689), ou le récepteur MT2 de la mélatonine-Luc (Ayoub et al. (2002) J Biol Chem 277, 21522-21528) . Nous avons alors exprimés différents rapports des partenaires de BRET (Fig. 6b). Le signal de BRET est augmenté de façon hyperbolique en fonction du ratio OB-RGRP-YFP / OB-RGRP-Luc, atteignant une asymptote qui correspond à la saturation des molécules donneurs d'énergie (OB-RGRP-Luc) par les molécules accepteurs (OB-RGRP-YFP), ce qui est attendu dans le cas d'une interaction spécifique.

Collectivement, ces résultats montrent qu'OB-RGRP est une protéine membranaire dimérique qui peut aussi être engagées dans des complexes oligomériques de plus haut poids moléculaire. Les 3ème et 4éme domaines transmembranaires potentiels semblent être important pour la formation des oligomères.

### Exemple 3: Interaction entre les OB-R et OB-RGRP et MY047.

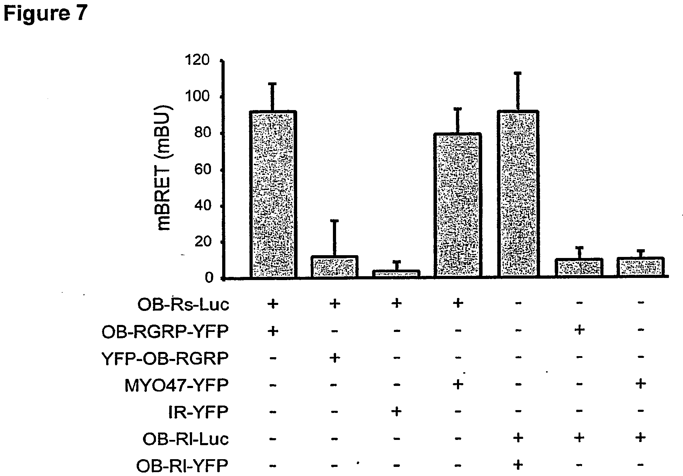

Nous avons utilisé la technologie de BRET pour étudier une possible interaction entre les OB-R et OB-RGRP dans les cellules vivantes. Un transfert d'énergie a été observé de façon constitutive dans les cellules co-exprimant la construction OB-Rₛ-Luc et la construction OB-RGRP-YFP indiquant une proximité des partenaires d'interaction (Fig. 7). Les mêmes résultats ont été obtenus dans des cellules co-exprimant OB-Rₛ-Luc et la construction MYO47-YFP, ainsi que dans l'orientation inverse : dans des cellules co-exprimant OB-RGRP-Luc et OB-Rₛ-YFP, ou dans des cellules co-exprimant MYO47-Luc et OB-Rₛ-YFP. La spécificité de ces interactions à été confirmée par l'absence de transfert d'énergie entre OB-Rₛ-Luc, OB-RGRP-Luc, MYO47-Luc et une construction du récepteur de l'insuline étiqueté avec la YFP(Boute et al. (2001) Mol Pharmacol 60, 640-645) , ainsi que dans l'orientation inverse : par l'absence de transfert d'énergie entre une construction du récepteur de l'insuline étiqueté avec la Luc et les constructions OB-Rₛ-YFP, OB-RGRP-YFP et MYO47-YFP. La co-expression d'OB-Rₛ-Luc et une construction d'OB-RGRP ou de MYO47 présentant l'étiquette YFP en N-terminal ne provoque pas de signal significatif, confirmant la spécificité de l'interaction avec OB-RGRP-YFP et MYO47 et indique que l'extrémité N-terminale d'OB-RGRP et MYO47 doit être impliquée dans l'interaction avec OB-R.

Aucun transfert d'énergie significatif n'a été observé dans les cellules co-exprimant les constructions OB-Rₗ-Luc et OB-RGRP-YFP ou YFP-OB-RGRP.

Ceci n'est pas du à une absence d'expression fonctionnelle OB-Rₗ-Luc puisqu'un signal de BRET spécifique a été observé dans des cellules co-exprimant OB-Rₗ₋YFP pour suivre la dimérisation des OB-R. L'absence de BRET entre les protéines de fusions OB-Rₗ-Luc et OB-RGRP-YFP n'exclue pas une interaction directe entre ces deux protéines puisque cela peut être expliqué par le fait que la distance entre les deux partenaires de BRET (Luc et YFP) soit plus importante que 100 Å, la distance maximale permettant d'obtenir un transfert. Cela doit être le cas puisque les extrémités N- et C-terminales d'OB-RGRP doivent être localisées proche de la région transmembranaire des OB-R, alors que l'extrémité C-terminale d'OB-Rₗ doit plus probablement pointer vers le cytoplasme à cause de sa longue queue intracellulaire d'environ 300 acides aminés. Etant donné que les isoformes courte et longue des OB-R partagent les mêmes régions trans- et juxta-membranaire et que l'interaction d'OB-RGRP avec OB-Rₛ est localisée à ce niveau, il est probable qu'OB-RGRP interagisse avec OB-R_{I} de la même manière qu'avec OB-Rₛ.

### Exemple 4: Effet de la surexpression d'OB-RGRP sur la signalisation des OB-R.

Des constructions contenant des éléments de réponse pour STAT3- ou STAT5-en amont d'un gène reporter luciférase ont été co-exprimées avec OB-R_{I} en absence ou en présence de différentes constructions d'OB-RGRP (Fig. 8). Les deux constructions ont été activées par la leptine de façon dose dépendante avec un EC50 d'environ 50 pM. Des résultats similaires ont été obtenus dans des cellules HEK 293 exprimant de façon stable un gène rapporteur pour STAT3. La surexpression de différentes constructions d'OB-RGRP n'a pas eu d'effet reproductible sur cette activation indiquant qu'OB-RGRP n'est pas un facteur limitant.

### Exemple 5: Effet de la surexpression d'OB-RGRP sur l'expression des OB-R à la surface.

Dans des levures knock-out pour OB-RGRP (Vps55), le transport de protéines est perturbé entre le golgi et les vacuoles (Belgareh-Touze et al. (2002) Molecular Biology Of The Cell 13, 1694-1708). Bien que les OB-R soit activés uniquement lorsqu'ils sont exprimés à la membrane plasmique, une quantité importante de récepteurs est accumulée dans des compartiments intracellulaires (Barr, et al. (1999) J Biol Chem, 274, 21416-21424)(Lundin et al. (2000) Biochimica et Biophysica Acta 1499, 130-138). C'est pourquoi nous avons testé les effets de la surexpression d'OB-RGRP sur l'expression des OB-R à la surface des cellules.

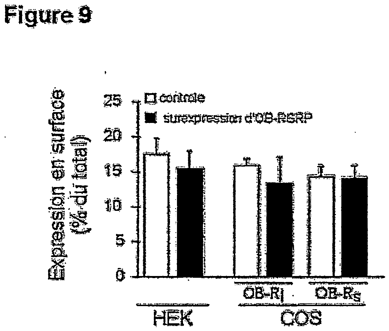

La répartition des récepteurs à été étudiée par des expériences de liaison de ¹²⁵I-leptine. En accord avec d'autres auteurs (Barr et al. 1999), nous avons montré que seulement 10 - 20 % des récepteurs OB-R_{I} et OB-Rₛ sont exprimés à la surface de cellules COS transfectées (Fig. 9) et des cellules HeLa. Ce n'est pas un artéfact due à l'expression de récepteurs exogènes car des valeurs similaires sont obtenues dans des cellules HEK 293 exprimant des récepteurs endogènes OB-R (Fig. 9). La surexpression d'OB-RGRP n'a pas montré de modification de la quantité totale dans les cellules, et le % de récepteurs exprimés à la surface (Fig. 9).

### Exemple 6 : Caracterisation de desoxynucléotides antisens spécifiques d'OB-RGRP

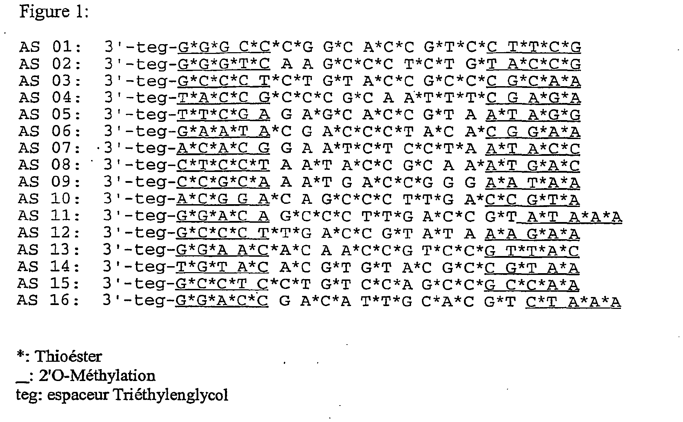

OB-RGRP semble avoir une expression ubiquitaire, c'est pourquoi la diminution de l'expression de cette protéine a été choisie comme approche alternative pour étudier son rôle dans la fonction des OB-R. Quatorze antisens spécifiques pour OB-RGRP (AS 1 à 14 ; **SEQ ID N°22 à SEQ ID N°34 et SEQ ID N°2**) et deux antisens aléatoires (AS 15 et 16 ; **SEQ ID N°35 et SEQ ID N°36**) ont été choisis (voir Figure 1), synthétisés puis testés pour leur capacité à inhiber l'expression d'OB-RGRP par des expériences de RT-PCR semi-quantitative dans les cellules HeLa exprimant OB-RGRP de façon endogène (Fig. 10). Seul un de ces antisens (AS-14), dérivé de la région 3' non traduite de l'ARNm d'OB-RGRP interfère avec l'expression d'OB-RGRP. Le marquage de cet antisens avec le fluorophore Cy3 a permit de montrer que l'ensemble des cellules était transfecté avec nos conditions expérimentales, lors de nos différentes expériences.

### Exemple 7: Effet des différents ARN interférants sur le niveau des messagers d'OB-RGRP observés par RT-PCR semi-quantitative.

Afin de diminuer l'expression d'OB-RGRP, une approche alternative a été d'utiliser des ARN interférant.Pour cela, nous avons utilisé d'une part, une séquence candidate ARN interférant synthétique dirigée à la fois contre la séquence humaine et murine (figure 11a) ; et d'autre part, un vecteur (PCR3.1-RNAi 14) exprimant un ARN interférant en épingle à cheveux dirigé contre une séquence murine d'OB-RGRP (figure 11c).

La capacité des ARNi à diminuer l'expression d'OB-RGRP endogène à été testée par RT-PCR.

L'ARNi synthétique transfecté dans des cellules HELA (d'origine humaine) provoque une diminution de l'expression d'OB-RGRP humaine.

La transfection du vecteur PCR3.1-RNAi-14 provoque le même effet dans des cellules L (d'origine murine).

### Exemple 8: Effet de l'antisens spécifique d'OB-RGRP sur la signalisation et l'expression en surface des OB-R.

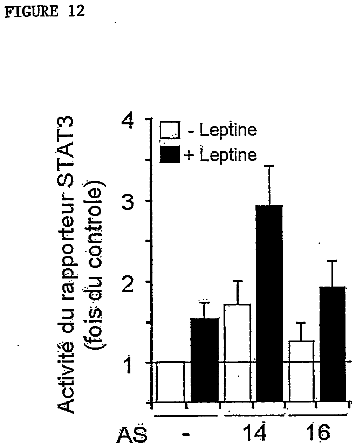

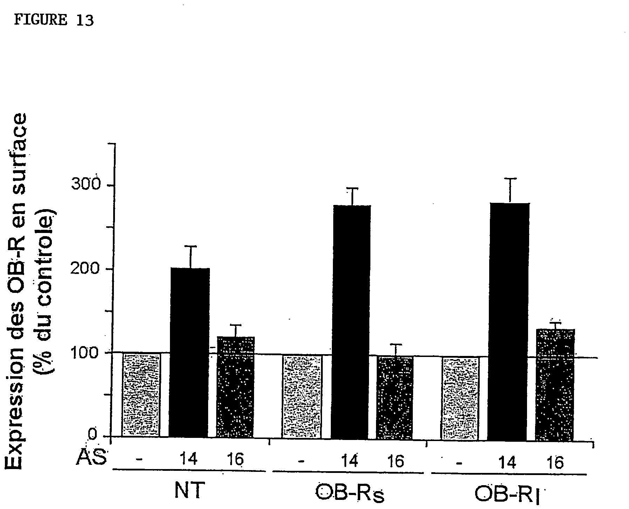

Les cellules HeLa ont d'abord été co-transfectées avec les vecteurs d'expression d'OB-Rₗ et le gène rapporteur pour STAT3 puis avec les antisens. La leptine provoque une augmentation de l'activation basale du gène rapporteur pour STAT 3 d'environ 1,5 fois dans les cellules contrôles sans antisens, ou avec un antisens contrôle (AS16) (Fig. 12). Dans les cellules transfectées avec l'antisens spécifique pour OB-RGRP (AS-14), la signalisation basale et stimulée par la leptine est relativement augmentée par rapport aux conditions contrôles. Ceci montre que l'activation de la voie JAK/STAT est augmentée dans les cellules présentant une diminution de l'expression d'OB-RGRP. Ces observations peuvent être expliquées par un effet inhibiteur d'OB-RGRP sur l'activité basale et stimulée des OB-R, et dans ce cas, OB-RGRP peut être considérée comme un régulateur de la signalisation des OB-R. Une autre alternative est qu'OB-RGRP pourrait réguler l'expression des récepteurs en surface en limitant le nombre des OB-R atteignant la surface des cellules. Ceci est en accord avec le fait que seulement 10 à 20 % des récepteurs exprimés atteignent la surface cellulaire. Dans cette hypothèse, la diminution de l'expression d'OB-RGRP devrait augmenter le nombre de récepteurs à la surface des cellules, ce qui devrait augmenter la signalisation par ces récepteurs. Pour tester cette hypothèse, nous avons quantifié le nombre de récepteurs OB-Rₗ et OB-Rₛ exprimés à la surface des cellules en présence (contrôle) et en absence (AS-14) d'OB-RGRP (Fig. 13). La transfection de l'antisens aléatoire n'a pas montré d'effet sur le nombre de récepteurs exprimés à la surface des cellules, alors que celle de l'antisens spécifique (AS-14) a provoqué une augmentation de 3 fois du nombre des OB-R exprimés à la membrane plasmique. Des résultats similaires ont été obtenus dans des cellules HeLa non transfectées exprimant des récepteurs endogènes. Dans ces conditions expérimentales, le nombre total de récepteurs, mesuré par des expériences de liaison de ¹²⁵I-leptine, n'a pas montré de variations significatives.

L'ensemble de nos résultats est consistant avec le rôle d'OB-RGRP chez la levure, dans le transport des protéines. L'augmentation de l'expression en surface des OB-R semble impliquée dans l'augmentation de la signalisation observée. Cependant, nous ne pouvons pas totalement exclure l'hypothèse qu'OB-RGRP régule directement l'activité des OB-R. L'application d'antisens spécifiques dirigés contre OB-RGRP devrait être utile pour augmenter la signalisation des OB-R dans les désordres associés à la leptine, comme l'obésité humaine où l'on observe une résistance à la leptine, caractérisée par une réponse inadaptée à cette hormone. L'augmentation de l'expression des récepteurs à la surface des cellules et de leur signalisation doit être importante pour augmenter la réponse à la leptine dans le cas de l'obésité humaine, premièrement par augmentation du transport de la leptine vers le cerveau à travers la barrière hémato-encéphalique et deuxièmement par une augmentation de la signalisation des OB-R au niveau de l'hypothalamus. L'interaction entre OB-RGRP et OB-Rₛ laisse supposer que l'action d'OB-RGRP se fait par cette interaction directe avec les récepteurs et qu'empêcher celle-ci peut conduire à reproduire les effets de l'ODN antisens spécifique. Nous proposons d'utiliser le test de BRET des interactions entre OB-RGRP et OB-Rₛ, et MYO47 et OB-Rₛ décrit plus haut, comme test de criblage de molécules pouvant moduler cette interaction. Ce test pourra être réalisé soit sur des cellules entières ou perméabilisées, co-exprimant les protéines de fusions des partenaires de BRET d'OB-RGRP et d'OB-Rₛ ou de MYO47 et d' OB-Rₛ, soit sur des fractions membranaires issues de ces cellules.

## Revendications

1. Oligonucléotide éventuellement modifié comprenant de 8 à 50 nucléotides qui hybride de manière spécifique à la séquence SEQ ID N° 1 et inhibe l'expression de OB-RGRP, **caractérisé en ce qu'**il comprend un séquence présentant une identité d'au moins 90% avec la séquence SEQ ID NO:2.

2. Oligonucléotide selon la revendication 1 caractétisé en ce qu'il favorise l'expression des récepteurs de la leptine à la surface cellulaire.

3. Oligonucléotide selon l'une des revendications 1 et 2 **caractérisé en ce qu'**il est un antisens.

4. Oligonucléotide selon l'une des revendications 1 à 3 **caractérisé en ce que** des nucléotides sont thioestérifiés.

5. Oligonucléotide selon l'une des revendications 1 à 3 **caractérisé en ce que** des nucléotides sont 2' O-méthylés.

6. Oligonucléotide selon l'une des revendications 1 à 3 **caractérisé en ce qu'**il présente un résidu triéthylèneglycol à son extrémité 3'.

7. Oligonucléotide selon l'une des revendications 1 à 3 **caractérisé en ce qu'**il est simple brin.

8. Oligonucléotide selon l'une des revendications 1 à 7 ans laquelle les nucléotides en positions 2, 4, 6, 7, 9, 11, 13, 15, 17, 19 et 20, dans le sens 5' vers 3', sont thioestérifiés.

9. Oligonucléotide selon l'une des revendications 1 à 7 dans laquelle les nucléotides en positions 1, 2, 3, 4, 5, 18, 17, 18, 19 et 20, dans le sens 5' vers 3', sont 2' O-méthylés.

10. Oligonucléotide selon l'une des revendications 1 à 9 **caractérisé en ce qu'**il est un ADN.

11. Oligonucleotide de type Acide Ribonucléique interférant (ARNi) comprenant de 15 à 60 nucléotides qui hybride de manière spécifique à la séquence SEQ ID N° 21 et inhibe l'expression de OB-RGRP, **caractérisé en ce qu'**il est un ARN double brin et **en ce qu'**au moins l'un des deux brins comprend une séquence présentant une identité d'au moins 90 %, avec l'une des séquences SEQ ID N° 37 ou SEQ ID° N 38.

12. Oligonucleotide de type Acide Ribonucléique interférant (ARNi) comprenant de 15 à 60 nucléotides qui hybride de manière spécifique à la séquence SEQ ID N° 21 et inhibe l'expression de OB-RGRP, carattérisé en ce qu'il est un ARN simple brin et en ce qu'il comprend une séquence présentant une identité d'au moins 90%, avec la séquence SEQ ID N° 42.

13. Vecteur exprimant un oligonuclétide selon l'une des revendications 1 à 3, 11 et 12.

14. Cellule contenant un vecteur selon la revendication 13.

## Claims

1. Optionally modified oligonucleotide comprising from 8 to 50 nucleotides, which hybridizes specifically to the sequence SEQ ID No. 1 and inhibits the expression of OB-RGRP, **characterized in that** it comprises a sequence exhibiting at least 90% identity with the sequence SEQ ID No. 2.

2. Oligonucleotide according to Claim 1, **characterized in that** it promotes the expression of leptin receptors at the cell surface.

3. Oligonucleotide according to either of Claims 1 and 2, **characterized in that** it is an antisense oligonucleotide.

4. Oligonucleotide according to one of Claims 1 to 3, **characterized in that** nucleotides are thioesterified.

5. Oligonucleotide according to one of Claims 1 to 3, **characterized in that** nucleotides are 2'-O-methylated.

6. Oligonucleotide according to one of Claims 1 to 3, **characterized in that** it has a triethylene glycol residue at is 3' end.

7. Oligonucleotide according to one of Claims 1 to 3, **characterized in that** it is single-stranded.

8. Oligonucleotide according to one of Claims 1 to 7, in which the nucleotides in positions 2, 4, 6, 7, 9, 11, 13, 15, 17, 19 and 20, in the 5' to 3' direction, are thioesterified.

9. Oligonucleotide according to one of Claims 1 to 7, in which the nucleotides in positions 1, 2, 3, 4, 5, 16, 17, 18, 19 and 20, in the 5' to 3' direction, are 2'-O-methylated.

10. Oligonucleotide according to one of Claims 1 to 9, **characterized in that** it is a DNA.

11. Oligonucleotide of interfering ribonucleic acid (iRNA) type comprising from 15 to 60 nucleotides, which hybridizes specifically to the sequence SEQ ID No. 21 and inhibits the expression of OB-RGRP, **characterized in that** it is a doublestranded RNA and **in that** at least one of the two strands comprises a sequence exhibiting at least 90% identity with one of the sequences SEQ ID No. 37 or SEQ ID No. 38.

12. Oligonucleotide of interfering ribonucleic acid (iRNA) type comprising from 15 to 60 nucleotides, which hybridizes specifically to the sequence SEQ ID No. 21 and inhibits the expression of OB-RGRP, **characterized in that** it is a single-stranded RNA and **in that** it comprises a sequence exhibiting at least 90% identity with the sequence SEQ ID No. 42.

13. Vector expressing an oligonucleotide according to one of Claims 1 to 3, 11 and 12.

14. Cell containing a vector according to Claim 13.

## Patentansprüche

1. Oligonukleotid, das gegebenenfalls modifiziert ist und 8 bis 50 Nukleotide umfasst und das spezifisch mit der Sequenz SEQ ID NR: 1 hybridisiert und die Expression von OB-RGRP inhibiert, **dadurch gekennzeichnet, dass** es eine Sequenz umfasst, die eine Identität von mindestens 90% mit der Sequenz SEQ ID NR: 2 aufweist.

2. Oligonukleotid nach Anspruch 1, **dadurch gekennzeichnet, dass** es die Expression von Rezeptoren für Leptin an der Zelloberfläche fördert.

3. Oligonukleotid nach einem der Ansprüche 1 und 2, **dadurch gekennzeichnet, dass** es sich um ein Antisense-Oligonukleotid handelt.

4. Oligonukleotid nach einem der Ansprüche 1 bis 3, **dadurch gekennzeichnet, dass** Nukleotide über Thioesterbindungen gebunden sind.

5. Oligonukleotid nach einem der Ansprüche 1 bis 3, **dadurch gekennzeichnet, dass** Nukleotide 2'-O-methyliert sind.

6. Oligonukleotid nach einem der Ansprüche 1 bis 3, **dadurch gekennzeichnet, dass** es einen Triethylenglykol-Rest an seinem 3'-Ende aufweist.

7. oligonukleotid nach einem der Ansprüche 1 bis 3, **dadurch gekennzeichnet, dass** es einzelsträngig ist.

8. Oligonukleotid nach einem der Ansprüche 1 bis 7, wobei die Nukleotide an den Positionen 2, 4, 6, 7, 9, 11, 13, 15, 17, 19 und 20 in 5'-3'-Richtung über Thioesterbindungen gebunden sind.

9. Oligonukleotid nach einem der Ansprüche 1 bis 7, wobei die Nukleotide an den Positionen 1, 2, 3, 4, 5, 16, 17, 18, 19 und 20 in 5'-3'-Richtung 2'-O-methyliert sind.

10. Oligonukleotid nach einem der Ansprüche 1 bis 9, **dadurch gekennzeichnet, dass** es sich um eine DNA handelt.

11. Oligonukleotid des Typs einer interferierenden Ribonukleinsäure (RNAi), umfassend 15 bis 60 Nukleotide, das spezifisch mit der Sequenz SEQ ID NR: 21 hybridisiert und die Expression von OB-RGRP inhibiert, **dadurch gekennzeichnet, dass** es sich um eine doppelsträngige RNA handelt und dass mindestens einer der beiden Stränge eine Sequenz umfasst, die eine Identität von mindestens 90% mit einer der Sequenzen SEQ ID NR: 37 oder SEQ ID NR: 38 aufweist.

12. Oligonukleotid des Typs einer interferierenden Ribonukleinsäure (RNAi), umfassend 15 bis 60 Nukleotide, das spezifisch mit der Sequenz SEQ ID NR: 21 hybridisiert und die Expression von OB-RGRP inhibiert, **dadurch gekennzeichnet, dass** es sich um eine einzelsträngige RNA handelt, und dadurch, dass es eine Sequenz umfasst, die eine Identität von mindestens 90% mit der Sequenz SEQ ID NR: 42 aufweist.

13. Vektor, der ein Oligonukleotid nach einem der Ansprüche 1 bis 3, 11 und 12 exprimiert.

14. Zelle, die einen Vektor nach Anspruch 13 enthält.
